# EUROPEAN PATENT APPLICATION

(11) **EP 2 510 934 A1**
(43) Date of publication of application: **17.10.2012**
(21) Application number: 12165781.1
(22) Date of filing: 03.11.2006
(51) Int. Cl.: A61K 38/00, A61K 39/00, A61K 48/00, C12N 15/11

(54) **Methods for promoting neurite outgrowth and survival of dopaminergic neurons**

(30) Priority: 04.11.2005 US 733166 P; 15.05.2006 US 800009 P; 19.06.2006 US 814523 P
(62) Divisional of application: 06836888.5
(71) Applicant: Biogen Idec MA Inc., Cambridge, MA 02142 (US); THE McLEAN HOSPITAL CORPORATION, Belmont, MA 02478 (US)
(72) Inventor: Mi, Sha, Belmont, MA 02478 (US); Isacson, Ole, Cambridge, MA 02138 (US)
(74) Representative: Miller, David James

(57) **Abstract**

The present invention relates generally to methods for promoting regeneration, outgrowth and survival of dopaminergic neurons comprising contacting said dopaminergic neurons with an effective amount of a composition comprising an Sp35 antagonist. Additionally, the invention is related generally to methods of treating various diseases, disorders or injuries associated with dopaminergic neuronal degeneration or death by administration of an Sp35 antagonist.

SEQUENCE LISTING

<110> Biogen IDEC MA Inc.
The McLean Hospital Corporation
Mi, Sha
Isacson, Ole

<120> Methods for Promoting Neurite Outgrowth and Survival of
Dopaminergic Neurons

<130> 2159.073PC03

<150> 60/814,523
<151> 2006-06-19

<150> 60/800,009
<151> 2006-05-15

<150> 60/733,166
<151> 2005-11-04

<160> 15

<170> PatentIn version 3.3

<210> 1
<211> 1845
<212> DNA
<213> Homo sapiens

<220>
<221> CDS
<222> (1)..(1845)

<400> 1
atg ctg gcg ggg ggc gtg agg agc atg ccc agc ccc ctc ctg gcc tgc 48
Met Leu Ala Gly Gly Val Arg Ser Met Pro Ser Pro Leu Leu Ala Cys
1               5                   10                  15

tgg cag ccc atc ctc ctg ctg gtg ctg ggc tca gtg ctg tca ggc tcg 96
Trp Gln Pro Ile Leu Leu Leu Val Leu Gly Ser Val Leu Ser Gly Ser
            20                  25                  30

gcc acg ggc tgc ccg ccc cgc tgc gag tgc tcc gcc cag gac cgc gct 144
Ala Thr Gly Cys Pro Pro Arg Cys Glu Cys Ser Ala Gln Asp Arg Ala
        35                  40                  45

gtg ctg tgc cac cgc aag cgc ttt gtg gca gtc ccc gag ggc atc ccc 192
Val Leu Cys His Arg Lys Arg Phe Val Ala Val Pro Glu Gly Ile Pro
    50                  55                  60

acc gag acg cgc ctg ctg gac cta ggc aag aac cgc atc aaa acg ctc 240
Thr Glu Thr Arg Leu Leu Asp Leu Gly Lys Asn Arg Ile Lys Thr Leu
65                  70                  75                  80

aac cag gac gag ttc gcc agc ttc ccg cac ctg gag gag ctg gag ctc 288
Asn Gln Asp Glu Phe Ala Ser Phe Pro His Leu Glu Glu Leu Glu Leu
                85                  90                  95

aac gag aac atc gtg agc gcc gtg gag ccc ggc gcc ttc aac aac ctc 336

Asn Glu Asn Ile Val Ser Ala Val Glu Pro Gly Ala Phe Asn Asn Leu
            100                 105                 110

ttc aac ctc cgg acg ctg ggt ctc cgc agc aac cgc ctg aag ctc atc 384
Phe Asn Leu Arg Thr Leu Gly Leu Arg Ser Asn Arg Leu Lys Leu Ile
        115                 120                 125

ccg cta ggc gtc ttc act ggc ctc agc aac ctg acc aag ctg gac atc 432
Pro Leu Gly Val Phe Thr Gly Leu Ser Asn Leu Thr Lys Leu Asp Ile
    130                 135                 140

agc gag aac aag att gtt atc ctg ctg gac tac atg ttt cag gac ctg 480
Ser Glu Asn Lys Ile Val Ile Leu Leu Asp Tyr Met Phe Gln Asp Leu
145                 150                 155                 160

tac aac ctc aag tca ctg gag gtt ggc gac aat gac ctc gtc tac atc 528
Tyr Asn Leu Lys Ser Leu Glu Val Gly Asp Asn Asp Leu Val Tyr Ile
                165                 170                 175

tct cac cgc gcc ttc agc ggc ctc aac agc ctg gag cag ctg acg ctg 576
Ser His Arg Ala Phe Ser Gly Leu Asn Ser Leu Glu Gln Leu Thr Leu
            180                 185                 190

gag aaa tgc aac ctg acc tcc atc ccc acc gag gcg ctg tcc cac ctg 624
Glu Lys Cys Asn Leu Thr Ser Ile Pro Thr Glu Ala Leu Ser His Leu
        195                 200                 205

cac ggc ctc atc gtc ctg agg ctc cgg cac ctc aac atc aat gcc atc 672
His Gly Leu Ile Val Leu Arg Leu Arg His Leu Asn Ile Asn Ala Ile
    210                 215                 220

cgg gac tac tcc ttc aag agg ctc tac cga ctc aag gtc ttg gag atc 720
Arg Asp Tyr Ser Phe Lys Arg Leu Tyr Arg Leu Lys Val Leu Glu Ile
225                 230                 235                 240

tcc cac tgg ccc tac ttg gac acc atg aca ccc aac tgc ctc tac ggc 768
Ser His Trp Pro Tyr Leu Asp Thr Met Thr Pro Asn Cys Leu Tyr Gly
                245                 250                 255

ctc aac ctg acg tcc ctg tcc atc aca cac tgc aat ctg acc gct gtg 816
Leu Asn Leu Thr Ser Leu Ser Ile Thr His Cys Asn Leu Thr Ala Val
            260                 265                 270

ccc tac ctg gcc gtc cgc cac cta gtc tat ctc cgc ttc ctc aac ctc 864
Pro Tyr Leu Ala Val Arg His Leu Val Tyr Leu Arg Phe Leu Asn Leu
        275                 280                 285

tcc tac aac ccc atc agc acc att gag ggc tcc atg ttg cat gag ctg 912
Ser Tyr Asn Pro Ile Ser Thr Ile Glu Gly Ser Met Leu His Glu Leu
    290                 295                 300

ctc cgg ctg cag gag atc cag ctg gtg ggc ggg cag ctg gcc gtg gtg 960
Leu Arg Leu Gln Glu Ile Gln Leu Val Gly Gly Gln Leu Ala Val Val
305                 310                 315                 320

gag ccc tat gcc ttc cgc ggc ctc aac tac ctg cgc gtg ctc aat gtc 1008
Glu Pro Tyr Ala Phe Arg Gly Leu Asn Tyr Leu Arg Val Leu Asn Val
                325                 330                 335

tct ggc aac cag ctg acc aca ctg gag gaa tca gtc ttc cac tcg gtg 1056
Ser Gly Asn Gln Leu Thr Thr Leu Glu Glu Ser Val Phe His Ser Val

340                 345                 350

ggc aac ctg gag aca ctc atc ctg gac tcc aac ccg ctg gcc tgc gac      1104
Gly Asn Leu Glu Thr Leu Ile Leu Asp Ser Asn Pro Leu Ala Cys Asp
        355                 360                 365

tgt cgg ctc ctg tgg gtg ttc cgg cgc cgc tgg cgg ctc aac ttc aac      1152
Cys Arg Leu Leu Trp Val Phe Arg Arg Arg Trp Arg Leu Asn Phe Asn
    370                 375                 380

cgg cag cag ccc acg tgc gcc acg ccc gag ttt gtc cag ggc aag gag      1200
Arg Gln Gln Pro Thr Cys Ala Thr Pro Glu Phe Val Gln Gly Lys Glu
385                 390                 395                 400

ttc aag gac ttc cct gat gtg cta ctg ccc aac tac ttc acc tgc cgc      1248
Phe Lys Asp Phe Pro Asp Val Leu Leu Pro Asn Tyr Phe Thr Cys Arg
                405                 410                 415

cgc gcc cgc atc cgg gac cgc aag gcc cag cag gtg ttt gtg gac gag      1296
Arg Ala Arg Ile Arg Asp Arg Lys Ala Gln Gln Val Phe Val Asp Glu
            420                 425                 430

ggc cac acg gtg cag ttt gtg tgc cgg gcc gat ggc gac ccg ccg ccc      1344
Gly His Thr Val Gln Phe Val Cys Arg Ala Asp Gly Asp Pro Pro Pro
        435                 440                 445

gcc atc ctc tgg ctc tca ccc cga aag cac ctg gtc tca gcc aag agc      1392
Ala Ile Leu Trp Leu Ser Pro Arg Lys His Leu Val Ser Ala Lys Ser
    450                 455                 460

aat ggg cgg ctc aca gtc ttc cct gat ggc acg ctg gag gtg cgc tac      1440
Asn Gly Arg Leu Thr Val Phe Pro Asp Gly Thr Leu Glu Val Arg Tyr
465                 470                 475                 480

gcc cag gta cag gac aac ggc acg tac ctg tgc atc gcg gcc aac gcg      1488
Ala Gln Val Gln Asp Asn Gly Thr Tyr Leu Cys Ile Ala Ala Asn Ala
                485                 490                 495

ggc ggc aac gac tcc atg ccc gcc cac ctg cat gtg cgc agc tac tcg      1536
Gly Gly Asn Asp Ser Met Pro Ala His Leu His Val Arg Ser Tyr Ser
            500                 505                 510

ccc gac tgg ccc cat cag ccc aac aag acc ttc gct ttc atc tcc aac      1584
Pro Asp Trp Pro His Gln Pro Asn Lys Thr Phe Ala Phe Ile Ser Asn
        515                 520                 525

cag ccg ggc gag gga gag gcc aac agc acc cgc gcc act gtg cct ttc      1632
Gln Pro Gly Glu Gly Glu Ala Asn Ser Thr Arg Ala Thr Val Pro Phe
    530                 535                 540

ccc ttc gac atc aag acc ctc atc atc gcc acc acc atg ggc ttc atc      1680
Pro Phe Asp Ile Lys Thr Leu Ile Ile Ala Thr Thr Met Gly Phe Ile
545                 550                 555                 560

tct ttc ctg ggc gtc gtc ctc ttc tgc ctg gtg ctg ctg ttt ctc tgg      1728
Ser Phe Leu Gly Val Val Leu Phe Cys Leu Val Leu Leu Phe Leu Trp
                565                 570                 575

agc cgg ggc aag ggc aac aca aag cac aac atc gag atc gag tat gtg      1776
Ser Arg Gly Lys Gly Asn Thr Lys His Asn Ile Glu Ile Glu Tyr Val
            580                 585                 590

ccc cga aag tcg gac gca ggc atc agc tcc gcc gac gcg ccc cgc aag 1824
Pro Arg Lys Ser Asp Ala Gly Ile Ser Ser Ala Asp Ala Pro Arg Lys
        595                 600                 605

ttc aac atg aag atg ata tga 1845
Phe Asn Met Lys Met Ile
    610

<210> 2
<211> 614
<212> PRT
<213> Homo sapiens

<400> 2

Met Leu Ala Gly Gly Val Arg Ser Met Pro Ser Pro Leu Leu Ala Cys
1               5                   10                  15

Trp Gln Pro Ile Leu Leu Leu Val Leu Gly Ser Val Leu Ser Gly Ser
            20                  25                  30

Ala Thr Gly Cys Pro Pro Arg Cys Glu Cys Ser Ala Gln Asp Arg Ala
        35                  40                  45

Val Leu Cys His Arg Lys Arg Phe Val Ala Val Pro Glu Gly Ile Pro
    50                  55                  60

Thr Glu Thr Arg Leu Leu Asp Leu Gly Lys Asn Arg Ile Lys Thr Leu
65                  70                  75                  80

Asn Gln Asp Glu Phe Ala Ser Phe Pro His Leu Glu Glu Leu Glu Leu
                85                  90                  95

Asn Glu Asn Ile Val Ser Ala Val Glu Pro Gly Ala Phe Asn Asn Leu
            100                 105                 110

Phe Asn Leu Arg Thr Leu Gly Leu Arg Ser Asn Arg Leu Lys Leu Ile
        115                 120                 125

Pro Leu Gly Val Phe Thr Gly Leu Ser Asn Leu Thr Lys Leu Asp Ile
    130                 135                 140

Ser Glu Asn Lys Ile Val Ile Leu Leu Asp Tyr Met Phe Gln Asp Leu
145                 150                 155                 160

Tyr Asn Leu Lys Ser Leu Glu Val Gly Asp Asn Asp Leu Val Tyr Ile
                165                 170                 175

Ser His Arg Ala Phe Ser Gly Leu Asn Ser Leu Glu Gln Leu Thr Leu
            180                 185                 190

Glu Lys Cys Asn Leu Thr Ser Ile Pro Thr Glu Ala Leu Ser His Leu
        195                 200                 205

His Gly Leu Ile Val Leu Arg Leu Arg His Leu Asn Ile Asn Ala Ile
    210                 215                 220

Arg Asp Tyr Ser Phe Lys Arg Leu Tyr Arg Leu Lys Val Leu Glu Ile
225                 230                 235                 240

Ser His Trp Pro Tyr Leu Asp Thr Met Thr Pro Asn Cys Leu Tyr Gly
                245                 250                 255

Leu Asn Leu Thr Ser Leu Ser Ile Thr His Cys Asn Leu Thr Ala Val
            260                 265                 270

Pro Tyr Leu Ala Val Arg His Leu Val Tyr Leu Arg Phe Leu Asn Leu
        275                 280                 285

Ser Tyr Asn Pro Ile Ser Thr Ile Glu Gly Ser Met Leu His Glu Leu
    290                 295                 300

Leu Arg Leu Gln Glu Ile Gln Leu Val Gly Gly Gln Leu Ala Val Val
305                 310                 315                 320

Glu Pro Tyr Ala Phe Arg Gly Leu Asn Tyr Leu Arg Val Leu Asn Val
                325                 330                 335

Ser Gly Asn Gln Leu Thr Thr Leu Glu Glu Ser Val Phe His Ser Val
            340                 345                 350

Gly Asn Leu Glu Thr Leu Ile Leu Asp Ser Asn Pro Leu Ala Cys Asp
        355                 360                 365

Cys Arg Leu Leu Trp Val Phe Arg Arg Arg Trp Arg Leu Asn Phe Asn
    370                 375                 380

Arg Gln Gln Pro Thr Cys Ala Thr Pro Glu Phe Val Gln Gly Lys Glu
385                 390                 395                 400

Phe Lys Asp Phe Pro Asp Val Leu Leu Pro Asn Tyr Phe Thr Cys Arg
                405                 410                 415

Arg Ala Arg Ile Arg Asp Arg Lys Ala Gln Gln Val Phe Val Asp Glu

            420                 425                 430

Gly His Thr Val Gln Phe Val Cys Arg Ala Asp Gly Asp Pro Pro Pro
        435                 440                 445

Ala Ile Leu Trp Leu Ser Pro Arg Lys His Leu Val Ser Ala Lys Ser
    450                 455                 460

Asn Gly Arg Leu Thr Val Phe Pro Asp Gly Thr Leu Glu Val Arg Tyr
465                 470                 475                 480

Ala Gln Val Gln Asp Asn Gly Thr Tyr Leu Cys Ile Ala Ala Asn Ala
                485                 490                 495

Gly Gly Asn Asp Ser Met Pro Ala His Leu His Val Arg Ser Tyr Ser
            500                 505                 510

Pro Asp Trp Pro His Gln Pro Asn Lys Thr Phe Ala Phe Ile Ser Asn
        515                 520                 525

Gln Pro Gly Glu Gly Glu Ala Asn Ser Thr Arg Ala Thr Val Pro Phe
    530                 535                 540

Pro Phe Asp Ile Lys Thr Leu Ile Ile Ala Thr Thr Met Gly Phe Ile
545                 550                 555                 560

Ser Phe Leu Gly Val Val Leu Phe Cys Leu Val Leu Leu Phe Leu Trp
                565                 570                 575

Ser Arg Gly Lys Gly Asn Thr Lys His Asn Ile Glu Ile Glu Tyr Val
            580                 585                 590

Pro Arg Lys Ser Asp Ala Gly Ile Ser Ser Ala Asp Ala Pro Arg Lys
        595                 600                 605

Phe Asn Met Lys Met Ile
    610

<210> 3
<211> 6
<212> PRT
<213> Homo sapiens

<400> 3

Met Gln Val Ser Lys Arg
1               5

<210>  4
<211>  200
<212>  DNA
<213>  Artificial Sequence

<220>
<223>  General Structure for an Oligonucleotide used in Preparation of
       an siRNA Molecule


<220>
<221>  misc_feature
<222>  (1)..(200)
<223>  n is a, c, g, t or u

<220>
<221>  misc_feature
<222>  (2)..(200)
<223>  nucleotide may be missing

<400>  4
nnnnnnnnnn nnnnnnnnnn nnnnnnnnnn nnnnnnnnnn nnnnnnnnnn nnnnnnnnnn      60

nnnnnnnnnn nnnnnnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn                                                  200


<210>  5
<211>  200
<212>  DNA
<213>  Artificial sequence

<220>
<223>  General Structure for an Oligonucleotide used in Preparation of
       an siRNA Molecule


<220>
<221>  misc_feature
<222>  (1)..(200)
<223>  n is a, c, g, t or u

<220>
<221>  misc_feature
<222>  (2)..(200)
<223>  nucleotide may be missing

<400>  5
nnnnnnnnnn nnnnnnnnnn nnnnnnnnnn nnnnnnnnnn nnnnnnnnnn nnnnnnnnnn      60

nnnnnnnnnn nnnnnnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn                                                  200

<210> 6
<211> 21
<212> DNA
<213> Artificial sequence

<220>
<223> Forward primer designed to determine genotype of Sp35 knockout
      mice

<400> 6
ctatccaagc actgcctgct c                                                21


<210> 7
<211> 23
<212> DNA
<213> Artificial Sequence

<220>
<223> Reverse primer designed to determine genotype of Sp35 knockout
      mice

<400> 7
gagttctagc tcctccaggt gtg                                              23


<210> 8
<211> 21
<212> DNA
<213> Artificial sequence

<220>
<223> Reverse primer designed to determine genotype of Sp35 knockout
      mice

<400> 8
gatgcccttc agctcgatgc g                                                21


<210> 9
<211> 55
<212> DNA
<213> Artificial sequence

<220>
<223> Sp35 RNAi sense oligonucleotide

<400> 9
tgatcgtcat cctgctagac ttcaagagag tctagcagga tgacgatctt ttttc          55


<210> 10
<211> 59
<212> DNA
<213> Artificial sequence

<220>
<223> Sp35 RNAi antisense oligonucleotide

<400> 10
tcgagaaaaa agatcgtcat cctgctagac tctcttgaag tctagcagga tgacgatca      59

<210>  11
<211>  59
<212>  DNA
<213>  Artificial sequence

<220>
<223>  Synthetic sequence designed for control RNAi sense
       oligonucleotide

<400>  11
tgatcctcat ccttctatac ttcaagagag tgtagcagga tgacgatctt ttttctcga       59


<210>  12
<211>  59
<212>  DNA
<213>  Artificial sequence

<220>
<223>  Synthetic sequence designed for control RNAi antisense
       oligonucleotide

<400>  12
tcgagaaaaa agatcgtcat cctgctagac tctcttgaag tatagaagga tgacgatca       59


<210>  13
<211>  55
<212>  RNA
<213>  Artificial sequence

<220>
<223>  Synthetic small-hairpin RNA (shRNA) sequence designed to compose
       Sp35 antagonist polynucleotide

<400>  13
ugaucgucau ccugcuagac uucaagagag ucuagcagga ugacgaucuu uuuuc           55


<210>  14
<211>  19
<212>  DNA
<213>  Artificial

<220>
<223>  Synthetic sequence for Sp35 Forward primer

<400>  14
agagacatgc gattggtga                                                   19


<210>  15
<211>  21
<212>  DNA
<213>  Artificial

<220>
<223>  Synthetic sequence for Sp35 Reverse primer

<400>  15
agagatgtag acgaggtcat t

## Description

### BACKGROUND OF THE INVENTION

### Field of the Invention.

This invention relates to neurology, neurobiology and molecular biology. More particularly, this invention relates to methods of promoting dopaminergic neurite regeneration, outgrowth and survival of dopaminergic neurons (DA neurons). Additionally, the invention relates to methods of treating conditions involving dopaminergic neuronal degeneration or death by the administration of Sp35 (LINGO-1) receptor antagonists.

### Background of the Invention

Certain neurodegenerative disorders are characterized by degeneration of dopaminergic neurons. For example, Parkinson's disease is associated with progressive destruction of dopaminergic neurons in the substantia nigra of the midbrain. This destruction results in reduced levels of the chemical transmitter dopamine. Physical symptoms of Parkinson's disease include impairment of voluntary movement and uncontrollable rhythmic twitching of groups of muscles producing characteristic shaking.

The most widely used treatment for Parkinson's disease is administration of a dopamine precursor, L-dopa (L-3, 4-dihydroxyphenylalanine), which acts indirectly by replacing the missing dopamine, after it is decarboxylated principally by the remaining dopamine neurons. However, disadvantages are associated with the use of L-dopa. Patients often suffer from side effects such as dyskinesia, nausea, vomiting, abdominal distension and psychiatric side effects and patients typically become less responsive to L-dopa treatment over time. One of the proposed reasons that L-dopa treatment becomes less effective over time is that the number of surviving dopaminergic neurons become depleted and thus cannot respond to the administered L-dopa. *See* Isacson O., "Problems and solutions for circuits and synapses in Parkinson's disease," Neuron 43: 165-168 (2004). Additionally, the L-dopa effectiveness decreases upon the continued loss of neuronal connections (terminals or synapses) due to the death of dopaminergic neurons. *See Id.* Alternative forms of therapy using postsynaptic dopamine agonists also are associated with side effects. Further, although L-dopa treatment improves quality of life for patients, it does not halt disease progression.

Other compounds, such as glial-cell-line-derived neurotrophic factor (GDNF), have shown promise in the treatment of Parkinson's disease in human patients when delivered by chronic infusion. *See, e. g*., Gill et al., "Direct brain infusion of glial cell line derived neurotrophic factor in Parkinson disease," Nature Med. 9: 589-95 (2003). However, these treatment regimens are still in the early stages of development.

Many other diseases and disorders may involve degeneration of dopaminergic neurons. These include multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervorden-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease, vascular parkinsonism, cerebral palsy, repeated head trauma, postencephalitic parkinsonism, schizophrenia and neurosyphilis.

Accordingly, there remains a need for additional treatment methods for Parkinson's disease and other conditions characterized by degeneration or death of dopaminergic neurons.

### BRIEF SUMMARY OF THE INVENTION

The present invention is based on the discovery that LINGO-1 is expressed in midbrain dopaminergic (DA) neurons and negatively regulates neurite outgrowth and survival of DA neurons. Based on these discoveries, the invention relates generally to methods for promoting proliferation, survival, repair, outgrowth and regeneration of dopaminergic neurons comprising contacting said dopaminergic neurons with an effective amount of a composition comprising an Sp35 antagonist. Additionally, the invention is related generally to methods of treating various diseases, disorders or injuries associated with dopaminergic neuronal degeneration or death by administration of an Sp35 antagonist.

In certain embodiments, the invention includes a method for promoting regeneration, outgrowth or survival of dopaminergic neurons in a mammal, comprising administering to a mammal in need thereof an effective amount of a composition comprising an Sp35 antagonist.

In additional embodiments, the mammal has been diagnosed with a disease, disorder, injury or condition involving dopaminergic neurite degeneration or death. In some embodiments, the disease, disorder, injury or condition is selected from the group consisting of Parkinson's disease (PD), multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervordern-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease (CJD), vascular parkinsonism, cerebral palsy, repeated head trauma, postencephalitic parkinsonism, neurosyphilis and schizophrenia.

In various embodiments of the above methods, the Sp35 antagonist may be any molecule which interferes with the ability of Sp35 to negatively regulate dopaminergic neuronal regeneration, outgrowth or survival. In certain embodiments, the Sp 35 antagonist is selected from the group consisting of a soluble Sp35 polypeptide, an Sp35 antibody or fragment thereof, an Sp35 antagonist polynucleotide (*e.g*. RNA interference), an Sp35 aptamer, or a combination of two or more Sp35 antagonists.

In certain embodiments, the Sp35 antagonist is a soluble Sp35 polypeptide. Certain soluble Sp35 polypeptides of the invention include, but are not limited to, soluble Sp35 polypeptides which comprise or lack one or more of the following domains: (i) an Sp35 Leucine-Rich Repeat (LRR) domain, (ii) an Sp35 basic region C-terminal to the LRR domain, and (iii) an Sp 35 immunoglobulin (Ig) domain. In some embodiments, the soluble Sp35 polypeptide lacks an Sp35 Ig domain, an Sp35 LRR domain, a transmembrane domain, and a cytoplasmic domain. Additional Sp35 soluble polypeptides of the invention include polypeptides which lack a transmembrane domain and a cytoplasmic domain. In some embodiments, the soluble Sp35 polypeptide comprises an Sp35 LRR domain and lacks an Sp35 Ig domain, an Sp35 basic region, a transmembrane domain, and a cytoplasmic domain. In some embodiments, the soluble Sp35 polypeptide comprises amino acid residues 34-532 of SEQ ID NO: 2 or 36-532 of SEQ ID NO:2.

In some embodiments, the Sp35 antagonist is a fusion polypeptide comprising a non-Sp35 moiety. In some embodiments, the non-Sp35 moiety is selected from the group consisting of an antibody Ig moiety, a serum albumin moiety, a targeting moiety, a reporter moiety, and a purification-facilitating inoiety. In some embodiments, the antibody Ig moiety is a hinge and Fc moiety.

In alternative embodiments, the Sp35 antagonist is an antibody or fragment thereof which binds to an Sp35 polypeptide comprising one or more of the following Sp35 domains: (i) an Sp35 Leucine-Rich Repeat (LRR) domain, (ii) an Sp35 basic region C-terminal to the LRR domain, and (iii) an Sp 35 immunoglobulin (Ig) domain. Additionally, the Sp35 antibody or fragment thereof specifically binds to an epitope within a polypeptide comprising an Sp35 polypeptide fragment as described herein.

In other embodiments, the Sp35 antagonist is an Sp35 antagonist polynucleotide such as an antisense polynucleotide, an aptamer, a ribozyme, a small interfering RNA (siRNA), or a small-hairpin RNA (shRNA).

In additional embodiments, the Sp35 antagonist is an Sp35 aptamer. An Sp35 aptamer is a small polypeptide or a polynucleotide which binds Sp35 and interferes with the ability of Sp35 to negatively regulate dopaminergic neuronal regneration, outgrowth and survival.

Further embodiments of the invention include a method for promoting regeneration, outgrowth and survival of dopaminergic neurons or a method of treating a disease, disorder or injury involving dopaminergic neurite degeneration or death by *in vivo* gene therapy, comprising administering to a mammal, at or near the site of the disease, disorder or injury, a nucleotide sequence that encodes an Sp35 antagonist so that the Sp35 antagonist is expressed from the nucleotide sequence in the mammal in an amount sufficient to reduce inhibition of dopaminergic neuronal regeneration, outgrowth or survival at or near the site of the injury. In certain embodiments, the vector is a viral vector which is selected from the group consisting of an adenoviral vector, an alphavirus vector, an enterovirus vector, a pestivirus vector, a lentiviral vector, a baculoviral vector, a herpesvirus vector, an Epstein Barr viral vector, a papovaviral vector, a poxvirus vector, a vaccinia viral vector, a parvovirus, and a herpes simplex viral vector. In some embodiments, the vector is administered by a route selected from the group consisting of topical administration, intraocular administration, parenteral administration, intrathecal administration, subdural administration and subcutaneous administration. In some embodiments, the disease, disorder, injury or condition is selected from the group consisting of Parkinson's disease (PD), multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervordern-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease (CJD), vascular parkinsonism, cerebral palsy, repeated head trauma, postencephalitic parkinsonism, neurosyphilis and schizophrenia.

Additionally, the invention includes a method for promoting regeneration, outgrowth and survival of dopaminergic neurons or a method of treating a disease, disorder or injury in a mammal involving dopaminergic neurite degeneration or death comprising (a) introducing into dopaminergic neurons a polynucleotide which encodes an Sp35 antagonist; and (b) allowing expression of said Sp35 antagonist. Additionally, the invention relates to a method comprising (a) administering to said mammal a polynucleotide which encodes an Sp35 antagonist through operable linkage to an expression control sequence and (b) allowing expression of said Sp35 antagonists. In some embodiments, the cultured host cell is derived from the mammal to be treated. In certain embodiments, the polynucleotide is introduced into the host cell or dopaminergic neuron via transfection, electroporation, viral transduction or direct microinjection. In certain embodiments, the disease, disorder, injury or condition to be treated is selected from the group consisting of Parkinson's disease (PD), multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervordern-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease (CJD), vascular parkinsonism, cerebral palsy, repeated head trauma, postencephalitic parkinsonism, neurosyphilis and schizophrenia.

In some embodiments, the polypeptides, aptamers and antibodies of the present invention are conjugated to a polymer. In some embodiments, the polymer is selected from the group consisting of a polyalkylene glycol, a sugar polymer, and a polypeptide. In some embodiments, the polyalkylene glycol is polyethylene glycol (PEG). In some embodiments, the polypeptides and antibodies of the present invention are conjugated to 1, 2, 3 or 4 polymers. In some embodiments, the total molecular weight of the polymers is from 5,000 Da to 100,000 Da.

### BRIEF DESCRIPTION OF THE DRAWINGS/FIGURES

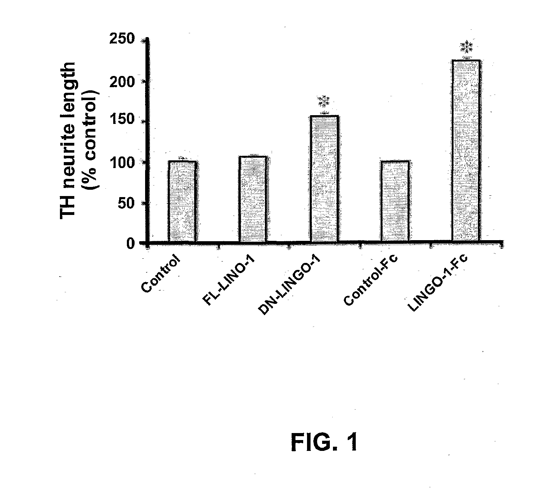

Figure 1: Graph showing dopaminergic neuronal outgrowth of primary rat DA neuronal cultures which have been transduced with vector control, FL-Sp35 (FL-LINGO-1) and DN-Sp35 (DN-LINGO-1) or have been treated with soluble Sp35-Fc (LINGO-1-Fc) protein as compared to control Fc without Sp35 protein (*p<0.003) and control lentivirus (*p<0.05).

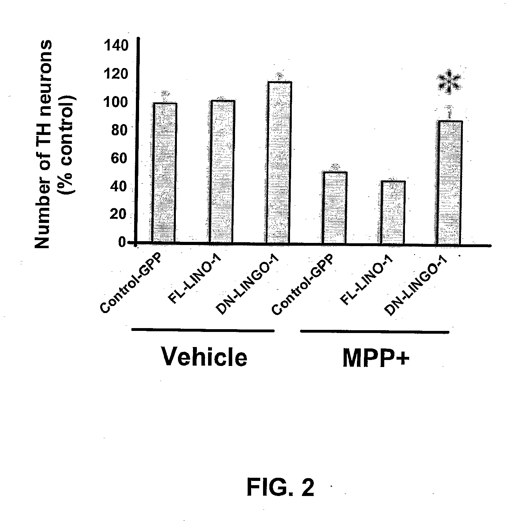

Figure 2: Graph showing survival of cultured rat ventral mesencephalon (VM) neurons infected with lentiviruses which produce the full-length Sp35 (FL-LINGO-1), dominant-negative Sp35 (DN-LINGO-1) or a control lentivirus and treated with 10µM 1-methyl-phenylpyridium ion (MPP+) or the just the vehicle composition used to administer the MPP+. When exposed to MPP+, the number of tyrosine hydrolase (TH) neurons incubated with DN-SP35 was significantly higher compared to FL-Sp35 and control (*p<0.05, One-way ANOVA).

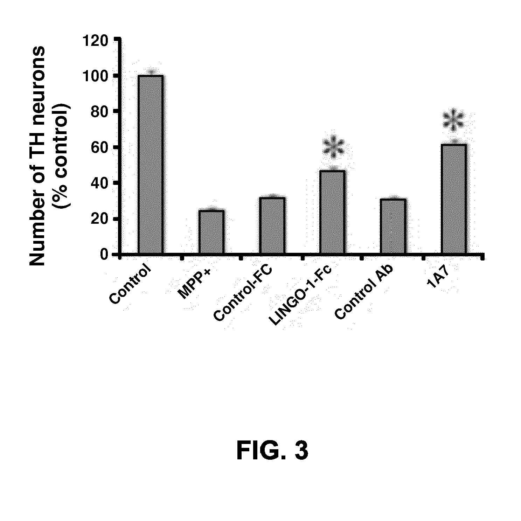

Figure 3: Graph showing TH neuron number of VM treated with Sp35-Fc (LINGO-1-Fc) or 1A7 Sp35 antagonist antibody and control Fc or control antibody. Treatment with Sp35-Fc or 1A7 resulted in a higher number of neurons when exposed to MPP+ as compared to control Fc or control antibody (*p<0.01, for 1A7 and *p<0.05 for Sp35-Fc, One-way ANOVA).

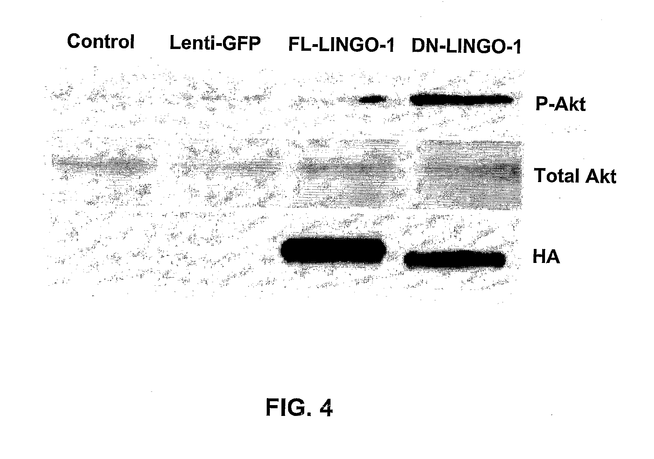

Figure 4: Western blot of phosphorylated Akt (p-Akt) in VM primary rat neuronal cultures after transduction of DN-LINGO-1, FL-LINGO-1 or a control. An increase in phosphorylated Akt is observed in cells transduced with DN-LINGO-1 when compared to FL-LINGO-1 and the control.

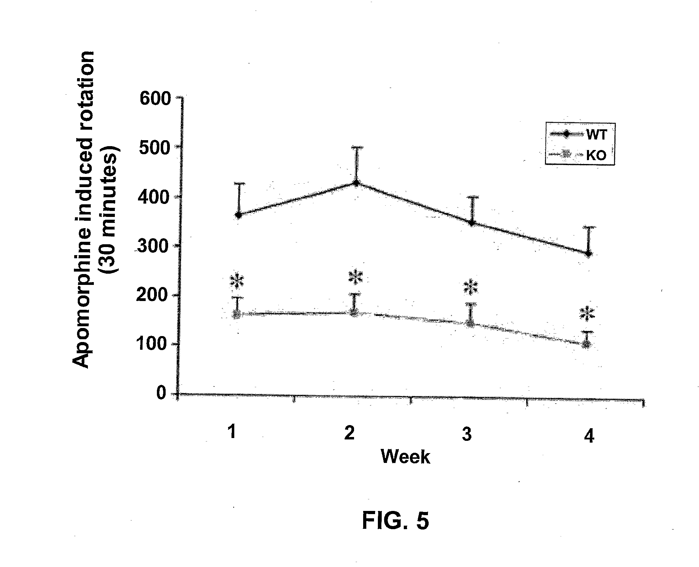

Figure 5: Graph demonstrating motor asymmetry in Sp35 knock-out mice compared to wild-type mice. Motor asymmetry was assessed 1, 2, 3 and 4 weeks after injecting 6-hydroxydopamine (6-OHDA) into the left striatum of wild-type (WT) and knock-out (KO) mice. Motor asymmetry was significantly lower in the knock-out mice compared to wild-type (*p=0.001, Two-way ANOVA).

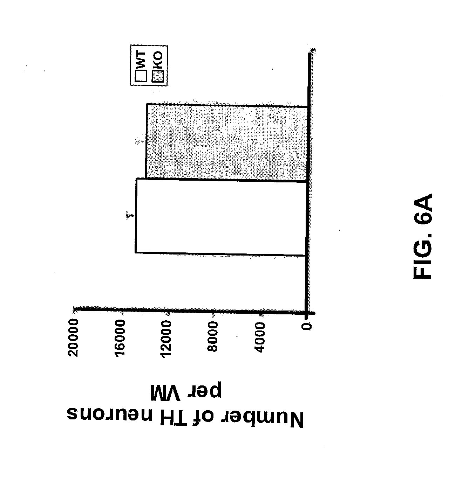

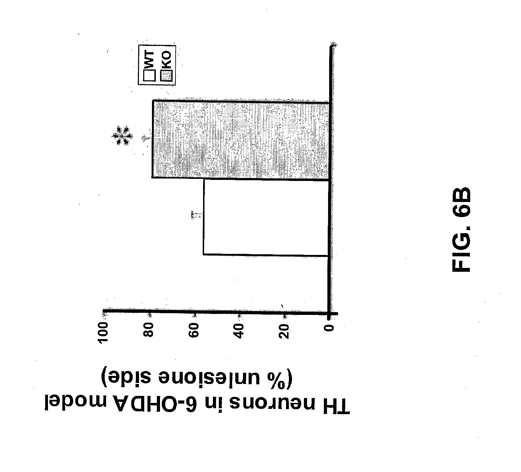

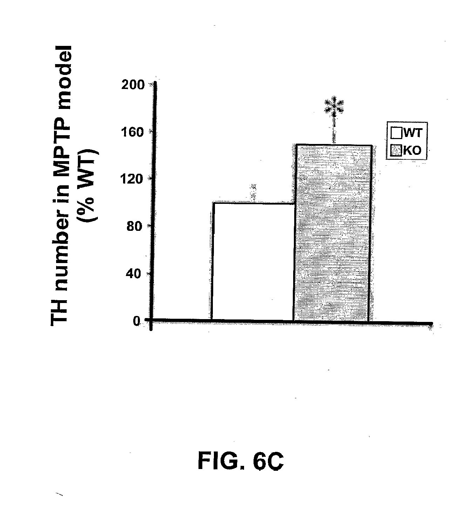

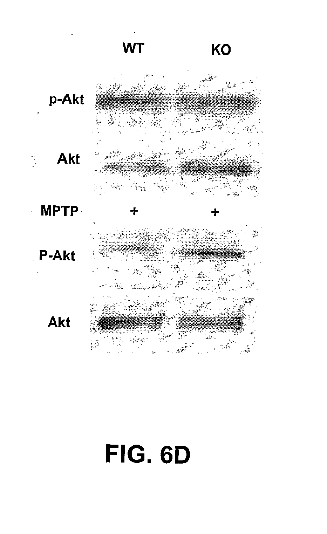

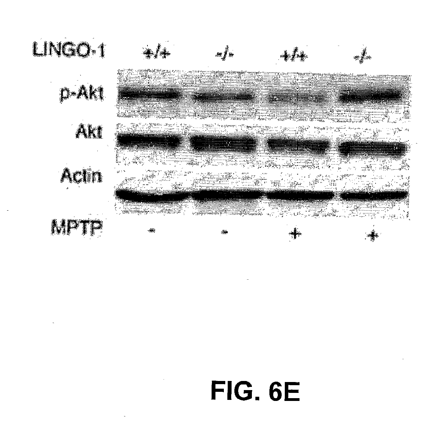

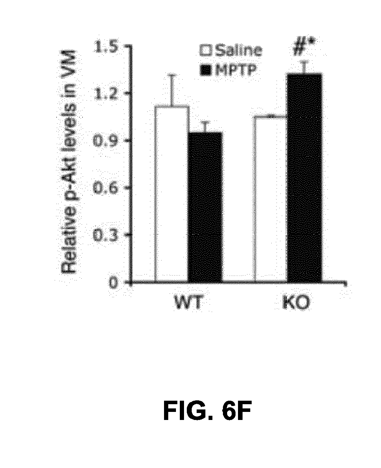

Figure 6A through 6F: Figure 6A is a graph showing the number of TH neurons in the unlesioned midbrains of wild-type and knock-out mice in the 6-OHDA experiment. Figure 6B is a graph showing the number of TH neurons in the lesioned side, normalized by the number in the controlateral unlesioned side of the knock-out mice and wild-type mice in the 6-OHDA experiment. The percentage of TH neurons was statistically greater in the KO than the WT mice (*p=0.001, unpaired t-test). Figure 6C is a graph showing the number of TH neurons in the midbrains of wild-type and knock-out mice in the 1-methyl-4-phenyl-1,2,3,6-tetrahydropyridine (MPTP) experiment. The percentage of TH neurons was statistically greater in the KO than the WT mice (*p=0.002, unpaired t-test). Figure 6D is a Western blot showing levels of phosphorylated Akt (P-Akt) in WT and KO mice after MPTP exposure compared to mice not exposed to MPTP. Figure 6E are Western blots of phosphorylated Akt (p-Akt), Akt and beta-actin in WT and KO VM treated with saline and MPTP (n=6 for each group). Figure 6F is a graph showing that p-Akt levels were signifcantly higher in MPTP treated mouse VMs at 7 days compared to MPTP treated WT (*p<0.05) and saline treated KO VMs (#p<0.05, n=6 for each group).

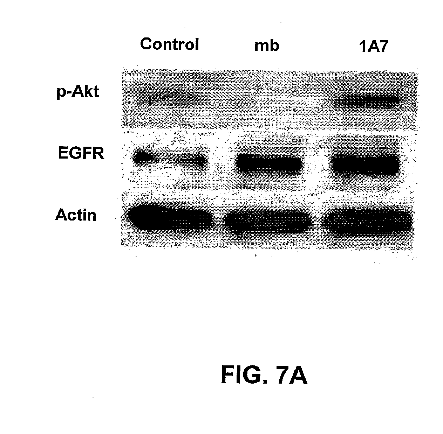

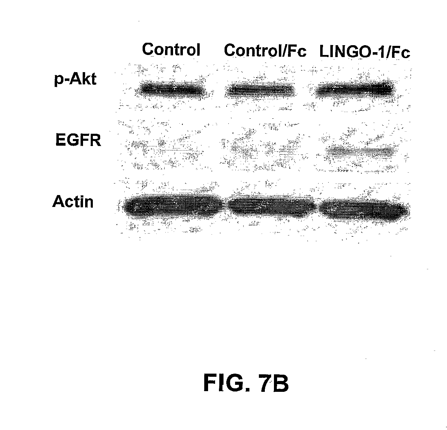

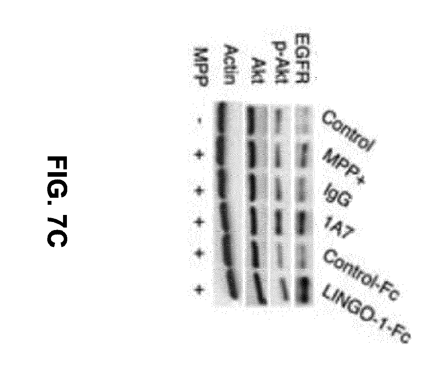

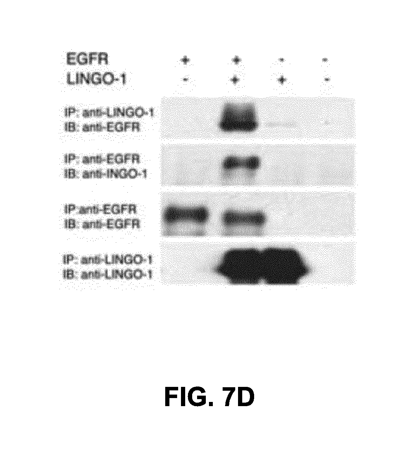

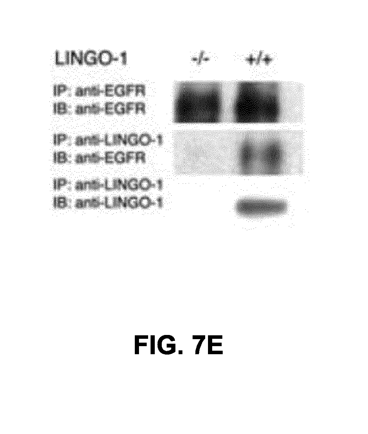

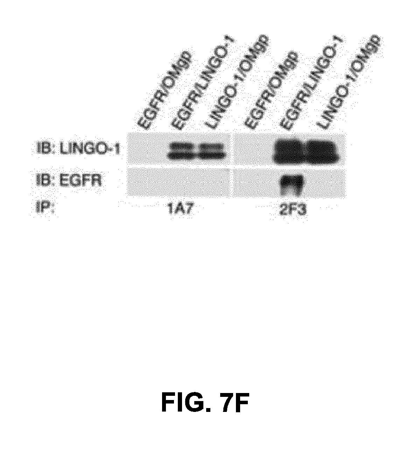

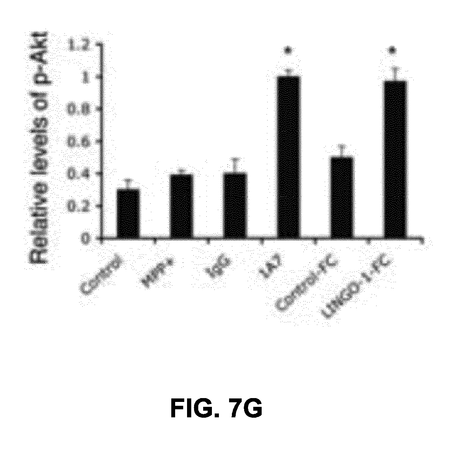

Figure 7A through 7G: Figure 7A is a Western blot of P-Akt and epidermal growth factor receptor (EGFR) expression of VM TH neurons treated with Sp35 antibody (1A7) or control antibodies. Figure 7B is a Western blot of EGFR and P-Akt expression of VM TH neurons treated with Sp35-Fc or controls. Figure 7C are Western blots of EGFR, p-Akt, total Akt and beta-actin of VM cultures treated with Sp35-Fc or Sp35 antibody (1A7). Figure 7D is a co-immunoprecipation of EGFR and Sp35 in cultured cells transfected with Sp35 and/or EGFR. + or - indicate the presence (+) or absence (-) of EGFR and Sp35. IP indicates the antibody used for the immunoprecipitation and IB indicates the antibody used for the Western blot. Figure 7E is a co-immunoprecipation of EGFR and LINGO-1 in the WT and KO VM. Figure 7F is a Western blot showing that the Sp35 antibody 1A7 blocks binding of Sp35 to EGFR in a co-transfected cell line. Additionally, another Sp35 antibody 2F3 does not block binding of Sp35 to EGFR. Transfection of oligodendrocyte-myelin glycoprotein (OMgp) was used as a control. Figure 7G is a graph showing the statistical analysis of p-Akt levels in 1A7 and Sp35-Fc treated cultures compared to control and Fc fragment, respectively.

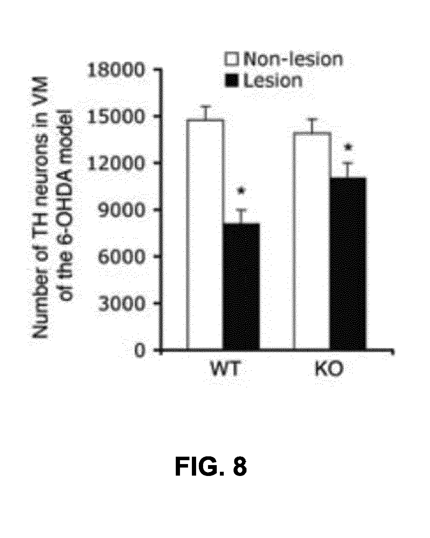

Figure 8: Graph showing the TH neuron number in non-lesioned and lesioned sides of the VM in WT and KO mice subjected to 6-OHDA induced experimental parkinsonism.

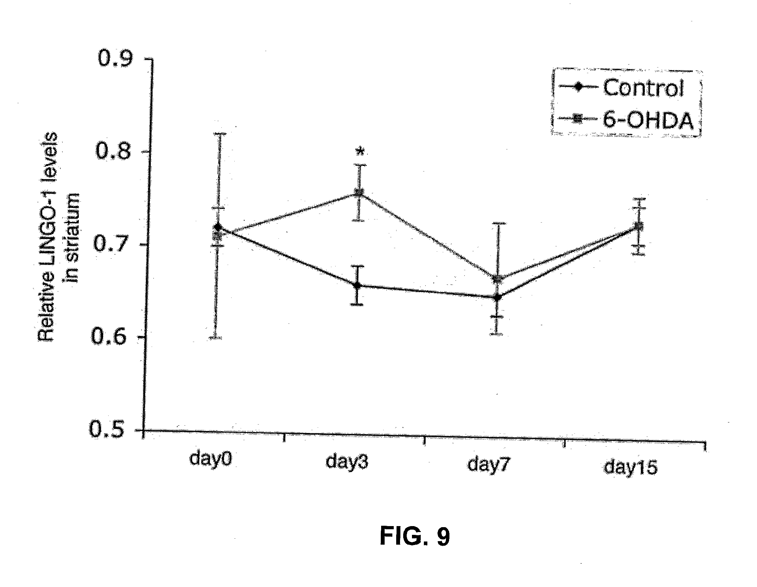

Figure 9: Graph showing the changes in Sp35 protein levels after 6-OHDA lesion. Sp35 is upregulated in the lesioned side (6-OHDA) compared to the contralateral side (control) 3-days after striatal 6-OHDA administration in wild-type mice (n=3, at each time point, unpaired Student's t-test, *p<0.05).

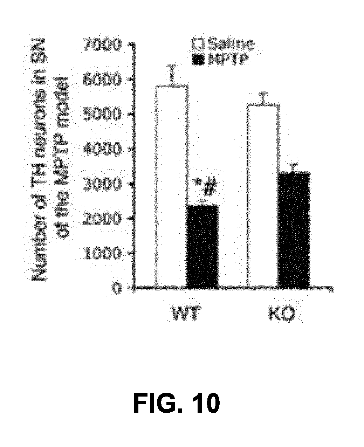

Figure 10: Graph showing the number of TH neurons in the substantial niagra compacta (SNc) of WT and KO mice treated with saline (WT: n=7, KO n=8) and MPTP (n=10 in each group).

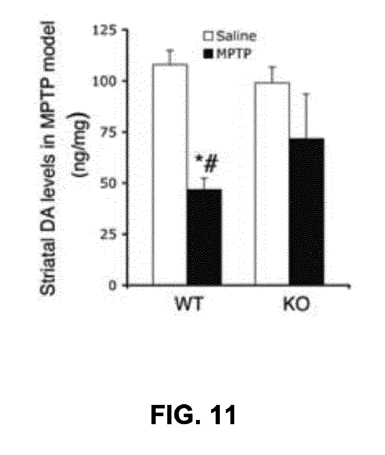

Figure 11: Graph showing the striatal dopamine (DA) levels (ng/ml) in KO and WT mice treated with saline or MPTP.

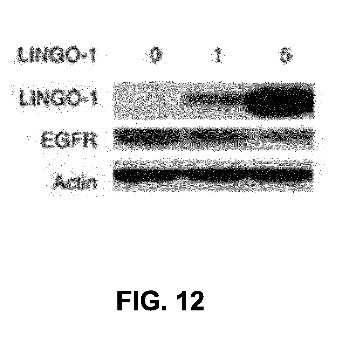

Figure 12: Western blot showing Sp35 and EGFR expression in COS-7 cells 2 days post transfection with a lentivirus expressing FL-Sp35 at 0, 1 and 5 MOI.

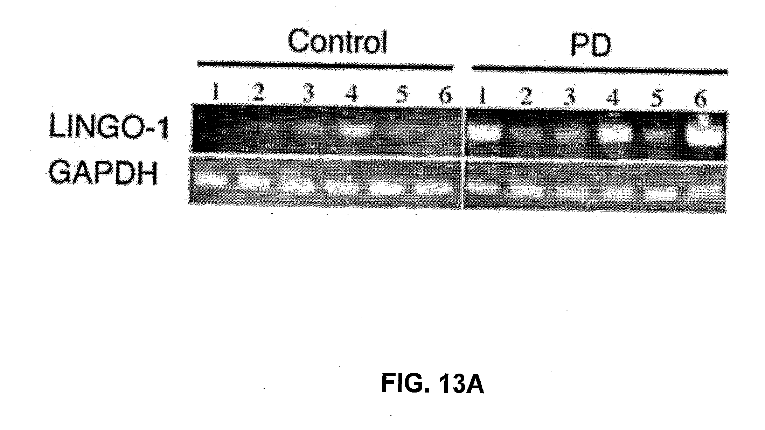

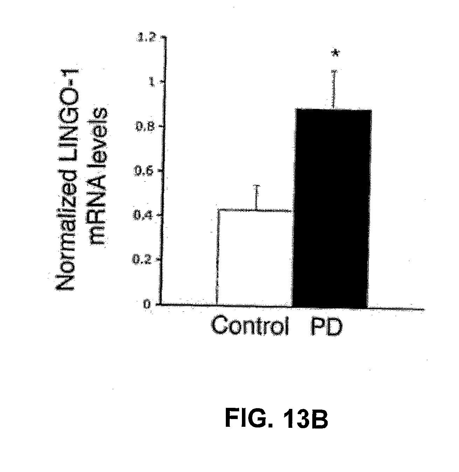

Figures 13A-13B: Figure 13A is a gel showing the results of a semi-quantitative PCR reaction showing significant elevation in Sp35 levels in the substantia nigra of Parkinson's disease patients (PD) compared to controls. Figure 13B is a graph showing the normalized mRNA levels of Sp35 in the substantia nigra of Parkinson's disease patients (PD) compared to controls.

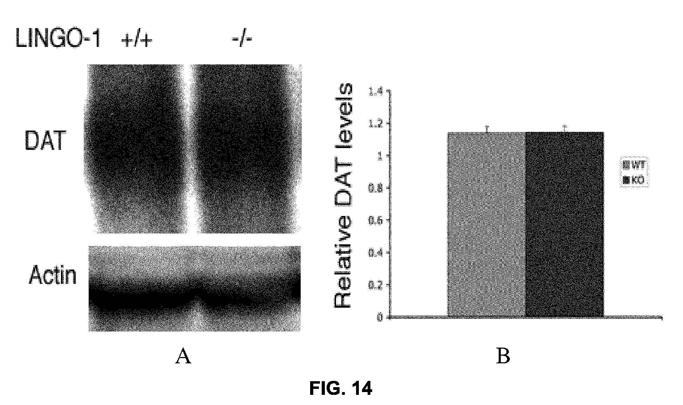

Figure 14A-B: Figure 14A is a Western blot showing the dopamine transport (DAT) levels in KO (-/-) and WT (+/+) mice (n=6 in each group). Figure 14B is a graph showing relative DAT levels in WT and KO mice. There is no significant difference in expression of DAT between WT and KO mice (unpaired Student's t-test, p>0.05).

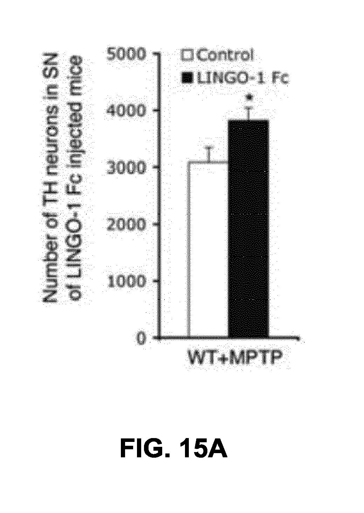

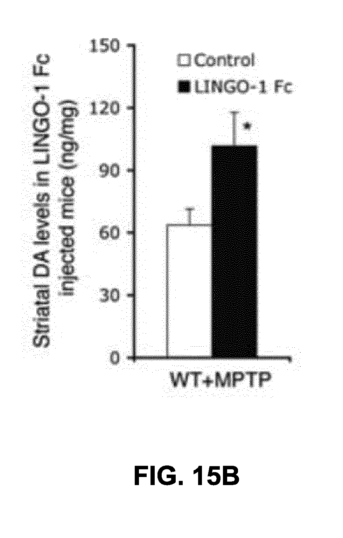

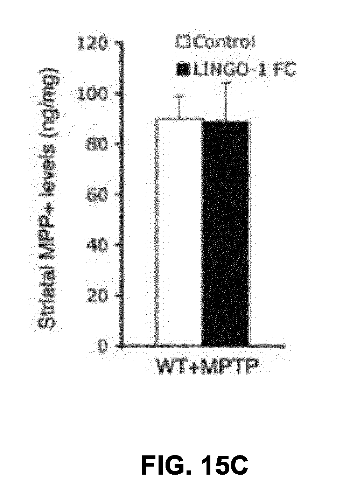

Figures 15A-15C: Figure 15A is a graph showing the number of TH neurons in the SNc of Sp-35-Fc injected mice compared to controls when exposed to MPTP (*p<0.05, n=9). Figure 15B is a graph showing the striatal dopamine levels in Sp35-Fc mice compared to controls when exposed to MPTP (*p<0.05, n=9). Figure 15C is a graph showing striatal MPP+ levels in Sp35-Fc injected mice compared to controls when exposed to MPTP.

### DETAILED DESCRIPTION OF THE INVENTION

### Definitions

Unless defined otherwise, all technical and scientific terms used herein have the same meaning as commonly understood by one of ordinary skill in the art to which this invention belongs. In case of conflict, the present application including the definitions will control. Unless otherwise required by context, singular terms shall include pluralities and plural terms shall include the singular. All publications, patents and other references mentioned herein are incorporated by reference in their entireties for all purposes as if each individual publication or patent application were specifically and individually indicated to be incorporated by reference.

Although methods and materials similar or equivalent to those described herein can be used in practice or testing of the present invention, suitable methods and materials are described below. The materials, methods and examples are illustrative only and are not intended to be limiting. Other features and advantages of the invention will be apparent from the detailed description and from the claims.

In order to further define this invention, the following terms and definitions are provided.

It is to be noted that the term "a" or "an" entity, refers to one or more of that entity; for example, "an immunoglobulin molecule," is understood to represent one or more immunoglobulin molecules. As such, the terms "a" (or "an"), "one or more," and "at least one" can be used interchangeably herein.

Throughout this specification and claims, the word "comprise," or variations such as "comprises" or "comprising," indicate the inclusion of any recited integer or group of integers but not the exclusion of any other integer or group of integers.

As used herein, a "therapeutically effective amount" refers to an amount effective, at dosages and for periods of time necessary, to achieve a desired therapeutic result. A therapeutic result may be, *e.g*., lessening of symptoms, prolonged survival, improved mobility, and the like. A therapeutic result need not be a "cure".

As used herein, the term "treatment" or "treating" refers to the administration of an agent to an animal in order to ameliorate or lessen the symptoms of a disease. Additionally, the terms "treatment" or "treating" refers to the administration of an agent to an animal to prevent the progression of a disease.

As used herein, a "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

As used herein, a "polynucleotide" can contain the nucleotide sequence of the full length cDNA sequence, including the untranslated 5' and 3' sequences, the coding sequences, as well as fragments, epitopes, domains, and variants of the nucleic acid sequence. The polynucleotide can be composed of any polyribonucleotide or polydeoxyribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotides can be composed of triple-stranded regions comprising RNA or DNA or both RNA and DNA. polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

In the present invention, a "polypeptide" can be composed of amino acids joined to each other by peptide bonds or modified peptide bonds, i.e., peptide isosteres, and may contain amino acids other than the 20 gene-encoded amino acids (*e.g*. non-naturally occuring amino acids). The polypeptides of the present invention may be modified by either natural processes, such as posttranslational processing, or by chemical modification techniques which are well known in the art. Such modifications are well described in basic texts and in more detailed monographs, as well as in a voluminous research literature. Modifications can occur anywhere in the polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (*See*, for instance, Proteins - Structure And Molecular Properties, 2nd Ed., T.E. Creighton, W.H. Freeman and Company, New York (1993); Posttranslational Covalent Modification of Proteins, B.C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

The terms' "fragment," "variant," "derivative" and "analog" when referring to an Sp35 antagonist of the present invention include any antagonist molecules which retain at least some ability to inhibit Sp35 activity. Sp35 antagonists as described herein may include fragment, variant, or derivative molecules therein without limitation, so long as the Sp35 antagonist still serves its function. Soluble Sp35 polypeptides of the present invention may include Sp35 proteolytic fragments, deletion fragments and in particular, fragments which more easily reach the site of action when delivered to an animal. Polypeptide fragments further include any portion of the polypeptide which comprises an antigenic or immunogenic epitope of the native polypeptide, including linear as well as three-dimensional epitopes. Soluble Sp35 polypeptides of the present invention may comprise variant Sp35 regions, including fragments as described above, and also polypeptides with altered amino acid sequences due to amino acid substitutions, deletions, or insertions. Variants may occur naturally, such as an allelic variant. By an "allelic variant" is intended alternate forms of a gene occupying a given locus on a chromosome of an organism. Genes II, Lewin, B., ed., John Wiley & Sons, New York (1985). Non-naturally occurring variants may be produced using art-known mutagenesis techniques. Soluble Sp35 polypeptides may comprise conservative or non-conservative amino acid substitutions, deletions or additions. Sp35 antagonists of the present invention may also include derivative molecules. For example, soluble Sp35 polypeptides of the present invention may include Sp35 regions which have been altered so as to exhibit additional features not found on the native polypeptide. Examples include fusion proteins and protein conjugates.

In the present invention, a "polypeptide fragment" refers to a short amino acid sequence of an Sp35 polypeptide. Protein fragments may be "free-standing," or comprised within a larger polypeptide of which the fragment forms a part or region. Representative examples of polypeptide fragments of the invention include, for example, fragments comprising about 5 amino acids, about 10 amino acids, about 15 amino acids, about 20 amino acids, about 30 amino acids, about 40 amino acids, about 50 amino acids, about 60 amino acids, about 70 amino acids, about 80 amino acids, about 90 amino acids, and about 100 amino acids or more in length.

In certain embodiments, Sp35 antagonists for use in the methods disclosed herein are "antibody" or "immunoglobulin" molecules, or immunospecific fragments thereof, e.g., naturally occurring antibody or immunoglobulin molecules or engineered antibody molecules or fragments that bind antigen in a manner similar to antibody molecules. The terms "antibody" and "immunoglobulin" are used interchangeably herein. Additionally, immunoglobulin molecules used in the methods of the invention are also described as "immunospecific" or "antigen-specific" or "antigen-binding" molecules and are used interchangeably to refer to antibody molecules and fragments thereof. An antibody or immunoglobulin comprises at least the variable domain of a heavy chain, and normally comprises at least the variable domains of a heavy chain and a light chain. Basic immunoglobulin structures in vertebrate systems are relatively well understood. *See, e.g.,* Harlow et al., Antibodies: A Laboratory Manual, (Cold Spring Harbor Laboratory Press, 2nd ed. 1988), incorporated herein by reference.

As will be discussed in more detail below, the term "immunoglobulin" comprises five broad classes of polypeptides that can be distinguished biochemically. All five classes are clearly within the scope of the present invention, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Both the light and heavy chains are divided into regions of structural and functional homology. The terms "constant" and "variable" are used functionally. In this regard, it will be appreciated that the variable domains of both the light (V_{L}) and heavy (V_{H}) chain portions determine antigen recognition and specificity. Conversely, the constant domains of the light chain (C_{L}) and the heavy chain (C_{H}1, C_{H}2 or C_{H}3) confer important biological properties such as secretion, transplacental mobility, Fc receptor binding, complement binding, and the like. By convention the numbering of the constant region domains increases as they become more distal from the antigen binding site or amino-terminus of the antibody. The N-terminal portion is a variable region and at the C-terminal portion is a constant region; the C_{H}3 and C_{L} domains actually comprise the carboxy-terminus of the heavy and light chain, respectively.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. Those skilled in the art will appreciate that heavy chains are classified as gamma, mu, alpha, delta, or epsilon, (γ, µ, α, δ, ε) with some subclasses among them (*e.g*., γ1-γ4). It is the nature of this chain that determines the "class" of the antibody as IgG, IgM, IgA IgG, or IgE, respectively. The immunoglobulin subclasses (isotypes) *e.g*., IgG₁, IgG₂, IgG₃, IgG₄, IgA₁, etc. are well characterized and are known to confer functional specialization. Modified versions of each of these classes and isotypes are readily discernable to the skilled artisan in view of the instant disclosure and, accordingly, are within the scope of the instant invention.

As indicated above, the variable region allows the antibody to selectively recognize and specifically bind epitopes on antigens. That is, the V_{L} domain and V_{H} domain of an antibody combine to form the variable region that defines a three dimensional antigen binding site. This quaternary antibody structure forms the antigen binding site present at the end of each arm of the Y. More specifically, the antigen binding site is defined by three complementary determining regions (CDRs) on each of the V_{H} and V_{L} chains. In some instances, e.g., certain immunoglobulin molecules derived from camelid species or engineered based on camelid immunoglobulins, a complete immunoglobulin molecule may consist of heavy chains only, with no light chains. *See, e.g.,* Hamers-Casterman et al., Nature 363:446-448 (1993).

In naturally occurring antibodies, the six "complementarity determining regions" or "CDRs" present in each antigen binding domain are short, non-contiguous sequences of amino acids that are specifically positioned to form the antigen binding domain as the antibody assumes its three dimensional configuration in an aqueous environment. The remainder of the amino acids in the antigen binding domains, referred to as "framework" regions, show less inter-molecular variability. The framework regions largely adopt a β-sheet conformation and the CDRs form loops which connect, and in some cases form part of, the β-sheet structure. Thus, framework regions act to form a scaffold that provides for positioning the CDRs in correct orientation by inter-chain, non-covalent interactions. The antigen binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined (*see,* "Sequences of Proteins of Immunological Interest," Kabat, E., et al., U.S. Department of Health and Human Services, (1983); and Chothia and Lesk, J. Mol. Biol., 196:901-917 (1987), which are incorporated herein by reference in their entireties).

In camelid species, however, the heavy chain variable region, referred to as V_{H}H, forms the entire CDR. The main differences between camelid V_{H}H variable regions and those derived from conventional antibodies (V_{H}) include (a) more hydrophobic amino acids in the light chain contact surface of V_{H} as compared to the corresponding region in V_{H}H, (b) a longer CDR3 in V_{H}H, and (c) the frequent occurrence of a disulfide bond between CDR1 and CDR3 in V_{H}H.

In one embodiment, an antigen binding molecule for use in the methods of the invention comprises at least one heavy or light chain CDR of an antibody molecule. In another embodiment, an antigen binding molecule for use in the methods of the invention comprises at least two CDRs from one or more antibody molecules. In another embodiment, an antigen binding molecule for use in the methods of the invention comprises at least three CDRs from one or more antibody molecules. In another embodiment, an antigen binding molecule for use in the methods of the invention comprises at least four CDRs from one or more antibody molecules. In another embodiment, an antigen binding molecule for use in the methods of the invention comprises at least five CDRs from one or more antibody molecules. In another embodiment, an antigen binding molecule for use in the methods of the invention comprises at least six CDRs from one or more antibody molecules. Exemplary antibody molecules comprising at least one CDR that can be included in the subject antigen binding molecules are known in the art and exemplary molecules are described herein.

Antibodies or immunospecific fragments thereof for use in the methods of the invention include, but are not limited to, polyclonal, monoclonal, multispecific, human, humanized, primatized, or chimeric antibodies, single chain antibodies, epitope-binding fragments, *e.g*., Fab, Fab' and F(ab')₂, Fd, Fvs, single-chain Fvs (scFv), single-chain antibodies, disulfide-linked Fvs (sdFv), fragments comprising either a V_{L} or V_{H} domain, fragments produced by a Fab expression library, and anti-idiotypic (anti-Id) antibodies (including, *e.g*., anti-Id antibodies to binding molecules disclosed herein). ScFv molecules are known in the art and are described, *e.g*., in US patent 5,892,019. Immunoglobulin or antibody molecules of the invention can be of any type (*e.g*., IgG, IgE, IgM, IgD, IgA, and IgY), class (*e.g*., IgG₁, TgG₂, IgG₃, IgG₄, IgA₁ and IgA₂) or subclass of immunoglobulin molecule.

Antibody fragments, including single-chain antibodies, may comprise the variable region(s) alone or in combination with the entirety or a portion of the following: hinge region, C_{H}1, C_{H}2, and C_{H}3 domains of the heavy chain, or C_{L} of the light chain. Also included in the invention are antigen-binding fragments also comprising any combination of variable region(s) with a hinge region, C_{H}1, C_{H}2, C_{H}3, or C_{L} domain. Antibodies or immunospecific fragments thereof for use in the methods disclosed herein may be from any animal origin including birds and mammals. Preferably, the antibodies are human, murine, donkey, rabbit, goat, guinea pig, camel, llama, horse, or chicken antibodies. In another embodiment, the variable region may be condricthoid in origin (*e.g*., from sharks). As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described *infra* and, for example, in U.S. Pat. No. 5,939,598 by Kucherlapati et al.

As used herein, the term "heavy chain portion" includes amino acid sequences derived from an immunoglobulin heavy chain. A polypeptide comprising a heavy chain portion comprises at least one of: a C_{H}1 domain, a hinge (*e.g*., upper, middle, and/or lower hinge region) domain, a C_{H}2 domain, a C_{H}3 domain, or a variant or fragment thereof. For example, a heavy chain portion may comprise a polypeptide chain comprising a C_{H}1 domain; a polypeptide chain comprising a C_{H}1 domain, at least a portion of a hinge domain, and a C_{H}2 domain; a polypeptide chain comprising a C_{H}1 domain and a C_{H}3 domain; a polypeptide chain comprising a C_{H}1 domain, at least a portion of a hinge domain, and C_{H}3 domain, or a polypeptide chain comprising a C_{H}1 domain, at least a portion of a hinge domain, a C_{H}2 domain, and a C_{H}3 domain. The heavy chain portion may also include a polypeptide comprising a polypeptide chain comprising a C_{H}3 domain. Further; a binding polypeptide for use in the invention may lack at least a portion of a C_{H}2 domain (*e.g*., all or part of a C_{H}2 domain). As set forth above, it will be understood by one of ordinary skill in the art that these domains (*e.g*., the heavy chain portions) may be modified such that they vary in amino acid sequence from the naturally occurring immunoglobulin molecule.

In certain Sp35 antagonist antibodies or immunospecific fragments thereof for use in the methods disclosed herein, the heavy chain portions of one polypeptide chain of a multimer are identical to those on a second polypeptide chain of the multimer. Alternatively, heavy chain portion-containing monomers for use in the methods of the invention are not identical. For example, each monomer may comprise a different target binding site, forming, for example, a bispecific antibody.

The heavy chain portions of a binding polypeptide for use in the methods disclosed herein may be derived from different immunoglobulin molecules. For example, a heavy chain portion of a polypeptide may comprise a C_{H}1 domain derived from an IgG₁ molecule and a hinge region derived from an IgG₃ molecule. In another example, a heavy chain portion can comprise a hinge region derived, in part, from an IgG₁ molecule and, in part, from an IgG₃ molecule. In another example, a heavy chain portion can comprise a chimeric hinge derived, in part, from an IgG₁ molecule and, in part, from an IgG₄ molecule.

As used herein, the term "light chain portion" includes amino acid sequences derived from an immunoglobulin light chain. Preferably, the light chain portion comprises at least one of a V_{L} or C_{L} domain.

An isolated nucleic acid molecule encoding a non-natural variant of a polypeptide derived from an immunoglobulin (*e.g*., an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. Preferably, conservative amino acid substitutions are made at one or more non-essential amino acid residues.

Antibodies or immunospecific fragments thereof for use in the methods disclosed herein may also be described or specified in terms of their binding affinity to a polypeptide of the invention. Preferred binding affinities include those with a dissociation constant or Kd less than 5 x 10⁻² M, 10⁻² M, 5 x 10⁻³ M, 10⁻³ M, 5 x 10⁻⁴ M, 10⁻⁴ M, 5 x 10⁻⁵ M, 10⁻⁵ M, 5 x 10⁻⁶ M, 10⁻⁶ M, 5 x 10⁻⁷ M, 10⁻⁷ M, 5 x 10⁻⁸ M, 10⁻⁸ M, 5 x 10⁻⁹ M, 10⁻⁹ M, 5 x 10⁻¹⁰ M, 10⁻¹⁰ M, 5 x 10⁻¹¹ M, 10⁻¹¹ M, 5 x 10⁻¹² M, 10⁻¹² M, 5 x 10⁻¹³ M, 10⁻¹³ M, 5 x 10⁻¹⁴ M, 10⁻¹⁴ M, 5 x 10⁻¹⁵ M, or 10⁻¹⁵ M.

Antibodies or immunospecific fragments thereof for use in the methods disclosed herein act as antagonists of Sp35 as described herein. For example, an antibody for use in the methods of the present invention may function as an antagonist, blocking or inhibiting the suppressive activity of the Sp35 polypeptide.

As used herein, the term "chimeric antibody" will be held to mean any antibody wherein the immunoreactive region or site is obtained or derived from a first species and the constant region (which may be intact, partial or modified in accordance with the instant invention) is obtained from a second species. In certain embodiments the target binding region or site will be from a non-human source (*e.g*. mouse or primate) and the constant region is human.

As used herein, the term "engineered antibody" refers to an antibody in which the variable domain in either the heavy and light chain or both is altered by at least partial replacement of one or more CDRs from an antibody of known specificity and, if necessary, by partial framework region replacement and sequence changing. Although the CDRs may be derived from an antibody of the same class or even subclass as the antibody from which the framework regions are derived, it is envisaged that the CDRs will be derived from an antibody of different class and preferably from an antibody from a different species. An engineered antibody in which one or more "donor" CDRs from a non-human antibody of known specificity is grafted into a human heavy or light chain framework region is referred to herein as a "humanized antibody." It may not be necessary to replace all of the CDRs with the complete CDRs from the donor variable region to transfer the antigen binding capacity of one variable domain to another. Rather, it may only be necessary to transfer those residues that are necessary to maintain the activity of the target binding site. Given the explanations set forth in, *e.g*., U. S. Pat. Nos. 5,585,089, 5,693,761, 5,693,762, and 6,180,370, it will be well within the competence of those skilled in the art, either by carrying out routine experimentation or by trial and error testing to obtain a functional engineered or humanized antibody.

As used herein, the terms "linked," "fused" or "fusion" are used interchangeably. These terms refer to the joining together of two more elements or components, by whatever means including chemical conjugation or recombinant means. An "in-frame fusion" refers to the joining of two or more open reading frames (ORFs) to form a continuous longer ORF, in a manner that maintains the correct reading frame of the original ORFs. Thus, the resulting recombinant fusion protein is a single protein containing two ore more segments that correspond to polypeptides encoded by the original ORFs (which segments are not normally so joined in nature.) Although the reading frame is thus made continuous throughout the fused segments, the segments may be physically or spatially separated by, for example, in-frame linker sequence.

In the context of polypeptides, a "linear sequence" or a "sequence" is an order of amino acids in a polypeptide in an amino to carboxyl terminal direction in which residues that neighbor each other in the sequence are contiguous in the primary structure of the polypeptide.

The term "expression" as used herein refers to a process by which a gene produces a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product and the translation of such mRNA into polypeptide(s). If the final desired product is biochemical, expression includes the creation of that biochemical and any precursors.

By "subject" or "individual" or "animal" or "patient" or "mammal," is meant any subject, particularly a mammalian subject, for whom diagnosis, prognosis, or therapy is desired. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. In certain embodiments, the mammal is a human subject.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

### Sp35 (LINGO-1/LRRN6)

The invention is based on the discovery that antagonists of Sp35 promote neurite outgrowth and survival of DA neurons. Naturally occurring human Sp35 is a glycosylated nervous-system -specific protein consisting of 614 amino acids (SEQ ID NO: 2). The human Sp35 polypeptide contains an LRR domain consisting of 14 leucine-rich repeats (including N- and C-terminal caps), an Ig domain, a transmembrane region, and a cytoplasmic domain. The cytoplasmic domain contains a canonical tyrosine phosphorylation site. In addition, the naturally occurring Sp35 protein contains a signal sequence, a short basic region between the LRRCT and Ig domain, and a transmembrane region between the Ig domain and the cytoplasmic domain. The human Sp35 gene contains alternative translation start codons, so that six additional amino acids (MQVSKR; SEQ ID NO:3) may or may not be present at the N-terminus of the Sp35 signal sequence. Table 1 lists the Sp35 domains and other regions, according to amino acid residue number, based on the sequence of SEQ ID NO:2.

**Table 1**

| **Domain or Region** | **Beginning Residue** | **Ending Residue** |
|---|---|---|
| Signal Sequence | 1 | 33 or 35 |
| LRRNT | 34 or 36 | 64 |
| LRR | 66 | 89 |
| LRR | 90 | 113 |
| LRR | 114 | 137 |
| LRR | 138 | 161 |
| LRR | 162 | 185 |
| LRR | 186 | 209 |
| LRR | 210 | 233 |
| LRR | 234 | 257 |
| LRR | 258 | 281 |
| LRR | 282 | 305 |
| LRR | 306 | 329 |
| LRR | 330 | 353 |
| LRRCT | 363 | 414 or 416 |
| Basic | 415 or 417 | 424 |
| Ig | 419 | 493 |
| Connecting sequence | 494 | 551 |
| Transmembrane | 552 | 576 |
| Cytoplasmic | 577 | 614 |

Tissue distribution and developmental expression of Sp35 have been studied in humans and rats. Sp35 biology has been studied in an experimental animal (rat) model. Expression of rat Sp35 is localized to nervous-system neurons and brain oligodendrocytes, as determined by northern blot and immuno-histochemical staining. Rat Sp35 mRNA expression level is regulated developmentally, peaking shortly after birth, i.e., ca. postnatal day one. In a rat spinal cord transection injury model, Sp35 is up-regulated at the injury site, as determined by RT-PCR. In addition, Sp35 has been shown to interact with Nogo66 Receptor (Nogo receptor). See, e.g., International Patent Application No. PCT/US2004/00832, PCT Publication No. WO2004/08564.

Sp35 (LINGO-1) is an additional component of the Nogo Receptor-1-p75 neurotrophin receptor complex. See Mi et al., Nat Neurosci. 7:221-228 (2004), which is incorporated herein by reference. Unlike Nogo receptor 1, Sp35 gene expression is increased when adult nerve cells in the spinal cord are exposed to traumatic injuries, suggesting that Sp35 has an important biological role for CNS neurological function. *Id*.

The nucleotide sequence for the full-length Sp35 molecule is as follows:

ATGCTGGCGGGGGCGTGAGGAGCATGCCCAGCCCCCTCCTGGCCTGCTGGCAGCCCATCCTCCTGCTGGTG
CTGGGCTCAGTGCTGTCAGGCTCGGCCACGGGCTGCCCGCCCCGCTGCGAGTGCTCCGCCCAGGACCGCGCT
GTGCTGTGCCACCGCAAGCGCTTTGTGGCAGTCCCCGAGGGCATCCCCACCGAGACGCGCCTGCTGGACCTA
GGCAAGAACCGCATCAAAACGCTCAACCAGGACGAGTTCGCCAGCTTCCCGCACCTGGAGGAGCTGGAGCTC
AACGAGAACATCGTGAGCGCCGTGGAGCCCGGCGCCTTCAACAACCTCTTCAACCTCCGGACGCTGGGTCTC
CGCAGCAACCGCCTGAAGCTCATCCCGCTAGGCGTCTTCACTGGCCTCAGCAACCTGACCAAGCTGGACATC
AGCGAGAACAAGATTGTTATCCTGCTGGACTACATGTTTCAGGACCTGTACAACCTCAAGTCACTGGAGGTT
GGCGACAATGACCTCGTCTACATCTCTCACCGCGCCTTCAGCGGCCTCAACAGCCTGGAGCAGCTGACGCTG
GAGAAATGCAACCTGACCTCCATCCCCACCGAGGCGCTGTCCCACCTGCACGGCCTCATCGTCCTGAGGCTC
CGGCACCTCAACATCAATGCCATCCGGGACTACTCCTTCAAGAGGCTCTACCGACTCAAGGTCTTGGAGATC
TCCCACTGGCCCTACTTGGACACCATGACACCCAACTGCCTCTACGGCCTCAACCTGACGTCCCTGTCCATC
ACACACTGCAATCTGACCGCTGTGCCCTACCTGGCCGTCCGCCACCTAGTCTATCTCCGCTTCCTCAACCTC
TCCTACAACCCCATCAGCACCATTGAGGGCTCCATGTTGCATGAGCTGCTCCGGCTGCAGGAGATCCAGCTG
GTGGGCGGGCAGCTGGCCGTGGTGGAGCCCTATGCCTTCCGCGGCCTCAACTACCTGCGCGTGCTCAATGTC
TCTGGCAACCAGCTGACCACACTGGAGGAATCAGTCTTCCACTCGGTGGGCAACCTGGAGACACTCATCCTG
GACTCCAACCCGCTGGCCTGCGACTGTCGGCTCCTGTGGGTGTTCCGGCGCCGCTGGCGGCTCAACTTCAAC
CGGCAGCAGCCCACGTGCGCCACGCCCGAGTTTGTCCAGGGCAAGGAGTTCAAGGACTTCCCTGATGTGCTA
CTGCCCAACTACTTCACCTGCCGCCGCGCCCGCATCCGGGACCGCAAGGCCCAGCAGGTGTTTGTGGACGAG
GGCCACACGGTGCAGTTTGTGTGCCGGGCCGATGGCGACCCGCCGCCCGCCATCCTCTGGCTCTCACCCCGA
AAGCACCTGGTCTCAGCCAAGAGCAATGGGCGGCTCACAGTCTTCCCTGATGGCACGCTGGAGGTGCGCTAC
GCCCAGGTACAGGACAACGGCACGTACCTGTGCATCGCGGCCAACGCGGGCGGCAACGACTCCATGCCCGCC
CACCTGCATGTGCGCAGCTACTCGCCCGACTGGCCCCATCAGCCCAACAAGACCTTCGCTTTCATCTCCAAC
CAGCCGGGCGAGGGAGAGGCCAACAGCACCCGCGCCACTGTGCCTTTCCCCTTCGACATCAAGACCCTCATC
ATCGCCACCACCATGGGCTTCATCTCTTTCCTGGGCGTCGTCCTCTTCTGCCTGGTGCTGCTGTTTCTCTGG
AGCCGGGGCAAGGGCAACACAAAGCACAACATCGAGATCGAGTATGTGCCCCGAAAGTCGGACGCAGGCATC
AGCTCCGCCGACGCGCCCCGCAAGTTCAACATGAAGATGATATGA (SEQ ID NO:1).

The polypeptide sequence for the full-length Sp35 polypeptide is as follows:

MLAGGVRSMPSPLLACWQPILLLVLGSVLSGSATGCPPRCECSAQDRAVLCHRKRFVAVPEGIPTETRLLDL
GKNRIKTLNQDEFASFPHLEELELNENIVSAVEPGAFNNLFNLRTLGLRSNRLKLIPLGVFTGLSNLTKLDI
SENKIVILLDYMFQDLYNLKSLEVGDNDLVYISHRAFSGLNSLEQLTLEKCNLTSIPTEALSHLHGLIVLRL
RHLNINAIRDYSFKRLYRLKVLEISHWPYLDTMTPNCLYGLNLTSLSITHCNLTAVPYLAVRHLVYLRFLNL
SYNPISTIEGSMLHELLRLQEIQLVGGQLAVVEPYAFRGLNYLRVLNVSGNQLTTLEESVFHSVGNLETLIL
DSNPLACDCRLLWVFRRRWRLNFNRQQPTCATPEFVQGKEFKDFPDVLLPNYFTCRRARIRDRKAQQVFVDE
GHTVQFVCRADGDPPPAILWLSPRKHLVSAKSNGRLTVFPDGTLEVRYAQVQDNGTYLCIAANAGGNDSMPA
HLHVRSYSPDWPHQPNKTFAFISNQPGEGEANSTRATVPFPFDIKTLIIATTMGFISFLGVVLFCLVLLFLW
SRGKGNTKHNIEIEYVPRKSDAGISSADAPRKFNMKMI (SEQ ID NO:2).

### Methods Using Antagonists of Sp35

One embodiment of the present invention provides methods for promoting regeneration, outgrowth or survival of dopaminergic (DA) neurons comprising contacting DA neurons with an effective amount of an Sp35 antagonist, or a composition comprising an Sp35 antagonist, where the Sp35 antagonist is selected from the group consisting of a soluble Sp35 polypeptide, an Sp35 antibody, an Sp35 antagonist polynucleotide, an Sp35 aptamer, and a combination of two or more of said Sp35 antagonists. Various exemplary Sp35 antagonists and methods and materials for obtaining these molecules for practicing the present invention are described below and/or may be found, *e.g*., in International Patent Application No. PCT/US2004/008323, PCT Publication No. WO2004/085648, incorporated herein by reference in its entirety. Sp35 receptor antagonists useful for the invention include, for example, those described in PCT/US2005/022881, PCT Publication No. WO2006/002437, incorporated herein by reference in its entirety.

An additional embodiment of the present invention provides methods for treating a disease, disorder or injury associated with DA neuronal degeneration or death, (e.g., Parkinson's disease) in an animal (e.g. a mammal) suffering from such disease, the method comprising, consisting essentially of, or consisting of administering to the animal in need thereof a therapeutically effective amount of an Sp35 antagonist, or composition comprising an Sp35 antagonist, selected from the group consisting of a soluble Sp35 polypeptide, an Sp35 antibody, an Sp35 antagonist polynucleotide, an Sp35 aptamer and a combination of two or more of said Sp35 antagonists.

Further embodiments of the invention include a method of promoting DA neuronal regeneration, outgrowth or survival to treat a disease, disorder or injury associated with DA neuronal death comprising administering to a mammal, at or near the site of the disease, disorder or injury, in an amount sufficient to reduce inhibition of regeneration, outgrowth or survival of DA neurons.

In methods of the present invention, an Sp35 antagonist can be administered via direct administration of a soluble Sp35 polypeptide, Sp35 antibody, Sp35 antagonist polynucleotide, Sp35 aptamer, or combinations thereof to the patient. Alternatively, the Sp35 antagonist can be administered via an expression vector which produces the specific Sp35 antagonist. In certain embodiments of the invention, an Sp35 antagonist is administered in a treatment method that includes: (1) transforming or transfecting an implantable host cell with a nucleic acid, e.g., a vector, that expresses an Sp35 antagonist; and (2) implanting the transformed host cell into a mammal, at the site of a disease, disorder or injury. For example, the transformed host cell can be implanted at certain affected sites of the source of dopamine neurons, such as the midbrain, or their targets of connections, such as putamen, caudate, cortex, globus pallidus or subthalamic nucleus. In some embodiments of the invention, the implantable host cell is removed from a mammal, temporarily cultured, transformed or transfected with an isolated nucleic acid encoding an Sp35 antagonist, and implanted back into the same mammal from which it was removed. The cell can be, but is not required to be, removed from the same site at which it is implanted. Such embodiments, sometimes known as ex vivo gene therapy, can provide a continuous supply of the Sp35 antagonist, localized at the site of action, for a limited period of time.

Diseases or disorders which may be treated or ameliorated by the methods of the present invention include diseases, disorders or injuries which relate to the death, degeneration or lack of regeneration or differentiation of DA neurons. Such diseases include, but are not limited to, Parkinson's disease (PD), multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervordern-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease (CJD), vascular parkinsonism, cerebral palsy, repeated head trauma, postencephalitic parkinsonism, neurosyphilis and schizophrenia.

An Sp35 antagonist, e.g., a soluble Sp35 polypeptide, an Sp35 antibody, an Sp35 antagonist polynucleotide, or an Sp35 aptamer, to be used in methods disclosed herein, can be prepared and used as a therapeutic agent that stops, reduces, prevents, or inhibits the ability of Sp35 to negatively regulate DA neurite outgrowth, survival or regeneration.

### Soluble Sp35 Polypeptides

Sp35 antagonists to be used in the methods of the present invention include those polypeptides which block, inhibit or interfere with the biological function of naturally occurring Sp35. Specifically, soluble Sp35 polypeptides of the present invention include fragments, variants, or derivative thereof of a soluble Sp35 polypeptide. Table 1 above describes the various domains of the Sp35 polypeptide. Soluble Sp35 polypeptides lack the transmembrane domain and typically lack the intracellular domain of the Sp35 polypeptide. For example, certain soluble Sp35 polypeptides lack amino acids 552-576 which comprise the transmembrane domain of Sp35 and/or amino acids 577-614 which comprise the intracellular domain of Sp35. Additionally, certain soluble Sp35 polypeptides comprise the LRR domains, Ig domain, basic region and/or the entire extracellular domain (corresponding to amino acids 34 to 532 of SEQ ID NO: 2) of the Sp35 polypeptide. As one of skill in the art would appreciate, the entire extracellular domain of Sp35 may comprise additional or fewer amino acids on either the C-terminal or N-terminal end of the extracellular domain polypeptide. As such, soluble Sp35 polypeptides for use in the methods of the present invention include, but are not limited to, an Sp35 polypeptide comprising, consisting essentially of, or consisting of amino acids 41 to 525 of SEQ ID NO:2; 40 to 526 of SEQ ID NO:2; 39 to 527 of SEQ ID NO:2; 38 to 528 of SEQ ID NO:2; 37 to 529 of SEQ ID NO:2; 36 to 530 of SEQ ID NO:2; 35 to 531 of SEQ ID NO:2; 34 to 531 of SEQ ID NO:2; 46 to 520 of SEQ ID NO:2; 45 to 521 of SEQ ID NO:2; 44 to 522 of SEQ ID NO:2; 43 to 523 of SEQ ID NO:2; and 42 to 524 of SEQ ID NO:2 or fragments, variants, or derivatives of such polypeptides. Sp35 polypeptide antagonists may include any combination of domains as described in Table 1.

Additional soluble Sp35 polypeptides for use in the methods of the present invention include, but are not limited to, an Sp35 polypeptide comprising, consisting essentially of, or consisting of amino acids 1 to 33 of SEQ ID NO:2; 1 to 35 of SEQ ID NO:2; 34 to 64 of SEQ ID NO:2; 36 to 64 of SEQ ID NO:2; 66 to 89 of SEQ ID NO:2; 90 to 113 of SEQ ID NO:2; 114 to 137 of SEQ ID NO:2; 138 to 161 of SEQ ID NO:2; 162 to 185 of SEQ ID NO:2; 186 to 209 of SEQ ID NO:2; 210 to 233 of SEQ ID NO:2; 234 to 257 of SEQ ID NO:2; 258 to 281 of SEQ ID NO:2; 282 to 305 of SEQ ID NO:2; 306 to 329 of SEQ ID NO:2; 330 to 353 of SEQ ID NO:2; 363 to 416 of SEQ ID NO:2; 417 to 424 of SEQ ID NO:2; 419 to 493 of SEQ ID NO:2; and 494 to 551 of SEQ ID NO:2 or fragments, variants, or derivatives of such polypeptides.

Further soluble Sp35 polypeptides for use in the methods of the present invention include, but are not limited to, an Sp35 polypeptide comprising, consisting essentially of, or consisting of amino acids 1 to 33 of SEQ ID NO:2; 1 to 35 of SEQ ID NO:2; 1 to 64 of SEQ ID NO:2; 1 to 89 of SEQ ID NO:2; 1 to 113 of SEQ ID NO:2; 1 to 137 of SEQ ID NO:2; 1 to 161 of SEQ ID NO:2; 1 to 185 of SEQ ID NO:2; 1 to 209 of SEQ ID NO:2; 1 to 233 of SEQ ID NO:2; 1 to 257 of SEQ ID NO:2; 1 to 281 of SEQ ID NO:2; 1 to 305 of SEQ ID NO:2; 1 to 329 of SEQ ID NO:2; 1 to 353 of SEQ ID NO:2; 1 to 416 of SEQ ID NO:2; 1 to 424 of SEQ ID NO:2; 1 to 493 of SEQ ID NO:2; 1 to 551 of SEQ ID NO:2; 1 to 531 of SEQ ID NO:2 and 1 to 532 of SEQ ID NO:2 or fragments, variants, or derivatives of such polypeptides.

Still further soluble Sp35 polypeptides for use in the methods of the present invention include, but are not limited to, an Sp35 polypeptide comprising, consisting essentially of, or consisting of amino acids 34 to 64 of SEQ ID NO:2; 34 to 89 of SEQ ID NO:2; 34 to 113 of SEQ ID NO:2; 34 to 137 of SEQ ID NO:2; 34 to 161 of SEQ ID NO:2; 34 to 185 of SEQ ID NO:2; 34 to 209 of SEQ ID NO:2; 34 to 233 of SEQ ID NO:2; 34 to 257 of SEQ ID NO:2; 34 to 281 of SEQ ID NO:2; 34 to 305 of SEQ ID NO:2; 34 to 329 of SEQ ID NO:2; 34 to 353 of SEQ ID NO:2; 34 to 416 of SEQ ID NO:2; 34 to 424 of SEQ ID NO:2; 34 to 493 of SEQ ID NO:2; and 34 to 551 of SEQ ID NO:2 or fragments, variants, or derivatives of such polypeptides.

Additional soluble Sp35 polypeptides for use in the methods of the present invention include, but are not limited to, an Sp35 polypeptide comprising, consisting essentially of, or consisting of amino acids 34 to 530 of SEQ ID NO:2; 34 to 531 of SEQ ID NO:2; 34 to 532 of SEQ ID NO:2; 34 to 533 of SEQ ID NO:2; 34 to 534 of SEQ ID NO:2; 34 to 535 of SEQ ID NO:2; 34 to 536 of SEQ ID NO:2; 34 to 537 of SEQ ID NO:2; 34 to 538 of SEQ ID NO:2; 34 to 539 of SEQ ID NO:2; 30 to 532 of SEQ ID NO:2; 31 to 532 of SEQ ID NO:2; 32 to 532 of SEQ ID NO:2; 33 to 532 of SEQ ID NO:2; 34 to 532 of SEQ ID NO:2; 35 to 532 of SEQ ID NO:2; 36 to 532 of SEQ ID NO:2; 30 to 531 of SEQ ID NO:2; 31 to 531 of SEQ ID NO:2; 32 to 531 of SEQ ID NO:2; 33 to 531 of SEQ ID NO:2; 34 to 531 of SEQ ID NO:2; 35 to 531 of SEQ ID NO:2; and 36 to 531 of SEQ ID NO:2 or fragments, variants, or derivatives of such polypeptides.

Still further soluble Sp35 polypeptides for use in the methods of the present invention include, but are not limited to, an Sp35 polypeptide comprising, consisting essentially of, or consisting of amino acids 36 to 64 of SEQ ID NO:2; 36 to 89 of SEQ ID NO:2; 36 to 113 of SEQ ID NO:2; 36 to 137 of SEQ ID NO:2; 36 to 161 of SEQ ID NO:2; 36 to 185 of SEQ ID NO:2; 36 to 209 of SEQ ID NO:2; 36 to 233 of SEQ ID NO:2; 36 to 257 of SEQ ID NO:2; 36 to 281 of SEQ ID NO:2; 36 to 305 of SEQ ID NO:2; 36 to 329 of SEQ ID NO:2; 36 to 353 of SEQ ID NO:2; 36 to 416 of SEQ. ID NO:2; 36 to 424 of SEQ ID NO:2; 36 to 493 of SEQ ID NO:2; and 36 to 551 of SEQ ID NO:2 or fragments, variants, or derivatives of such polypeptides.

Additional soluble Sp35 polypeptides for use in the methods of the present invention include, but are not limited to, an Sp35 polypeptide comprising, consisting essentially of, or consisting of amino acids 36 to 530 of SEQ ID NO:2; 36 to 531 of SEQ ID NO:2; 36 to 532 of SEQ ID NO:2; 36 to 533 of SEQ ID NO:2; 36 to 534 of SEQ ID NO:2; 36 to 535 of SEQ ID NO:2; 36 to 536 of SEQ ID NO:2; 36 to 537 of SEQ ID NO:2; 36 to 538 of SEQ ID NO:2; and 36 to 539 of SEQ ID NO:2; or fragments, variants, or derivatives of such polypeptides.

Additional soluble Sp35 polypeptides, fragments, variants or derivatives thereof include polypeptides comprising the Ig domain of Sp35. For example, an Sp35 polypeptide comprising, consisting essentially of, or consisting of amino acids 417 to 493 of SEQ ID NO:2; 417 to 494 of SEQ ID NO:2; 417 to 495 of SEQ ID NO:2; 417 to 496 of SEQ ID NO:2; 417 to 497 of SEQ ID NO:2; 417 to 498 of SEQ ID NO:2; 417 to 499 of SEQ ID NO:2; 417 to 500 of SEQ ID NO:2; 417 to 492 of SEQ ID NO:2; 417 to 491 of SEQ ID NO:2; 412 to 493 of SEQ ID NO:2; 413 to 493 of SEQ ID NO:2; 414 to 493 of SEQ ID NO:2; 415 to 493 of SEQ ID NO:2; 416 to 493 of SEQ ID NO:2; 411 to 493 of SEQ ID NO:2; 410 to 493 of SEQ ID NO:2; 410 to 494 of SEQ ID NO:2; 411 to 494 of SEQ ID NO:2; 412 to 494 of SEQ ID NO:2; 413 to 494 of SEQ ID NO:2; 414 to 494 of SEQ ID NO:2; 415 to 494 of SEQ ID NO:2; 416 to 494 of SEQ ID NO:2; 417 to 494 of SEQ ID NO:2; and 418 to 494 of SEQ ID NO:2 or fragments, variants, or derivatives of such polypeptides.

Various exemplary soluble Sp35 polypeptides and methods and materials for obtaining these molecules for practicing the present invention are described below and/or may be found, e.g., in International Patent Application No. PCT/US2004/008323, PCT Publication No. WO2004/085648, incorporated herein by reference in its entirety.

Soluble Sp35 polypeptides for use in the methods of the present invention described herein may be cyclic. Cyclization of the soluble Sp35 polypeptides reduces the conformational freedom of linear peptides and results in a more structurally constrained molecule. Many methods of peptide cyclization are known in the art, for example, "backbone to backbone" cyclization by the formation of an amide bond between the N-terminal and the C-terminal amino acid residues of the peptide. The "backbone to backbone" cyclization method includes the formation of disulfide bridges between two ω-thio amino acid residues (e.g. cysteine, homocysteine). Certain soluble Sp35 peptides of the present invention include modifications on the N- and C- terminus of the peptide to form a cyclic Sp35 polypeptide. Such modifications include, but are not limited to, cysteine residues, acetylated cysteine residues, cysteine residues with a NH2 moiety and biotin. Other methods of peptide cyclization are described in Li & Roller. Curr. Top. Med. Chem. 3:325-341 (2002), which is incorporated by reference herein in its entirety.

Soluble Sp35 polypeptides described herein may have various alterations such as substitutions, insertions or deletions. For examples, substitutions include, but are not limited to the following substitutions: valine at position 6 of the Sp35 polypeptide of SEQ ID NO:2 to methionine; serine at position 294 of the Sp35 polypeptide of SEQ ID NO:2 to glycine; valine at position 348 of the Sp35 polypeptide of SEQ ID NO:2 to alanine; arginine at position 419 of the Sp35 polypeptide to histidine; arginine at position 456 to glutamic acid; and histidine at position 458 of SEQ NO:2 to valine.

Corresponding fragments of soluble Sp35 polypeptides at least 70%, 75%, 80%, 85%, 90%, or 95% identical to polypeptides of SEQ ID NO:2 described herein are also contemplated.

As known in the art, "sequence identity" between two polypeptides is determined by comparing the amino acid sequence of one polypeptide to the sequence of a second polypeptide. When discussed herein, whether any particular polypeptide is at least about 70%, 75%, 80%, 85%, 90% or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full length of the reference polypeptide sequence and that gaps in homology of up to 5% of the total number of amino acids in the reference sequence are allowed.

Soluble Sp35 polypeptides for use in the methods of the present invention may include any combination of two or more soluble Sp35 polypeptides.

### Antibodies or Antigen-binding Fragments Thereof

In one embodiment, an Sp35 antagonist for use in the methods of the invention is an antibody molecule, or immunospecific fragment thereof. Unless it is specifically noted, as used herein, a "fragment thereof" in reference to an antibody refers to an immunospecific fragment, i.e., an antigen-specific fragment. In one embodiment, an antibody for use in the methods of the invention is a bispecific binding molecule, binding polypeptide, or antibody, e.g., a bispecific antibody, minibody, domain deleted antibody, or fusion protein having binding specificity for more than one epitope, e.g., more than one antigen or more than one epitope on the same antigen. In one embodiment, a bispecific antibody has at least one binding domain specific for at least one epitope on Sp35. A bispecific antibody may be a tetravalent antibody that has two target binding domains specific for an epitope of Sp35 and two target binding domains specific for a second target. Thus, a tetravalent bispecific antibody may be bivalent for each specificity.

Sp35 antagonists for use in the methods of the present invention also include Sp35-specific antibodies or antigen-binding fragments, variants, or derivatives which are antagonists of Sp35 activity. For example, binding of certain Sp35 antibodies to Sp35, as expressed on DA neurons, blocks inhibition of DA neurite outgrowth, differentiation and survival.

Certain antagonist antibodies for use in the methods described herein specifically or preferentially bind to a particular Sp35 polypeptide fragment or domain. Such Sp35 polypeptide fragments include, but are not limited to, an Sp35 polypeptide comprising, consisting essentially of, or consisting of amino acids 34 to 532; 34 to 417, 34 to 425, 34 to 493, 66 to 532, 66 to 417 (LRR domain), 66 to 426, 66 to 493, 66 to 532, 417 to 532, 417 to 425 (the Sp35 basic region), 417 to 424 (the Sp35 basic region), 417 to 493, 417 to 532, 419 to 493 (the Sp35 Ig region), or 425 to 532 of SEQ ID NO:2, or an Sp35 variant polypeptide at least 70%, 75%, 80%, 85%, 90%, or 95% identical to amino acids 34 to 532; 34 to 417, 34 to 425, 34 to 493, 66 to 532, 66 to 417, 66 to 426, 66 to 493, 66 to 532, 417 to 532, 417 to 425 (the Sp35 basic region), 417 to 493, 417 to 532, 419 to 493 (the Sp35 Ig region), or 425 to 532 of SEQ ID NO:2.

Additional Sp35 peptide fragments to which certain Sp35 specific antibodies, or antigen-binding fragments, variants, or derivatives thereof for use in the methods of the present invention bind include, but are not limited to, those fragments comprising, consisting essentially of, or consisting of one or more leucine-rich-repeats (LRR) of Sp35. Such fragments, include, for example, fragments comprising, consisting essentially of, or consisting of amino acids 66 to 89, 66 to 113, 66 to 137, 90 to 113, 114 to 137, 138 to 161, 162 to 185, 186 to 209, 210 to 233, 234 to 257, 258 to 281, 282 to 305, 306 to 329, or 330 to 353 of SEQ ID NO:2. Corresponding fragments of a variant Sp35 polypeptide at least 70%, 75%, 80%, 85%, 90%, or 95% identical to amino acids 66 to 89, 66 to 113, 90 to 113, 114 to 137, 138 to 161, 162 to 185, 186 to 209, 210 to 233, 234 to 257, 258 to 281, 282 to 305, 306 to 329, or 330 to 353 of SEQ ID NO:2 are also contemplated.

Additional Sp35 peptide fragments to which certain antibodies, or antigen-binding fragments, variants, or derivatives thereof of the present invention bind include, but are not limited to those fragments comprising, consisting essentially of, or consisting of one or more cysteine rich regions flanking the LRR of Sp35. Such fragments, include, for example, a fragment comprising, consisting essentially of, or consisting of amino acids 34 to 64 of SEQ ID NO:2 (the N-terminal LRR flanking region (LRRNT)), or a fragment comprising, consisting essentially of, or consisting of amino acids 363 to 416 of SEQ ID NO:2 (the C-terminal LRR flanking region (LRRCT)). Corresponding fragments of a variant Sp35 polypeptide at least 70%, 75%, 80%, 85%, 90%, or 95% identical to amino acids 34 to 64 and 363 to 416 of SEQ ID NO:2 are also contemplated.

In other embodiments, the Sp35 antagonists to be used in the methods described herein include an antibody, or antigen-binding fragment, variant, or derivative thereof which specifically or preferentially binds to at least one epitope of Sp35, where the epitope comprises, consists essentially of, or consists of at least about four to five amino acids of SEQ ID NO:2, at least seven, at least nine, or between at least about 15 to about 30 amino acids of SEQ ID NO:2. The amino acids of a given epitope of SEQ ID NO:2 as described may be, but need not be, contiguous or linear. In certain embodiments, at least one epitope of Sp35 comprises, consists essentially of, or consists of a nonlinear epitope formed by the extracellular domain of Sp35 as expressed on the surface of a cell or as a soluble fragment, e.g., fused to an IgG Fc region. Thus, in certain embodiments the at least one epitope of Sp35 comprises, consists essentially of, or consists of at least 4, at least 5, at least 6, at least 7, at least 8, at least 9, at least 10, at least 15, at least 20, at least 25, between about 15 to about 30, or at least 10, 15, 20, 25, 30, 35, 40, 45, 50, 55, 60, 65, 70, 75, 80, 85, 90, 95, or 100 contiguous or non-contiguous amino acids of SEQ ID NO:2, where non-contiguous amino acids form an epitope through protein folding.

In other embodiments, the Sp35 antagonists to be used in the methods of the present invention include Sp35 antibodies, or antigen-binding fragments, variants, or derivatives thereof which specifically or preferentially bind to at least one epitope of Sp35, where the epitope comprises, consists essentially of, or consists of, in addition to one, two, three, four, five, six or more contiguous or non-contiguous amino acids of SEQ ID NO:2 as described above, and an additional moiety which modifies the protein, e.g., a carbohydrate moiety may be included such that the Sp35 antibody binds with higher affinity to modified target protein than it does to an unmodified version of the protein. Alternatively, the Sp35 antibody does not bind the unmodified version of the target protein at all.

In certain embodiments, the Sp35 antagonists to be used in the methods of the present invention include an antibody, or antigen-binding fragment, variant, or derivative thereof of the invention binds specifically to at least one epitope of Sp35 or fragment or variant described above, i.e., binds to such an epitope more readily than it would bind to an unrelated, or random epitope; binds preferentially to at least one epitope of Sp35 or fragment or variant described above, i.e., binds to such an epitope more readily than it would bind to a related, similar, homologous, or analogous epitope; competitively inhibits binding of a reference antibody which itself binds specifically or preferentially to a certain epitope of Sp35 or fragment or variant described above; or binds to at least one epitope of Sp35 or fragment or variant described above with an affinity characterized by a dissociation constant K_{D} of less than about 5 x 10⁻² M, about 10⁻² M, about 5 x 10⁻³ M, about 10⁻³ M, about 5 x 10⁻⁴ M, about 10⁻⁴ M, about 5 x 10⁻⁵ M, about 10⁻⁵ M, about 5 x 10⁻⁶ M, about 10⁻⁶ M, about 5 x 10⁻⁷ M, about 10⁻⁷ M, about 5 x 10⁻⁸ M, about 10⁻⁸ M, about 5 x 10⁻⁹ M, about 10⁻⁹ M, about 5 x 10⁻¹⁰ M, about 10⁻¹⁰ M, about 5 x 10⁻¹¹ M, about 10⁻¹¹ M, about 5 x 10⁻¹² M, about 10⁻¹² M, about 5 x 10⁻¹³ M, about 10⁻¹³ M, about 5 x 10⁻¹⁴ M, about 10⁻¹⁴ M, about 5 x 10⁻¹⁵ M, or about 10-¹⁵ M. In a particular aspect, the antibody or fragment thereof preferentially binds to a human Sp35 polypeptide or fragment thereof, relative to a murine Sp35 polypeptide or fragment thereof.

As used in the context of antibody binding dissociation constants, the term "about" allows for the degree of variation inherent in the methods utilized for measuring antibody affinity. For example, depending on the level of precision of the instrumentation used, standard error based on the number of samples measured, and rounding error, the term "about 10-2 M" might include, for example, from 0.05 M to 0.005 M.

In specific embodiments, the Sp35 antagonists for use in the methods of the present invention include an antibody, or antigen-binding fragment, variant, or derivative thereof of the invention binds Sp35 polypeptides or fragments or variants thereof with an off rate (k(off)) of less than or equal to 5 X 10⁻² sec⁻¹, 10⁻² sec⁻¹, 5 X 10⁻³ sec⁻¹ or 10⁻³ sec⁻¹. Alternatively, an antibody, or antigen-binding fragment, variant, or derivative thereof of the invention binds Sp35 polypeptides or fragments or variants thereof with an off rate (k(off)) of less than or equal to 5 X 10⁻⁴ sec⁻¹, 10⁻⁴ sec⁻¹, 5 X 10⁻⁵ sec⁻¹, or 10⁻⁵ sec⁻¹ 5 X 10⁻⁶ sec⁻¹, 10⁻⁶ sec⁻¹, 5 X 10⁻⁷ sec⁻¹ or 10⁻⁷ sec⁻¹.

In other embodiments, the Sp35 antagonists for use in the methods of the present invention include an antibody, or antigen-binding fragment, variant, or derivative thereof of the invention binds Sp35 polypeptides or fragments or variants thereof with an on rate (k(on)) of greater than or equal to 10³ M⁻¹ sec⁻¹, 5 X 10³ M⁻¹ sec⁻¹, 10⁴ M⁻¹ sec⁻¹ or 5 X 10⁴ M⁻¹ see⁻¹. Alternatively, an antibody, or antigen-binding fragment, variant, or derivative thereof of the invention binds Sp35 polypeptides or fragments or variants thereof with an on rate (k(on)) greater than or equal to 10⁵ M⁻¹ sec⁻¹, 5 X 10⁵ M⁻¹ sec⁻¹, 10⁶ M⁻¹ sec⁻¹, or 5 X 10⁶ M⁻¹ sec⁻¹ or 10⁷ M⁻¹ sec⁻¹.

Certain methods of the present invention comprise administration of an Sp35 antagonist antibody, or immunospecific fragment thereof, in which at least a fraction of one or more of the constant region domains has been deleted or otherwise altered so as to provide desired biochemical characteristics such as reduced effector functions, the ability to non-covalently dimerize, increased ability to localize at the site of a tumor, reduced serum half-life, or increased serum half-life when compared with a whole, unaltered antibody of approximately the same immunogenicity. For example, certain antibodies for use in the methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the CH2 domain will be deleted.

In certain Sp35 antagonist antibodies or immunospecific fragments thereof for use in the methods described herein, the Fc portion may be mutated to decrease effector function using techniques known in the art. For example, the deletion or inactivation (through point mutations or other means) of a constant region domain may reduce Fc receptor binding of the circulating modified antibody thereby increasing tumor localization. In other cases it may be that constant region modifications consistent with the instant invention moderate complement binding and thus reduce the serum half life and nonspecific association of a conjugated cytotoxin. Yet other modifications of the constant region may be used to modify disulfide linkages or oligosaccharide moieties that allow for enhanced localization due to increased antigen specificity or antibody flexibility. The resulting physiological profile, bioavailability and other biochemical effects of the modifications, such as tumor localization, biodistribution and serum half-life, may easily be measured and quantified using well-known immunological techniques without undue experimentation.

Modified forms of antibodies or immunospecific fragments thereof for use in the methods disclosed herein can be made from whole precursor or parent antibodies using techniques known in the art. Exemplary techniques are discussed in more detail herein.

In certain embodiments both the variable and constant regions of Sp35 antagonist antibodies or immunospecific fragments thereof for use in the methods disclosed herein are fully human. Fully human antibodies can be made using techniques that are known in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in US patents: 6,150,584; 6,458,592; 6,420,140. Other techniques are known in the art. Fully human antibodies can likewise be produced by various display technologies, e.g., phage display or other viral display systems, as described in more detail elsewhere herein.

Sp35 antagonist antibodies or immunospecific fragments thereof for use in the methods disclosed herein can be made or manufactured using techniques that are known in the art. In certain embodiments, antibody molecules or fragments thereof are "recombinantly produced," i.e., are produced using recombinant DNA technology. Exemplary techniques for making antibody molecules or fragments thereof are discussed in more detail elsewhere herein.

Sp35 antagonist antibodies or immunospecific fragments thereof for use in the methods disclosed herein include derivatives that are modified, e.g., by the covalent attachment of any type of molecule to the antibody such that covalent attachment does not prevent the antibody from specifically binding to its cognate epitope. For example, but not by way of limitation, the antibody derivatives include antibodies that have been modified, *e.g*., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

In preferred embodiments, an Sp35 antagonist antibody or immunospecific fragment thereof for,use in the methods disclosed herein will not elicit a deleterious immune response in the animal to be treated, *e.g*., in a human. In one embodiment, Sp35 antagonist antibodies or immunospecific fragments thereof for use in the methods disclosed herein can be modified to reduce their immunogenicity using art-recognized techniques. For example, antibodies can be humanized, primatized, deimmunized, or chimeric antibodies can be made. These types of antibodies are derived from a non-human antibody, typically a murine or primate antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting at least a part of one or more of the non-human complementarity determining regions (CDRs) into a human framework and constant regions with or without retention of critical framework residues; or (c) transplanting the entire non-human variable domains, but "cloaking" them with a human-like section by replacement of surface residues. Such methods are disclosed in Morrison et al., Proc. Natl. Acad. Sci. 81:6851-6855 (1984); Morrison et al., Adv. Immunol. 44:65-92 (1988); Verhoeyen et al., Science 239:1534-1536 (1988); Padlan, Molec. Immun. 28:489-498 (1991); Padlan, Molec. Immun. 31:169-217 (1994), and U.S. Pat. Nos. 5,585,089, 5,693,761, 5,693,762, and 6,190,370, all of which are hereby incorporated by reference in their entirety.

De-immunization can also be used to decrease the immunogenicity of an antibody. As used herein, the term "de-immunization" includes alteration of an antibody to modify T cell epitopes (see, *e.g*., WO9852976A1, W00034317A2). For example, V_{H} and V_{L} sequences from the starting antibody are analyzed and a human T cell epitope "map" from each V region showing the location of epitopes in relation to complementarity-determining regions (CDRs) and other key residues within the sequence. Individual T cell epitopes from the T cell epitope map are analyzed in order to identify alternative amino acid substitutions with a low risk of altering activity of the final antibody. A range of alternative V_{H} and V_{L} sequences are designed comprising combinations of amino acid substitutions and these sequences are subsequently incorporated into a range of binding polypeptides, *e.g*., Sp35 antagonist antibodies or immunospecific fragments thereof for use in the methods disclosed herein, which are then tested for function. Typically, between 12 and 24 variant antibodies are generated and tested. Complete heavy and light chain genes comprising modified V and human C regions are then cloned into expression vectors and the subsequent plasmids introduced into cell lines for the production of whole antibody. The antibodies are then compared in appropriate biochemical and biological assays, and the optimal variant is identified.

Sp35 antagonist antibodies or fragments thereof for use in the methods of the present invention may be generated by any suitable method known in the art. Polyclonal antibodies can be produced by various procedures well known in the art. For example, a Sp35 immunospecific fragment can be administered to various host animals including, but not limited to, rabbits, mice, rats, etc. to induce the.production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum.* Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, Cold Spring Harbor Laboratory Press, 2nd ed. (1988); Hammerling et al., in: Monoclonal Antibodies and T-Cell Hybridomas Elsevier, N.Y., 563-681 (1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Thus, the term "monoclonal antibody" is not limited to antibodies produced through hybridoma technology. Monoclonal antibodies can be prepared using a wide variety of techniques known in the art including the use of hybridoma and recombinant and phage display technology.

Using art recognized protocols, in one example, antibodies are raised in mammals by multiple subcutaneous or intraperitoneal injections of the relevant antigen (*e.g*., purified Sp35 antigens or cells or cellular extracts comprising such antigens) and an adjuvant. This immunization typically elicits an immune response that comprises production of antigen-reactive antibodies from activated splenocytes or lymphocytes. While the resulting antibodies may be harvested from the serum of the animal to provide polyclonal preparations, it is often desirable to isolate individual lymphocytes from the spleen, lymph nodes or peripheral blood to provide homogenous preparations of monoclonal antibodies (mAbs). Preferably, the lymphocytes are obtained from the spleen.

In this well known process (Kohler et al., Nature 256:495 (1975)) the relatively short-lived, or mortal, lymphocytes from a mammal which has been injected with antigen are fused with an immortal tumor cell line (*e.g*. a myeloma cell line), thus producing hybrid cells or "hybridomas" which are both immortal and capable of producing the genetically coded antibody of the B cell. The resulting hybrids are segregated into single genetic strains by selection, dilution, and regrowth with each individual strain comprising specific genes for the formation of a single antibody. They produce antibodies which are homogeneous against a desired antigen and, in reference to their pure genetic parentage, are termed "monoclonal."

Hybridoma cells thus prepared are seeded and grown in a suitable culture medium that preferably contains one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. Preferably, the binding specificity of the monoclonal antibodies produced by hybridoma cells is determined by *in vitro* assays such as immunoprecipitation, radioimmunoassay (RIA) or enzyme-linked immunoabsorbent assay (ELISA). After hybridoma cells are identified that produce antibodies of the desired specificity, affinity and/or activity, the clones may be subcloned by limiting dilution procedures and grown by standard methods (Goding, Monoclonal Antibodies: Principles and Practice, Academic Press, pp 59-103 (1986)). It will further be appreciated that the monoclonal antibodies secreted by the subclones may be separated from culture medium, ascites fluid or serum by conventional purification procedures such as, for example, protein-A, hydroxylapatite chromatography, gel electrophoresis, dialysis or affinity chromatography.

Antibody fragments that recognize specific epitopes may be generated by known techniques. For example, Fab and F(ab')₂ fragments may be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the C_{H}1 domain of the heavy chain.

Those skilled in the art will also appreciate that DNA encoding antibodies or antibody fragments (*e.g*., antigen binding sites) may also be derived from antibody phage libraries. In a particular, such phage can be utilized to display antigen-binding domains expressed from a repertoire or combinatorial antibody library (*e.g*., human or murine). Phage expressing an antigen binding domain that binds the antigen of interest can be selected or identified with antigen, *e.g*., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Exemplary methods are set forth, for example, in EP 368 684 B1; U.S. patent. 5,969,108, Hoogenboom, H.R. and Chames, Immunol. Today 21:371 (2000); Nagy et al. Nat. Med. 8:801 (2002); Huie et al., Proc. Natl. Acad. Sci. USA 98:2682 (2001); Lui et al., J. Mol. Biol. 315:1063 (2002), each of which is incorporated herein by reference. Several publications (*e.g*., Marks et al., Bio/Technology 10:779-783 (1992)) have described the production of high affinity human antibodies by chain shuffling, as well as combinatorial infection and *in vivo* recombination as a strategy for constructing large phage libraries. In another embodiment, ribosomal display can be used to replace bacteriophage as the display platform (*see, e.g.,* Hanes et al., Nat. Biotechnol. 18:1287 (2000); Wilson et al., Proc. Natl. Acad. Sci. USA 98:3750 (2001); or Irving et al., J. Immunol. Methods 248:31 (2001)). In yet another embodiment, cell surface libraries can be screened for antibodies (Boder et al., Proc. Natl. Acad. Sci. USA 97:10701 (2000); Daugherty et al., J. Immunol. Methods 243:211 (2000)). Such procedures provide alternatives to traditional hybridoma techniques for the isolation and subsequent cloning of monoclonal antibodies.

In phage display methods, functional antibody domains are displayed on the surface of phage particles which carry the polynucleotide sequences encoding them. In particular, DNA sequences encoding V_{H} and V_{L} regions are amplified from animal cDNA libraries (*e.g*., human or murine cDNA libraries of lymphoid tissues) or synthetic cDNA libraries. In certain embodiments, the DNA encoding the V_{H} and V_{L} regions are joined together by an scFv linker by PCR and cloned into a phagemid vector (*e.g*., p CANTAB 6 or pComb 3 HSS). The vector is electroporated in *E. coli* and the *E. coli* is infected with helper phage. Phage used in these methods are typically filamentous phage including fd and M13 and the V_{H} or V_{L} regions are usually recombinantly fused to either the phage gene III or gene VIII. Phage expressing an antigen binding domain that binds to an antigen of interest (*i.e*., a Sp35 polypeptide or a fragment thereof) can be selected or identified with antigen, *e.g*., using labeled antigen or antigen bound or captured to a solid surface or bead.

Additional examples of phage display methods that can be used to make the antibodies include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187:9-18 (1997); Burton et al., Advances in Immunology 57:191-280 (1994); PCT Application No. PCT/GB91/01134; PCT publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties).

Examples of techniques which can be used to produce single-chain Fvs and antibodies include those described in U.S. Pat. Nos. 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, including *in vivo* use of antibodies in humans and *in vitro* detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. *See*, *e.g*., Morrison, Science 229:1202 (1985); Oi et al., BioTechniques 4:214 (1986); Gillies et al., J. Immunol. Methods 125:191-202 (1989); U.S. Pat. Nos. 5,807,715; 4,816,567; and 4,816397, which are incorporated herein by reference in their entireties. Humanized antibodies are antibody molecules from non-human species antibody that binds the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, and preferably improve, antigen binding. These framework substitutions are identified by methods well known in the art, *e.g*., by modeling of the interactions of the CDR and framework residues to identify framework residues important for antigen binding and sequence comparison to identify unusual framework residues at particular positions. (See, *e.g.*, Queen et al., U.S. Pat. No. 5,585,089; Riechmann et al., Nature 332:323 (1988), which are incorporated herein by reference in their entireties.) Antibodies can be humanized using a variety of techniques known in the art including, for example, CDR-grafting (EP 239,400; PCT publication WO 91/09967; U.S. Pat. Nos. 5,225,539; 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Pat. No. 5,565,332).

Completely human antibodies are particularly desirable for therapeutic treatment of human patients. Human antibodies can be made by a variety of methods known in the art including phage display methods described above using antibody libraries derived from human immunoglobulin sequences. See also, U.S. Pat. Nos. 4,444,887 and 4,716,111; and PCT publications WO 98/46645, WO 98/50433, WO 98/24893, WO 98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region; and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, e.g., all or a portion of a desired target polypeptide. Monoclonal antibodies directed against the antigen can be obtained from the immunized, transgenic mice using conventional hybridoma technology. The human immunoglobulin transgenes harbored by the transgenic mice rearrange during B-cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar Int. Rev. Immunol. 13:65-93 (1995). For a detailed discussion of this technology for producing human antibodies and human monoclonal antibodies and protocols for producing such antibodies, see, e.g., PCT publications WO 98/24893; WO 96/34096; WO 96/33735; U.S. Pat. Nos. 5,413,923; 5,625,126; 5,633,425; 5,569,825; 5,661,016; 5,545,806; 5,814,318; and 5,939,598, which are incorporated by reference herein in their entirety. In addition, companies such as Abgenix, Inc. (Freemont, Calif.) and GenPharm (San Jose, Calif.) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g*., a mouse antibody, is used to guide the selection of a completely human antibody recognizing the same epitope. (Jespers et al., Bio/Technology 12:899-903 (1988)). *See also*, U.S. Patent No. 5,565,332.

In another embodiment, DNA encoding desired monoclonal antibodies for use in the methods of the present invention may be readily isolated and sequenced using conventional procedures (e.g., by using oligonucleotide probes that are capable of binding specifically to genes encoding the heavy and light chains of murine antibodies). The isolated and subcloned hybridoma cells serve as a preferred source of such DNA. Once isolated, the DNA may be placed into expression vectors, which are then transfected into prokaryotic or eukaryotic host cells such as *E. coli* cells, simian COS cells, Chinese Hamster Ovary (CHO) cells or myeloma cells that do not otherwise produce immunoglobulins. More particularly, the isolated DNA (which may be synthetic as described herein) may be used to clone constant and variable region sequences for the manufacture antibodies as described in Newman et al., U.S. Pat. No. 5,658,570, filed January 25; 1995, which is incorporated by reference herein. Essentially, this entails extraction of RNA from the selected cells, conversion to cDNA, and amplification by PCR using Ig specific primers. Suitable primers for this purpose are also described in U.S. Pat. No. 5,658,570. As will be discussed in more detail below, transformed cells expressing the desired antibody may be grown up in relatively large quantities to provide clinical and commercial supplies of the immunoglobulin.

In a specific embodiment, the amino acid sequence of the heavy and/or light chain variable domains may be inspected to identify the sequences of the complementarity determining regions (CDRs) by methods that are well known in the art, *e.g*., by comparison to known amino acid sequences of other heavy and light chain variable regions to determine the regions of sequence hypervariability. Using routine recombinant DNA techniques, one or more of the CDRs may be inserted within framework regions, *e.g.*, into human framework regions to humanize a non-human antibody. The framework regions may be naturally occurring or consensus framework regions, and preferably human framework regions (see, *e.g.,* Chothia et al., J. Mol. Biol. 278:457-479 (1998) for a listing of human framework regions). Preferably, the polynucleotide generated by the combination of the framework regions and CDRs encodes an antibody that specifically binds to at least one epitope of a desired polypeptide, *e.g*., Sp35. Preferably, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As used herein, a chimeric antibody is a molecule in which different portions are derived from different animal species, such as those having a variable region derived from a murine monoclonal antibody and a human immunoglobulin constant region, *e.g*., humanized antibodies.

Alternatively, techniques described for the production of single chain antibodies (U.S. Pat. No. 4,694,778; Bird, Science 242:423-442 (1988); Huston et al., Proc. Natl. Acad. Sci. USA 85:5879-5883 (1988); and Ward et al., Nature 334:544-554 (1989)) can be adapted to produce single chain antibodies. Single chain antibodies are formed by linking the heavy and light chain fragments of the Fv region via an amino acid bridge, resulting in a single chain antibody. Techniques for the assembly of functional Fv fragments in E coli may also be used (Skerra et al., Science 242:1038-1041 (1988)):

Sp35 antagonist antibodies may also be human or substantially human antibodies generated in transgenic animals (*e.g*., mice) that are incapable of endogenous immunoglobulin production (see *e.g*., U:S. Pat. Nos. 6,075,181, 5,939,598, 5,591,669 and 5,589,369 each of which is incorporated herein by reference). For example, it has been described that the homozygous deletion of the antibody heavy-chain joining region in chimeric and germ-line mutant mice results in complete inhibition of endogenous antibody production. Transfer of a human immunoglobulin gene array to such germ line mutant mice will result in the production of human antibodies upon antigen challenge. Another preferred means of generating human antibodies using SCID mice is disclosed in U.S. Pat. No. 5,811,524 which is incorporated herein by reference. It will be appreciated that the genetic material associated with these human antibodies may also be isolated and manipulated as described herein.

Yet another highly efficient means for generating recombinant antibodies is disclosed by Newman, Biotechnology 10: 1455-1460 (1992). Specifically, this technique results in the generation of primatized antibodies that contain monkey variable domains and human constant sequences. This reference is incorporated by reference in its entirety herein. Moreover, this technique is also described in commonly assigned U.S. Pat. Nos. 5,658,570, 5,693,780 and 5,756,096 each of which is incorporated herein by reference.

In another embodiment, lymphocytes can be selected by micromanipulation and the variable genes isolated. For example, peripheral blood mononuclear cells can be isolated from an immunized mammal and cultured for about 7 days *in vitro.* The cultures can be screened for specific IgGs that meet the screening criteria. Cells from positive wells can be isolated. Individual Ig-producing B cells can be isolated by FACS or by identifying them in a complement-mediated hemolytic plaque assay. Ig-producing B cells can be micromanipulated into a tube and the V_{H} and V_{L} genes can be amplified using, *e.g*., RT-PCR. The V_{H} and V_{L} genes can be cloned into an antibody expression vector and transfected into cells (*e.g*., eukaryotic or prokaryotic cells) for expression.

Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991) which is herein incorporated by reference in its entirety, including supplements.

Antibodies for use in the methods disclosed herein can be produced by any method known in the art for the synthesis of antibodies, in particular, by chemical synthesis or preferably, by recombinant expression techniques as described herein.

It will further be appreciated that the scope of this invention further encompasses all alleles, variants and mutations of antigen binding DNA sequences.

As is well known, RNA may be isolated from the original hybridoma cells or from other transformed cells by standard techniques, such as guanidinium isothiocyanate extraction and precipitation followed by centrifugation or chromatography. Where desirable, mRNA may be isolated from total RNA by standard techniques such as chromatography on oligo dT cellulose. Suitable techniques are familiar in the art.

In one embodiment, cDNAs that encode the light and the heavy chains of the antibody for use in the methods of the present invention may be made, either simultaneously or separately, using reverse transcriptase and DNA polymerase in accordance with well known methods. PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as mouse constant region probes.

DNA, typically plasmid DNA, may be isolated from the cells using techniques known in the art, restriction mapped and sequenced in accordance with standard, well known techniques set forth in detail, *e.g*., in the foregoing references relating to recombinant DNA techniques. Of course, the DNA may be synthetic according to the present invention at any point during the isolation process or subsequent analysis.

Recombinant expression of an antibody, or fragment, derivative or analog thereof, *e.g*., a heavy or light chain of an antibody which is an Sp35 antagonist, requires construction of an expression vector containing a polynucleotide that encodes the antibody. Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, or a heavy or light chain thereof, or a heavy or light chain variable domain, operably linked to a promoter. Such vectors may include the nucleotide sequence encoding the constant region of the antibody molecule (see, *e.g*., PCT Publication WO 86/05807; PCT Publication WO 89/01036; and U.S. Pat. No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody for use in the methods described herein. Thus, the invention includes host cells containing a polynucleotide encoding an antibody of the invention, or a heavy or light chain thereof, operably linked to a heterologous promoter. In preferred embodiments for the expression of double-chained antibodies; vectors encoding both the heavy and light chains may be co-expressed in the host cell for expression of the entire immunoglobulin molecule, as detailed below.

A variety of host-expression vector systems may be utilized to express antibody molecules for use in the methods described herein. Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria (*e.g., E. coli, B. subtilis)* transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BLK, 293, 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., EMBO J. 2:1791 (1983)), in which the antibody coding sequence may be ligated individually into the vector in frame with the lacZ coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is typically used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing the antibody molecule in infected hosts. (*e.g*., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*see* Bittner et al., Methods in Enzymol. 153:51-544 (1987)).

In addition, a host cell strain may be chosen which modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, HeLa, COS, MDCK, 293, 3T3, WI38, and in particular, breast cancer cell lines such as, for example, BT483, Hs578T, HTB2, BT20 and T47D, and normal mammary gland cell line such as, for example, CRL7030 and Hs578Bst.

For long-term, high-yield production of recombinant proteins, stable expression may be used. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 1980) genes, can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); TIB TECH 11(5):155-215 (May, 1993); and hygro, which confers resistance to hygromycin (Santerre et al., Gene 30:147 (1984). Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981), which are incorporated by reference herein in their entireties.

The expression levels of an antibody molecule can be increased by vector amplification (for a review, *see* Bebbington and Hentschel, The use of vectors based on gene amplification for the expression of cloned genes in mammalian cells in DNA cloning, Academic Press, New York, Vol. 3. (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Once an antibody molecule of the invention has been recombinantly expressed, it may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Alternatively, a preferred method for increasing the affinity of antibodies of the invention is disclosed in US 2002 0123057 A1.

In one embodiment, a binding molecule or antigen binding molecule for use in the methods of the invention comprises a synthetic constant region wherein one or more domains are partially or entirely deleted ("domain-deleted antibodies"). In certain embodiments compatible modified antibodies will comprise domain deleted constructs or variants wherein the entire C_{H}2 domain has been removed (□C_{H}2 constructs). For other embodiments a short connecting peptide may be substituted for the deleted domain to provide flexibility and freedom of movement for the variable region. Those skilled in the art will appreciate that such constructs are particularly preferred due to the regulatory properties of the C_{H}2 domain on the catabolic rate of the antibody.

In certain embodiments, modified antibodies for use in the methods disclosed herein are minibodies. Minibodies can be made using methods described in the art (*see*, *e.g*., US patent 5,837,821 or WO 94/09817A1).

In another embodiment, modified antibodies for use in the methods disclosed herein are C_{H}2 domain deleted antibodies which are known in the art. Domain deleted constructs can be derived using a vector (*e.g*., from Biogen IDEC Incorporated) encoding an IgG₁ human constant domain (see, *e.g*., WO 02/060955A2 and WO02/096948A2). This exemplary vector was engineered to delete the C_{H}2 domain and provide a synthetic vector expressing a domain deleted IgG₁ constant region.

In one embodiment, an Sp35 antagonist antibody or fragment thereof for use in the methods disclosed herein comprises an immunoglobulin heavy chain having deletion or substitution of a few or even a single amino acid as long as it permits association between the monomeric subunits. For example, the mutation of a single amino acid in selected areas of the C_{H}2 domain may be enough to substantially reduce Fc binding and thereby increase tumor localization. Similarly, it may be desirable to simply delete that part of one or more constant region domains that control the effector function (*e.g*. complement binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be synthetic through the mutation or substitution of one or more amino acids that enhances the profile of the resulting construct. In this respect it may be possible to disrupt the activity provided by a conserved binding site (*e.g*. Fc binding) while substantially maintaining the configuration and immunogenic profile of the modified antibody. Yet other embodiments comprise the addition of one or more amino acids to the constant region to enhance desirable characteristics such as effector function or provide for more cytotoxin or carbohydrate attachment. In such embodiments it may be desirable to insert or replicate specific sequences derived from selected constant region domains.

The present invention also provides the use of antibodies that comprise, consist essentially of, or consist of, variants (including derivatives) of antibody molecules (*e.g*., the V_{H} regions and/or V_{L} regions) described herein, which antibodies or fragments thereof immunospecifically bind to a Sp35 polypeptide. Standard techniques known to those of skill in the art can be used to introduce mutations in the nucleotide sequence encoding a binding molecule, including, but not limited to, site-directed mutagenesis and PCR-mediated mutagenesis which result in amino acid substitutions. Preferably, the variants (including derivatives) encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference V_{H} region, V_{H}CDR1, V_{H}CDR2, V_{H}CDR3, V_{L} region, V_{L}CDR1, V_{L}CDR2, or V_{L}CDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains (*e.g.*, lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity.

For example, it is possible to introduce mutations only in framework regions or only in CDR regions of an antibody molecule. Introduced mutations may be silent or neutral missense mutations, *i.e*., have no, or little, effect on an antibody's ability to bind antigen. These types of mutations may be useful to optimize codon usage, or improve a hybridoma's antibody production. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen binding activity or alteration in binding activity (*e.g*., improvements in antigen binding activity or change in antibody specificity). Following mutagenesis, the encoded protein may routinely be expressed and the functional and/or biological activity of the encoded protein can be determined using techniques described herein or by routinely modifying techniques known in the art.

Exemplary antibodies or fragments thereof for use in the methods of the present invention include, but are not limited to, isolated antibodies or antigen binding fragments thereof which specifically binds to the same Sp35 epitope as a reference monoclonal antibody selected from the group consisting of 201', 3A3, 3A6, 1A7, 1G7, 2B10, 2C11, 2F3, 3P1D10.2C3, 3P1E11.3B7, 3P2C6.3G10.2H7, 3P2C9.2G4, 3P4A6.1D9, 3P4A1.2B9, 3P4C2.2D2, 3P4C5.1D8, 3P4C8.2G9, 30-C12 (Li01), 38-D01 (Li02), 35-E04 (Li03), 36-C09 (Li04), 30-A11 (Li05), 34-F02 (Li06), 29-E07 (Li07), 34-G04 (Li08), 36-A12 (Li09), 28-D02 (Li10), 30-B01 (Li11) 34-B03 (Li12), 3383 (L1a.1), 3495(L1a.2), 3563 (L1a.3), 3564 (L1a.4), 3565 (L1a.5), 3566 (L1a.6), 3567 (L1a.7), 3568 (Lla.8), 3569 (Lla.9), 3570 (L1a.10), 3571 (L1a.11), 3582 (L1a.12), and 1968 (L1a.13), all as described in U.S. Provisional Patent Application Nos. 60/697,336, 60/771,990 and 60/814,522 which are all incorporated herein by reference in their entireties.

### Fusion Proteins and Conjugated Polypeptides and Antibodies

Sp35 antagonist polypeptides, aptamers and antagonist antibodies for use in the methods disclosed herein may further be recombinantly fused to a heterologous polypeptide at the N- or C-terminus or chemically conjugated (including covalent and non-covalent conjugations) to polypeptides or other compositions. For example, Sp35 antagonist polypeptides, aptamers and antibodies may be recombinantly fused or conjugated to molecules useful as labels in detection assays and effector molecules such as heterologous polypeptides, drugs, radionuclides, or toxins. *See, e.g.,* PCT publications WO 92/08495; WO 91/14438; WO 89/12624; U.S. Patent No. 5,314,995; and EP 396,387.

Sp35 antagonist polypeptides, aptamers and antibodies for use in the methods disclosed herein include derivatives that are modified, *i.e*., by the covalent attachment of any type of molecule such that covalent attachment does not prevent the Sp35 antagonist polypeptide, aptamer or antibody from inhibiting the biological function of Sp35. For example, but not by way of limitation, the Sp35 antagonist polypeptides, aptamers and antibodies of the present invention may be modified *e.g*., by glycosylation, acetylation, pegylation, phosphylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

Sp35 antagonist polypeptides, aptamers and antibodies for use in the methods disclosed herein can be composed of amino acids joined to each other by peptide bonds or modified peptide bonds, *i.e*., peptide isosteres, and may contain amino acids other than the 20 gene-encoded amino acids. Sp35 antagonist polypeptides, aptamers and antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the Sp35 antagonist polypeptide or antibody, including the peptide backbone, the amino acid side-chains and the amino or carboxyl termini, or on moieties such as carbohydrates. It will be appreciated that the same type of modification may be present in the same or varying degrees at several sites in a given Sp35 antagonist polypeptide, aptamer or antibody. Also, a given Sp35 antagonist polypeptide, aptamer or antibody may contain many types of modifications. Sp35 antagonist polypeptides, aptamers or antibodies may be branched, for example, as a result of ubiquitination, and they may be cyclic, with or without branching. Cyclic, branched, and branched cyclic Sp35 antagonist polypeptides, aptamers and antibodies may result from posttranslational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (*See*, for instance, Proteins - Structure And Molecular Properties, T. E. Creighton, W. H. Freeman and Company, New York 2nd Ed., (1993); Posttranslational Covalent Modification Of Proteins, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992)).

The present invention also provides for fusion proteins comprising, consisting essentially of, or consisting of a Sp35 antagonist polypeptide, aptamer or antibody fusion that inhibits Sp35 function. Preferably, the heterologous polypeptide to which the Sp35 antagonist polypeptide, aptamer or antibody is fused is useful for function or is useful to target the Sp35 antagonist polypeptide or antibody. In certain embodiments of the invention a soluble Sp35 antagonist polypeptide, *e.g*., an Sp35 polypeptide comprising the LRR domains, Ig domain, or the entire extracellular domain (corresponding to amino acids 34 to 532 of SEQ ID NO: 2), is fused to a heterologous polypeptide moiety to form a Sp35 antagonist fusion polypeptide. Sp35 antagonist fusion proteins, aptamers and antibodies can be used to accomplish various objectives, *e.g*., increased serum half-life, improved bioavailability, *in vivo* targeting to a specific organ or tissue type, improved recombinant expression efficiency, improved host cell secretion, ease of purification, and higher avidity. Depending on the objective(s) to be achieved, the heterologous moiety can be inert or biologically active. Also, it can be chosen to be stably fused to the Sp35 antagonist polypeptide, aptamer or antibody or to be cleavable, *in vitro* or *in vivo.* Heterologous moieties to accomplish these other objectives are known in the art.

As an alternative to expression of an Sp35 antagonist fusion polypeptide, aptamer or antibody, a chosen heterologous moiety can be preformed and chemically conjugated to the Sp35 antagonist polypeptide, aptamer or antibody. In most cases, a chosen heterologous moiety will function similarly, whether fused or conjugated to the Sp35 antagonist polypeptide, aptamer or antibody. Therefore, in the following discussion of heterologous amino acid sequences, unless otherwise noted, it is to be understood that the heterologous sequence can be joined to the Sp35 antagonist polypeptide, aptamer or antibody in the form of a fusion protein or as a chemical conjugate.

Pharmacologically active polypeptides such as Sp35 antagonist polypeptides, aptamers or antibodies often exhibit rapid *in vivo* clearance, necessitating large doses to achieve therapeutically effective concentrations in the body. In addition, polypeptides smaller than about 60 kDa potentially undergo glomerular filtration, which sometimes leads to nephrotoxicity. Fusion or conjugation of relatively small polypeptides such as Sp35 antagonist polypeptides, aptamers or antibodies can be employed to reduce or avoid the risk of such nephrotoxicity. Various heterologous amino acid sequences, *i.e*., polypeptide moieties or "carriers," for increasing the *in vivo* stability, *i.e*., serum half-life, of therapeutic polypeptides are known.

Due to its long half-life, wide *in vivo* distribution, and lack of enzymatic or immunological function, essentially full-length human serum albumin (HSA), or an HSA fragment, is commonly used as a heterologous moiety. Through application of methods and materials such as those taught in Yeh et al., Proc. Natl. Acad. Sci. USA 89:1904-08 (1992) and Syed et al., Blood 89:3243-52 (1997), HSA can be used to form an Sp35 antagonist fusion polypeptide, aptamer, antibody or polypeptide/antibody conjugate that displays pharmacological activity by virtue of the Sp35 moiety while displaying significantly increased *in vivo* stability, *e.g*., 10-fold to 100-fold higher. The C-terminus of the HSA can be fused to the N-terminus of the soluble Sp35 moiety. Since HSA is a naturally secreted protein, the HSA signal sequence can be exploited to obtain secretion of the soluble Sp35 fusion protein into the cell culture medium when the fusion protein is produced in a eukaryotic, *e.g*., mammalian, expression system.

In certain embodiments, Sp35 antagonist polypeptides, aptamers, antibodies and antibody fragments thereof for use in the methods of the present invention further comprise a targeting moiety. Targeting moieties include a protein or a peptide which directs localization to a certain part of the body, for example, to the brain or compartments therein. In certain embodiments, Sp35 antagonist polypeptides, aptamers, antibodies or antibody fragments thereof for use in the methods of the present invention are attached or fused to a brain targeting moiety. The brain targeting moieties are attached covalently (*e.g*., direct, translational fusion, or by chemical linkage either directly or through a spacer molecule, which can be optionally cleavable) or non-covalently attached (*e.g*., through reversible interactions such as avidin, biotin, protein A, IgG, etc.). In other embodiments, the Sp35 antagonist polypeptides, aptamers, antibodies or antibody fragments thereof for use in the methods of the present invention are attached to one more brain targeting moieties. In additional embodiments, the brain targeting moiety is attached to a plurality of Sp35 antagonist polypeptides, aptamers, antibodies or antibody fragments thereof for use in the methods of the present invention.

A brain targeting moiety associated with an Sp35 antagonist polypeptide, aptamer, antibody or antibody fragment thereof enhances brain delivery of such an Sp35 antagonist polypeptide, aptamer, antibody or antibody fragment thereof. A number of polypeptides have been described which, when fused to a protein or therapeutic agent, delivers the protein or therapeutic agent through the blood brain barrier (BBB). Non-limiting examples include the single domain antibody FC5 (Abulrob et al. (2005) J. Neurochem. 95, 1201-1214); mAB 83-14, a monoclonal antibody to the human insulin receptor (Pardridge et al. (1995) Pharmacol. Res. 12, 807-816); the B2, B6 and B8 peptides binding to the human transferrin receptor (hTfR) (Xia et al. (2000) J. Virol. 74, 11359-11366); the OX26 monoclonal antibody to the transferrin receptor (Pardridge et al. (1991) J. Pharmacol. Exp. Ther. 259, 66-70); and SEQ ID NOs: 1-18 of U.S. Patent No. 6,306,365. The contents of the above references are incorporated herein by reference in their entirety.

Enhanced brain delivery of an Sp35 composition is determined by a number of means well established in the art. For example, administering to an animal a radioactively labeled Sp35 antagonist polypeptide, aptamer, antibody or antibody fragment thereof linked to a brain targeting moiety; determining brain localization; and comparing localization with an equivalent radioactively labeled Sp35 antagonist polypeptide, aptamer, antibody or antibody fragment thereof that is not associated with a brain targeting moiety. Other means of determining enhanced targeting are described in the above references.

The signal sequence is a polynucleotide that encodes an amino acid sequence that initiates transport of a protein across the membrane of the endoplasmic reticulum. Signal sequences useful for constructing an immunofusin include antibody light chain signal sequences, *e.g*., antibody 14.18 (Gillies et al., J. Immunol. Meth. 125:191-202 (1989)), antibody heavy chain signal sequences, *e.g*.; the MOPC141 antibody heavy chain signal sequence (Sakano et al., Nature 286:5774 (1980)). Alternatively, other signal sequences can be used. *See*, *e.g*., Watson, Nucl. Acids Res. 12:5145 (1984). The signal peptide is usually cleaved in the lumen of the endoplasmic reticulum by signal peptidases. This results in the secretion of an immunofusin protein containing the Fc region and the soluble Sp35 moiety.

In some embodiments, the DNA sequence may encode a proteolytic cleavage site between the secretion cassette and the soluble Sp35 moiety. Such a cleavage site may provide, *e.g*., for the proteolytic cleavage of the encoded fusion protein, thus separating the Fc domain from the target protein. Useful proteolytic cleavage sites include amino acid sequences recognized by proteolytic enzymes such as trypsin, plasmin, thrombin, factor Xa, or enterokinase K.

The secretion cassette can be incorporated into a replicable expression vector. Useful vectors include linear nucleic acids, plasmids, phagemids, cosmids and the like. An exemplary expression vector is pdC, in which the transcription of the immunofusin DNA is placed under the control of the enhancer and promoter of the human cytomegalovirus. *See*, *e.g*., Lo et al., Biochim. Biophys. Acta 1088:712 (1991); and Lo et al., Protein Engineering 11:495-500 (1998). An appropriate host cell can be transformed or transfected with a DNA that encodes a soluble Sp35 polypeptide and used for the expression and secretion of the soluble Sp35 polypeptide. Host cells that are typically used include immortal hybridoma cells, myeloma cells, 293 cells, Chinese hamster ovary (CHO) cells, HeLa cells, and COS cells.

In one embodiment, a soluble Sp35 polypeptide is fused to a hinge and Fc region, *i.e*., the C-terminal portion of an Ig heavy chain constant region. Potential advantages of an Sp35-Fc fusion include solubility, *in vivo* stability, and multivalency, *e.g*., dimerization. The Fc region used can be an IgA, IgD, or IgG Fc region (hinge- C_{H}2- C_{H}3). Alternatively, it can be an IgE or IgM Fc region (hinge- C_{H}2- C_{H}3-C_{H}4). An IgG Fc region is generally used, *e.g*., an IgG₁ Fc region or IgG₄ Fc region. In one embodiment, a sequence beginning in the hinge region just upstream of the papain cleavage site which defines IgG Fc chemically (*i.e*. residue 216, taking the first residue of heavy chain constant region to be 114 according to the Kabat system), or analogous sites of other immunoglobulins, is used in the fusion. The precise site at which the fusion is made is not critical; particular sites are well known and may be selected in order to optimize the biological activity, secretion, or binding characteristics of the molecule. Materials and methods for constructing and expressing DNA encoding Fc fusions are known in the art and can be applied to obtain soluble Sp35 fusions without undue experimentation. Some embodiments of the invention employ an Sp35 fusion protein such as those described in Capon et al., U.S. Patent Nos. 5,428,130 and 5,565,335.

Fully intact, wild-type Fc regions display effector functions that normally are unnecessary and undesired in an Fc fusion protein used in the methods of the present invention. Therefore, certain binding sites typically are deleted from the Fc region during the construction of the secretion cassette. For example, since coexpression with the light chain is unnecessary, the binding site for the heavy chain binding protein, Bip (Hendershot et al., Immunol. Today 8:111-14 (1987)), is deleted from the C_{H}2 domain of the Fc region of IgE, such that this site does not interfere with the efficient secretion of the immunofusin. Transmembrane domain sequences, such as those present in IgM, also are generally deleted.

The IgG₁ Fc region is most often used. Alternatively, the Fc region of the other subclasses of immunoglobulin gamma (gamma-2, gamma-3 and gamma-4) can be used in the secretion cassette. The IgG₁ Fc region of immunoglobulin gamma-1 is generally used in the secretion cassette and includes at least part of the hinge region, the C_{H}2 region, and the C_{H}3 region. In some embodiments, the Fc region of immunoglobulin gamma-1 is a C_{H}2-deleted-Fc, which includes part of the hinge region and the C_{H}3 region, but not the C_{H}2 region. A C_{H}2-deleted-Fc has been described by Gillies et al. (1990) Hum. Antibod. Hybridomas 1:47. In some embodiments, the Fc region of one of IgA, IgD, IgE, or IgM, is used.

Sp35-Fc fusion proteins can be constructed in several different configurations. In one configuration the C-terminus of the soluble Sp35 moiety is fused directly to the N-terminus of the Fc hinge moiety. In a slightly different configuration, a short polypeptide, *e.g*., 2-10 amino acids, is incorporated into the fusion between the N-terminus of the soluble Sp35 moiety and the C-terminus of the Fc moiety. Such a linker provides conformational flexibility, which may improve biological activity in some circumstances. If a sufficient portion of the hinge region is retained in the Fc moiety, the Sp35-Fc fusion will dimerize, thus forming a divalent molecule. A homogeneous population of monomeric Fc fusions will yield monospecific, bivalent dimers. A mixture of two monomeric Fc fusions each having a different specificity will yield bispecific, bivalent dimers.

Any of a number of cross-linkers that contain a corresponding amino-reactive group and thiol-reactive group can be used to link Sp35 antagonist polypeptides to serum albumin. Examples of suitable linkers include amine reactive cross-linkers that insert a thiol-reactive maleimide, *e.g*., SMCC, AMAS, BMPS, MBS, EMCS, SMPB, SMPH, KMUS, and GMBS. Other suitable linkers insert a thiol-reactive haloacetate group, *e.g*., SBAP, SIA, SIAB. Linkers that provide a protected or non-protected thiol for reaction with sulfhydryl groups to product a reducible linkage include SPDP, SMPT, SATA, and SATP. Such reagents are commercially available (*e.g*., Pierce Chemicals).

Conjugation does not have to involve the N-terminus of a soluble Sp35 polypeptide or the thiol moiety on serum albumin. For example, soluble Sp35-albumin fusions can be obtained using genetic engineering techniques, wherein the soluble Sp35 moiety is fused to the serum albumin gene at its N-terminus, C-terminus, or both.

Soluble Sp35 polypeptides can be fused to heterologous peptides to facilitate purification or identification of the soluble Sp35 moiety. For example, a histidine tag can be fused to a soluble Sp35 polypeptide to facilitate purification using commercially available chromatography media.

In some embodiments of the invention, a soluble Sp35 fusion construct is used to enhance the production of a soluble Sp35 moiety in bacteria. In such constructs a bacterial protein normally expressed and/or secreted at a high level is employed as the N-terminal fusion partner of a soluble Sp35 polypeptide. *See*, *e.g*., Smith et al., Gene 67:31 (1988); Hopp et al., Biotechnology 6:1204 (1988); La Vallie et al., Biotechnology 11:187 (1993).

By fusing a soluble Sp35 moiety at the amino and carboxy termini of a suitable fusion partner, bivalent or tetravalent forms of a soluble Sp35 polypeptide can be obtained. For example, a soluble Sp35 moiety can be fused to the amino and carboxy termini of an Ig moiety to produce a bivalent monomeric polypeptide containing two soluble Sp35 moieties. Upon dimerization of two of these monomers, by virtue of the Ig moiety, a tetravalent form of a soluble Sp35 protein is obtained. Such multivalent forms can be used to achieve increased binding affinity for the target. Multivalent forms of soluble Sp35 also can be obtained by placing soluble Sp35 moieties in,tandem to form concatamers, which can be employed alone or fused to a fusion partner such as Ig or HSA. Conjugated Polymers (other than polypeptides)

Some embodiments of the invention involve a soluble Sp35 polypeptide, Sp35 aptamer, or Sp35 antibody wherein one or more polymers are conjugated (covalently linked) to the Sp35 polypeptide, aptamer or antibody for use in the methods of the present invention. Examples of polymers suitable for such conjugation include polypeptides (discussed above), aptamers, sugar polymers and polyalkylene glycol chains. Typically, but not necessarily, a polymer is conjugated to the soluble Sp35 polypeptide, aptamer or Sp35 antibody for the purpose of improving one or more of the following: solubility, stability, or bioavailability.

The class of polymer generally used for conjugation to a Sp35 antagonist polypeptide, aptamer or antibody is a polyalkylene glycol. Polyethylene glycol (PEG) is most frequently used. PEG moieties, *e.g*., 1, 2, 3, 4 or 5 PEG polymers, can be conjugated to each Sp35 antagonist polypeptide, aptamer, or antibody to increase serum half life, as compared to the Sp35 antagonist polypeptide, aptamer or antibody alone. PEG moieties are non-antigenic and essentially biologically inert. PEG moieties used in the practice of the invention may be branched or unbranched.

The number of PEG moieties attached to the Sp35 antagonist polypeptide, aptamer or antibody and the molecular weight of the individual PEG chains can vary. In general, the higher the molecular weight of the polymer, the fewer polymer chains attached to the polypeptide. Usually, the total polymer mass attached to the Sp35 antagonist polypeptide aptamer or antibody is from 20 kDa to 40 kDa. Thus, if one polymer chain is attached, the molecular weight of the chain is generally 20-40 kDa. If two chains are attached, the molecular weight of each chain is generally 10-20 kDa. If three chains are attached, the molecular weight is generally 7-14 kDa.

The polymer, *e.g*., PEG, can be linked to the Sp35 antagonist polypeptide, aptamer or antibody through any suitable, exposed reactive group on the polypeptide. The exposed reactive group(s) can be, *e.g*., an N-terminal amino group or the epsilon amino group of an internal lysine residue, or both. An activated polymer can react and covalently link at any free amino group on the Sp35 antagonist polypeptide, aptamer or antibody. Free carboxylic groups, suitably activated carbonyl groups, hydroxyl, guanidyl, imidazole, oxidized carbohydrate moieties and mercapto groups of the Sp35 antagonist polypeptide, aptamer or antibody (if available) also can be used as reactive groups for polymer attachment.

In a conjugation reaction, from about 1.0 to about 10 moles of activated polymer per mole of polypeptide, depending on polypeptide concentration, is typically employed. Usually, the ratio chosen represents a balance between maximizing the reaction while minimizing side reactions (often non-specific) that can impair the desired pharmacological activity of the Sp35 antagonist polypeptide or antibody. Preferably, at least 50% of the biological activity (as demonstrated, *e.g*., in any of the assays described herein or known in the art) of the Sp35 antagonist polypeptide, aptamer or antibody is retained, and most preferably nearly 100% is retained.

The polymer can be conjugated to the Sp35 antagonist polypeptide, aptamer or antibody using conventional chemistry. For example, a polyalkylene glycol moiety can be coupled to a lysine epsilon amino group of the Sp35 antagonist polypeptide, aptamer or antibody. Linkage to the lysine side chain can be performed with an N-hydroxylsuccinimide (NHS) active ester such as PEG succinimidyl succinate (SS-PEG) and succinimidyl propionate (SPA-PEG). Suitable polyalkylene glycol moieties include, *e.g*., carboxymethyl-NHS and norleucine-NHS, SC. These reagents are commercially available. Additional amine-reactive PEG linkers can be substituted for the succinimidyl moiety. These include, *e.g*., isothiocyanates, nitrophenylcarbonates (PNP), epoxides, benzotriazole carbonates, SC-PEG, tresylate, aldehyde, epoxide, carbonylimidazole and PNP carbonate. Conditions are usually optimized to maximize the selectivity and extent of reaction. Such optimization of reaction conditions is within ordinary skill in the art.

PEGylation can be carried out by any of the PEGylation reactions known in the art. *See*, *e.g*., Focus on Growth Factors 3:4-10 (1992), and European patent applications EP 0 154 316 and EP 0 401 384. PEGylation may be carried out using an acylation reaction or an alkylation reaction with a reactive polyethylene glycol molecule (or an analogous reactive water-soluble polymer).

PEGylation by acylation generally involves reacting an active ester derivative of polyethylene glycol. Any reactive PEG molecule can be employed in the PEGylation. PEG esterified to N-hydroxysuccinimide (NHS) is a frequently used activated PEG ester. As used herein, "acylation" includes without limitation the following types of linkages between the therapeutic protein and a water-soluble polymer such as PEG: amide, carbamate, urethane, and the like. *See*, *e.g.,* Bioconjugate Chem. 5:133-140, 1994. Reaction parameters are generally selected to avoid temperature, solvent, and pH conditions that would damage or inactivate the soluble Sp35 polypeptide, aptamer or antibody.

Generally, the connecting linkage is an amide and typically at least 95% of the resulting product is mono-, di- or tri-PEGylated. However, some species with higher degrees of PEGylation may be formed in amounts depending on the specific reaction conditions used. Optionally, purified PEGylated species are separated from the mixture, particularly unreacted species, by conventional purification methods, including, *e.g*., dialysis, salting-out, ultrafiltration, ion-exchange chromatography, gel filtration chromatography, hydrophobic exchange chromatography, and electrophoresis.

PEGylation by alkylation generally involves reacting a terminal aldehyde derivative of PEG with Sp35 antagonist polypeptide, aptamer or antibody in the presence of a reducing agent. In addition, one can manipulate the reaction conditions to favor PEGylation substantially only at the N-terminal amino group of Sp35 antagonist polypeptide, aptamer or antibody, *i.e*. a mono-PEGylated protein. In either case of mono-PEGylation or poly-PEGylation, the PEG groups are typically attached to the protein via a - CH₂-NH- group. With particular reference to the - CH₂- group, this type of linkage is known as an "alkyl" linkage.

Derivatization via reductive alkylation to produce an N-terminally targeted mono-PEGylated product exploits differential reactivity of different types of primary amino groups (lysine versus the N-terminal) available for derivatization. The reaction is performed at a pH that allows one to take advantage of the pKa differences between the epsilon-amino groups of the lysine residues and that of the N-terminal amino group of the protein. By such selective derivatization, attachment of a water-soluble polymer that contains a reactive group, such as an aldehyde, to a protein is controlled: the conjugation with the polymer takes place predominantly at the N-terminus of the protein and no significant modification of other reactive groups, such as the lysine side chain amino groups, occurs.

The polymer molecules used in both the acylation and alkylation approaches are selected from among water-soluble polymers. The polymer selected is typically modified to have a single reactive group, such as an active ester for acylation or an aldehyde for alkylation, so that the degree of polymerization may be controlled as provided for in the present methods. An exemplary reactive PEG aldehyde is polyethylene glycol propionaldehyde, which is water stable, or mono C1-C10 alkoxy or aryloxy derivatives thereof (*see*, *e.g*., Harris et al., U.S. Pat. No. 5,252,714). The polymer may be branched or unbranched. For the acylation reactions, the polymer(s) selected typically have a single reactive ester group. For reductive alkylation, the polymer(s) selected typically have a single reactive aldehyde group. Generally, the water-soluble polymer will not be selected from naturally occurring glycosyl residues, because these are usually made more conveniently by mammalian recombinant expression systems.

Methods for preparing a PEGylated soluble Sp35 polypeptide, aptamer or antibody generally includes the steps of (a) reacting a Sp35 antagonist polypeptide, aptamer or antibody with polyethylene glycol (such as a reactive ester or aldehyde derivative of PEG) under conditions whereby the molecule becomes attached to one or more PEG groups, and (b) obtaining the reaction product(s). In general, the optimal reaction conditions for the acylation reactions will be determined case-by-case based on known parameters and the desired result. For example, a larger ratio of PEG to protein generally leads to a greater the percentage of poly-PEGylated product.

Reductive alkylation to produce a substantially homogeneous population of mono-polymer/soluble Sp35 polypeptide, Sp35 aptamer or Sp35 antibody generally includes the steps of: (a) reacting a soluble Sp35 protein or polypeptide with a reactive PEG molecule under reductive alkylation conditions, at a pH suitable to pen-nit selective modification of the N-terminal amino group of the polypeptide or antibody; and (b) obtaining the reaction product(s).

For a substantially homogeneous population of mono-polymer/soluble Sp35 polypeptide, Sp35 aptamer or Sp35 antibody, the reductive alkylation reaction conditions are those that permit the selective attachment of the water-soluble polymer moiety to the N-terminus of the polypeptide ,or antibody. Such reaction conditions generally provide for pKa differences between the lysine side chain amino groups and the N-terminal amino group. For purposes of the present invention, the pH is generally in the range of 3-9, typically 3-6.

Soluble Sp35 polypeptides, aptamers or antibodies can include a tag, *e.g*., a moiety that can be subsequently released by proteolysis. Thus, the lysine moiety can be selectively modified by first reacting a His-tag modified with a low-molecular-weight linker such as Traut's reagent (Pierce) which will react with both the lysine and N-terminus, and then releasing the His tag. The polypeptide will then contain a free SH group that can be selectively modified with a PEG containing a thiol-reactive head group such as a maleimide group, a vinylsulfone group, a haloacetate group, or a free or protected SH.

Traut's reagent can be replaced with any linker that will set up a specific site for PEG attachment. For example, Traut's reagent can be replaced with SPDP, SMPT, SATA, or SATP (Pierce). Similarly, one could react the protein with an amine-reactive linker that inserts a maleimide (for example SMCC, AMAS, BMPS, MBS, EMCS, SMPB, SMPH, KMUS, or GMBS), a haloacetate group (SBAP, SIA, SIAB), or a vinylsulfone group and react the resulting product with a PEG that contains a free SH.

In some embodiments, the polyalkylene glycol moiety is coupled to a cysteine group of the Sp35 antagonist polypeptide, aptamer or antibody for use in the methods of the present invention. Coupling can be effected using, *e.g.*, a maleimide group, a vinylsulfone group, a haloacetate group, or a thiol group.

Optionally, the soluble Sp35 polypeptide, aptamer or antibody is conjugated to the polyethylene-glycol moiety through a labile bond. The labile bond can be cleaved in, *e.g*., biochemical hydrolysis, proteolysis, or sulfhydryl cleavage. For example, the bond can be cleaved under *in vivo* (physiological) conditions.

The reactions may take place by any suitable method used for reacting biologically active materials with inert polymers, generally at about pH 5-8, *e.g*., pH 5, 6, 7, or 8, if the reactive groups are on the alpha amino group at the N-terminus. Generally the process involves preparing an activated polymer and thereafter reacting the protein with the activated polymer to produce the soluble protein suitable for formulation.

### Sp35 Polynucleotide Antagonists

Sp35 antagonists in the methods of the present invention include an Sp35 polynucleotide antagonist which comprises a nucleic acid molecule which specifically binds to a polynucleotide which encodes Sp35. The Sp35 polynucleotide antagonist prevents expression of Sp35 (knockdown). Sp35 polynucleotide antagonists include, but are not limited to antisense molecules, ribozymes, siRNA, shRNA, RNAi. Typically, such binding molecules are separately administered to the animal (*see*, for example, O'Connor, J. Neurochem. 56:560 (1991), but such binding molecules may also be expressed *in vivo* from polynucleotides taken up by a host cell and expressed *in vivo*. *See also* Oligodeoxynucleotides as Antisense Inhibitors of Gene Expression, CRC Press, Boca Raton, FL (1988).

RNAi refers to the expression of an RNA which interferes with the expression of the targeted mRNA. Specifically, the RNAi silences a targeted gene via interacting with the specific mRNA (*e.g*. Sp35) through an siRNA (short interfering RNA). The ds RNA complex is then targeted for degradation by the cell. Additional RNAi molecules include short hairpin RNA (shRNA); also short interfering hairpin. The shRNA molecule contains sense and antisense sequences from a target gene connected by a loop. The shRNA is transported from the nucleus into the cytoplasm, it is degraded along with the mRNA. Pol III or U6 promoters can be used to express RNAs for RNAi. In some embodiments of the invention, the shRNA is expressed from a lentiviral vector (*e.g*. pLL3.7).

RNAi is mediated by double stranded RNA (dsRNA) molecules that have sequence-specific homology to their "target" mRNAs (Caplen et al., Proc Natl Acad Sci USA 98:9742-9747, 2001). Biochemical studies in Drosophila cell-free lysates indicates that the mediators of RNA-dependent gene silencing are 21-25 nucleotide "small interfering" RNA duplexes (siRNAs). Accordingly, siRNA molecules are advantageously used in the methods of the present invention. The siRNAs are derived from the processing of dsRNA by an RNase known as DICER (Bernstein et al., Nature 409:363-366, 2001). It appears that siRNA duplex products are recruited into a multi-protein siRNA complex termed RISC (RNA Induced Silencing Complex). Without wishing to be bound by any particular theory, it is believed that a RISC is guided to a target mRNA, where the siRNA duplex interacts sequence-specifically to mediate cleavage in a catalytic fashion (Bernstein et al., Nature 409:363-366,2001; Boutla et al., Curr Biol 11:1776-1780, 2001).

RNAi has been used to analyze gene function and to identify essential genes in mammalian cells (Elbashir et al., Methods 26:199-213, 2002; Harborth et al., J Cell Sci 114:4557-4565, 2001), including by way of non-limiting example neurons (Krichevsky et al., Proc Natl Acad Sci USA 99:11926-11929, 2002). RNAi is also being evaluated for therapeutic modalities, such as inhibiting or blocking the infection, replication and/or growth of viruses, including without limitation poliovirus (Gitlin et al., Nature 418:379-3 80, 2002) and HIV (Capodici et al., J Immunol 169:5196-5201, 2002), and reducing expression of oncogenes (*e.g*., the bcr-abl gene; Scherr et al., Blood 101(4):1566-9, 2002). RNAi has been used to modulate gene expression in mammalian (mouse) and amphibian (*Xenopus*) embryos (respectively, Calegari et al., Proc Natl Acad Sci USA 99:14236-14240, 2002; and Zhou, et al., Nucleic Acids Res 30:1664-1669, 2002), and in postnatal mice (Lewis et al., Nat Genet 32:107-108, 2002), and to reduce transgene expression in adult transgenic mice (McCaffrey et al., Nature 418:38-39, 2002). Methods have been described for determining the efficacy and specificity of siRNAs in cell culture and *in vivo* (see, *e.g*., Bertrand et al., Biochem Biophys Res Commun 296:1000-1004, 2002; Lassus et al., Sci STKE 2002(147):PL13, 2002; and Leirdal et al., Biochem Biophys Res Commun 295:744-748, 2002).

Molecules that mediate RNAi, including without limitation siRNA, can be produced *in vitro* by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as E. coli RNase III (Yang et al., Proc Natl Acad Sci USA 99:9942-9947, 2002).

siRNA molecules may also be formed by annealing two oligonucleotides to each other, typically have the following general structure, which includes both double-stranded and single-stranded portions:

|—*m*—| (Overhang)
|————*x*————| ("Core")
5'-XXXXXXXXXXXXXXNNNNN-3' (SEQ ID NO:4)

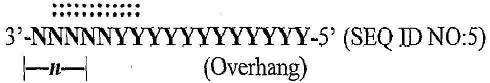
3'-NNNNNYYYYYYYYYYY-5' (SEQ ID NO:5)
|—n—|          (Overhang)

Wherein N, X and Y are nucleotides; X hydrogen bonds to Y; ":" signifies a hydrogen bond between two bases; *x* is a natural integer having a value between 1 and about 100; and ***m*** and ***n*** are whole integers having, independently, values between 0 and about 100. In some embodiments, N, X and Y are independently A, G, C and T or U. Non-naturally occurring bases and nucleotides can be present, particularly in the case of synthetic siRNA (*i.e*., the product of annealing two oligonucleotides). The double-stranded central section is called the "core" and has base pairs (bp) as units of measurement; the single-stranded portions are overhangs, having nucleotides (nt) as units of measurement. The overhangs shown are 3' overhangs, but molecules with 5' overhangs are also within the scope of the invention. Also within the scope of the invention are siRNA molecules with no overhangs (*i.e*., ***m*** = 0 and ***n*** = 0), and those having an overhang on one side of the core but not the other (*e.g*., ***m*** = 0 and ***n*** ≥ 1, or vice-versa).

Initially, RNAi technology did not appear to be readily applicable to mammalian systems. This is because, in mammals, dsRNA activates dsRNA-activated protein kinase (PKR) resulting in an apoptotic cascade and cell death (Der et al, Proc. Natl. Acad. Sci. USA 94:3279-3283, 1997). In addition, it has long been known that dsRNA activates the interferon cascade in mammalian cells, which can also lead to altered cell physiology (Colby et al, Annu. Rev. Microbiol. 25:333, 1971; Kleinschmidt et al., Annu. Rev. Biochem. 41:517, 1972; Lampson et al., Proc. Natl. Acad. Sci. USA 58L782, 1967; Lomniczi et al., J. Gen. Virol. 8:55, 1970; and Younger et al., J. Bacteriol. 92:862, 1966). However, dsRNA-mediated activation of the PKR and interferon cascades requires dsRNA longer than about 30 base pairs. In contrast, dsRNA less than 30 base pairs in length has been demonstrated to cause RNAi in mammalian cells (Caplen et al., Proc. Natl. Acad. Sci. USA 98:9742-9747, 2001). Thus, it is expected that undesirable, non-specific effects associated with longer dsRNA molecules can be avoided by preparing short RNA that is substantially free from longer dsRNAs.

References regarding siRNA: Bernstein et al., Nature 409:363-366, 2001; Boutla et al., Curr Biol 11:1776-1780, 2001; Cullen, Nat Immunol. 3:597-599, 2002; Caplen et al., Proc Natl Acad Sci USA 98:9742-9747, 2001; Hamilton et al., Science 286:950-952, 1999; Nagase et al., DNA Res. 6:63-70, 1999; Napoli et al., Plant Cell 2:279-289, 1990; Nicholson et al., Mamm. Genome 13:67-73, 2002; Parrish et al., Mol Cell 6:1077-1087, 2000; Romano et al., Mol Microbiol 6:3343-3353, 1992; Tabara et al., Cell 99:123-132, 1999; and Tuschl, Chembiochem. 2:239-245, 2001.

Paddison et al. (Genes & Dev. 16:948-958, 2002) have used small RNA molecules folded into hairpins as a means to effect RNAi. Accordingly, such short hairpin RNA (shRNA) molecules are also advantageously used in the methods of the invention. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the dsRNA products of the DICER RNase and, in any event, have the same capacity for inhibiting expression of a specific gene.

Antisense technology can be used to control gene expression through antisense DNA or RNA, or through triple-helix formation. Antisense techniques are discussed, for example, in Okano, J. Neurochem. 56:560 (1991); Oligodeoxynucleotides as Antisense Inhibitors of Gene Expression, CRC Press, Boca Raton, FL (1988). Triple helix formation is discussed in, for instance, Lee et al., Nucleic Acids Research 6:3073 (1979); Cooney et al., Science 241:456 (1988); and Dervan et al., Science 251:1300 (1991). The methods are based on binding of a polynucleotide to a complementary DNA or RNA.

For example, the 5' coding portion of a polynucleotide that encodes Sp35 may be used to design an antisense RNA oligonucleotide of from about 10 to 40 base pairs in length. A DNA oligonucleotide is designed to be complementary to a region of the gene involved in transcription, thereby preventing transcription and the production of the target protein. The antisense RNA oligonucleotide hybridizes to the mRNA *in vivo* and blocks translation of the mRNA molecule into the target polypeptide.

In one embodiment, antisense nucleic acids specific for the Sp35 gene are produced intracellularly by transcription from an exogenous sequence. For example, a vector or a portion thereof, is transcribed, producing an antisense nucleic acid (RNA). Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the antisense molecule, can be by any promoter known in the art to act in vertebrate, preferably human cells, such as those described elsewhere herein.

Absolute complementarity of an antisense molecule, although preferred, is not required. A sequence complementary to at least a portion of an RNA encoding Sp35 means a sequence having sufficient complementarity to be able to hybridize with the RNA, forming a stable duplex; or triplex formation may be assayed. The ability to hybridize will depend on both the degree of complementarity and the length of the antisense nucleic acid. Generally, the larger the hybridizing nucleic acid, the more base mismatches it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of a messenger RNA, *e.g*., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. *See*, generally, Wagner, R., Nature 372:333-335 (1994). Thus, oligonucleotides complementary to either the 5'- or 3'- non-translated, non-coding regions could be used in an antisense approach to inhibit translation of Sp35. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

In yet another embodiment, an antisense oligonucleotide for use in the methods disclosed herein is an α-anomeric oligonucleotide. An α-anomeric oligonucleotide forms specific double-stranded hybrids with complementary RNA in which, contrary to the usual situation, the strands run parallel to each other (Gautier et al., Nucl. Acids Res. 15:6625-6641(1987)). The oligonucleotide is a 2'-O-methylribonucleotide (Inoue et al., Nucl. Acids Res. 15:6131-6148(1987)), or a chimeric RNA-DNA analogue (Inoue et al., FEBS Lett. 215:327-330(1987)).

Polynucleotide compositions for use in the methods disclosed herein further include catalytic RNA, or a ribozyme (*See*, *e.g*., PCT International Publication WO 90/11364, published October 4, 1990; Sarver et al., Science 247:1222-1225 (1990). The use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, Nature 334:585-591 (1988). Preferably, the ribozyme is engineered so that the cleavage recognition site is located near the 5' end of the target mRNA; *i.e*., to increase efficiency and minimize the intracellular accumulation of non-functional mRNA transcripts.

As in the antisense approach, ribozymes for use in the methods disclosed herein can be composed of modified oligonucleotides (*e.g*. for improved stability, targeting, etc.) and may be delivered to cells which express Sp35 *in vivo.* DNA constructs encoding the ribozyme may be introduced into the cell in the same manner as described above for the introduction of antisense encoding DNA. A preferred method of delivery involves using a DNA construct "encoding" the ribozyme under the control of a strong constitutive promoter, such as, for example, pol III or pol II promoter, so that transfected cells will produce sufficient quantities of the ribozyme to destroy endogenous Sp35 messages and inhibit translation. Since ribozymes, unlike antisense molecules, are catalytic, a lower intracellular concentration is required for efficiency.

Polynucleotides for use in the methods disclosed herein, including aptamers described below, can be DNA or RNA or chimeric mixtures or derivatives or modified versions thereof, single-stranded or double-stranded. The polynucleotide can be modified at the base moiety, sugar moiety, or phosphate backbone, for example, to improve stability of the molecule, hybridization, etc. The polynucleotide may include other appended groups such as peptides (*e.g*., for targeting host cell receptors *in vivo*), or agents facilitating transport across the cell membrane (see, *e.g*., Letsinger et al., Proc. Natl. Acad. Sci. U.S.A. 86:6553-6556 (1989); Lemaitre et al., Proc. Natl. Acad. Sci. 84:648-652 (1987)); PCT Publication No. WO88/09810, published December 15, 1988) or the blood-brain barrier (see, *e.g*., PCT Publication No. WO89/10134 published April 25, 1988), hybridization-triggered cleavage agents. (*See*, *e.g*., Krol et al., BioTechniques 6:958-976 (1988)) or intercalating agents. (*See*, *e.g*., Zon, Pharm. Res. 5:539-549(1988)). To this end, the polynucleotide may be conjugated to another molecule, *e.g*., a peptide, hybridization triggered cross-linking agent, transport agent, hybridization-triggered cleavage agent, etc.

Polynucleotides, including aptamers, for use in the methods disclosed herein may comprise at least one modified base moiety which is selected from the group including, but not limited to, 5fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N-6-isopentenyladenine, 1-methylguanine, 1-methylinosine, 2,2-dimethylguanine, 2-methyladenine, 2-methylguanine, 3-methylcytosine, 5-methylcytosine, N-6-adenine, 7-methylguanine, 5-methylaminomethyluracil, 5-methoxyaminomethyl-2-thiouracil, beta-D-mannosylqueosine, 5'methoxycarboxymethyluracil, 5-methoxyuracil, 2-methylthio-N-6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3(3-amino-3-N2-carboxypropyl) uracil, (acp3)w, and 2,6-diaminopurine.

Polynucleotides, including aptamers, for use in the methods disclosed herein may also comprise at least one modified sugar moiety selected from the group including, but not limited to, arabinose, 2-fluoroarabinose, xylulose, and hexose.

In yet another embodiment, a polynucleotide, including an aptamer, for use in the methods disclosed herein comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

Polynucleotides, including aptamers, for use in the methods of the invention may be synthesized by standard methods known in the art, *e.g*. by use of an automated DNA synthesizer (such as are commercially available from Biosearch, Applied Biosystems, etc.). As examples, phosphorothioate oligonucleotides may be synthesized by the method of Stein et al., Nucl. Acids Res. 16:3209 (1988), methylphosphonate oligonucleotides can be prepared by use of controlled pore glass polymer supports (Sarin et al., Proc. Natl. Acad. Sci. U.S.A. 85:7448-7451(1988)), etc.

### Aptamers

In another embodiment, the Sp35 antagonist for use in the methods of the present invention is an aptamer. An aptamer can be a nucleotide or a polypeptide which has a unique sequence, has the property of binding specifically to a desired target (e.g. a polypeptide), and is a specific ligand of a given target. Nucleotide aptamers of the invention include double stranded DNA and single stranded RNA molecules that bind to Sp35.

Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in e.g. U.S. Pat. Nos. 5,475,096, 5,580,737, 5,567,588, 5,707,796, 5,763,177, 6, 011,577, and 6,699,843, incorporated herein by reference in their entirety. Another screening method to identify aptamers is described in U.S. Pat. No. 5,270,163 (also incorporated herein by reference). The SELEX process is based on the capacity of nucleic acids for forming a variety of two- and three-dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets.

The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, preferably comprising a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield highly specific high affinity nucleic acid ligands to the target molecule.

Nucleotide aptamers may be used, for example, as diagnostic tools or as specific inhibitors to dissect intracellular signaling and transport pathways (James (2001) Curr. Opin. Pharmacol. 1:540-546). The high affinity and specificity of nucleotide aptamers makes them good candidates for drug discovery. For example, aptamer antagonists to the toxin ricin have been isolated and have IC50 values in the nanomolar range (Hesselberth JR et al. (2000) J Biol Chem 275:4937-4942). Nucleotide aptamers may also be used against infectious disease, malignancy and viral surface proteins to,reduce cellular infectivity.

Nucleotide aptamers for use in the methods of the present invention may be modified (e.g., by modifying the backbone or bases or conjugated to peptides) as described herein for other polynucleotides.

Using the protein structure of Sp35, screening for aptamers that act on Sp35 using the SELEX process would allow for the identification of aptamers that inhibit Sp35-mediated processes (e.g. Sp35-mediated inhibition of axonal regeneration).

Polypeptide aptamers for use in the methods of the present invention are random peptides selected for their ability to bind to and thereby block the action of Sp35. Polypeptide aptamers may include a short variable peptide domain attached at both ends to a protein scaffold. This double structural constraint greatly increases the binding affinity of the peptide aptamer to levels comparable to an antibody's (nanomolar range). See, e.g., Hoppe-Seyler F et al. (2000) J Mol Med 78(8):426-430. The length of the short variable peptide is typically about 10 to 20 amino acids, and the scaffold may be any protein which has good solubility and compacity properties. One non-limiting example of a scaffold protein is the bacterial protein Thioredoxin-A. *See*, *e.g*., Cohen BA et al. (1998) PNAS 95(24): 14272-14277. An additional, non-limiting example, of a polypeptide aptamer for use in the methods of the present invention is a Ligand Regulated Peptide Aptamer (LiRPA). The LiRPA scaffold may be composed of three protein domains: FK506 binding protein (FKBP), FRBP-Rapamycin binding domain (FRB) and glutathione-S-transferase (GST). *See*, *e.g.,* Binkowski BF et al., (2005) Chem & Biol 12(7): 847-855, incorporated herein by reference.

Polypeptide aptamers are peptides or small polypeptides that act as dominant inhibitors of protein function. Peptide aptamers specifically bind to target proteins, blocking their functional ability (Kolonin et al. (1998) Proc. Natl. Acad. Sci. 95: 14,266-14,271). Peptide aptamers that bind with high affinity and specificity to a target protein can be isolated by a variety of techniques known in the art. Peptide aptamers can be isolated from random peptide libraries by yeast two-hybrid screens (Xu, C.W., et al. (1997) Proc. Natl. Acad. Sci. 94:12,473-12,478) or by ribosome display (Hanes et al. (1997) Proc. Natl. Acad. Sci. 94:4937-4942). They can also be isolated from phage libraries (Hoogenboom, H.R., et al. (1998) Immunotechnology 4:1-20) or chemically generated peptide libraries. Although the difficult means by which peptide aptamers are synthesized makes their use more complex than polynucleotide aptamers, they have unlimited chemical diversity.

Peptide aptamers for use in the methods of the present invention may be modified (e.g., conjugated to polymers or fused to proteins) as described for other polypeptides elsewhere herein.

### Vectors

Vectors comprising nucleic acids encoding Sp35 antagonists may also be used to produce antagonists for use in the methods of the invention. The choice of vector and expression control sequences to which such nucleic acids are operably linked depends on the functional properties desired, *e.g*., protein expression, and the host cell to be transformed.

Expression control elements useful for regulating the expression of an operably linked coding sequence are known in the art. Examples include, but are not limited to, inducible promoters, constitutive promoters, secretion signals, and other regulatory elements. When an inducible promoter is used, it can be controlled, *e.g*., by a change in nutrient status, or a change in temperature, in the host cell medium.

The vector can include a prokaryotic replicon, *i.e.,* a DNA sequence having the ability to direct autonomous replication and maintenance of the recombinant DNA molecule extra-chromosomally in a bacterial host cell. Such replicons are well known in the art. In addition, vectors that include a prokaryotic replicon may also include a gene whose expression confers a detectable marker such as a drug resistance. Examples of bacterial drug-resistance genes are those that confer resistance to ampicillin or tetracycline.

Vectors that include a prokaryotic replicon can also include a prokaryotic or bacteriophage promoter for directing expression of the coding gene sequences in a bacterial host cell. Promoter sequences compatible with bacterial hosts are typically provided in plasmid vectors containing convenient restriction sites for insertion of a DNA segment to be expressed. Examples of such plasmid vectors are pUC8, pUC9, pBR322 and pBR329 (BioRad), pPL and pKK223 (Pharmacia). Any suitable prokaryotic host can be used to express a recombinant DNA molecule encoding a protein used in the methods of the invention.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (*e.g*., antibiotics) or resistance to heavy metals such as copper. The selectable marker gene can either be directly linked to the DNA sequences to be expressed, or introduced into the same cell by cotransformation. The neomycin phosphotransferase (neo) gene is an example of a selectable marker gene (Southern et al., J. Mol. Anal. Genet. 1:327-341 (1982)). Additional elements may also be needed for optimal synthesis of mRNA. These elements may include signal sequences or splice signals, as well as transcriptional promoters, enhancers, and termination signals.

In one embodiment, a proprietary expression vector of Biogen IDEC, Inc., referred to as NEOSPLA (U.S. patent 6,159,730) may be used. This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. This vector has been found to result in very high-level expression upon transfection in CHO cells, followed by selection in G418-containing medium and methotrexate amplification. Of course, any expression vector which is capable of eliciting expression in eukaryotic cells may be used in the present invention. Examples of suitable vectors include, but are not limited to, plasmids pcDNA3, pHCMV/Zeo, pCR3.1, pEF1/His, pIND/GS, pRc/HCMV2, pSV40/Zeo2, pTRACER-HCMV, pUB6/V5-His, pVAX1, and pZeoSV2 (available from Invitrogen, San Diego, CA), and plasmid pCI (available from Promega, Madison, WI). Additional eukaryotic cell expression vectors are known in the art and are commercially available. Typically, such vectors contain convenient restriction sites for insertion of the desired DNA segment. Exemplary vectors include pSVL and pKSV-10 (Pharmacia), pBPV-1, pm12d (International Biotechnologies), pTDT1 (ATCC 31255), retroviral expression vector pMIG and pLL3.7, adenovirus shuttle vector pDC315, and AAV vectors. Other exemplary vector systems are disclosed *e.g*., in U.S. Patent 6,413,777.

In general, screening large numbers of transformed cells for those which express suitably high levels of the antagonist is routine experimentation which can be carried out, for example, by robotic systems.

Frequently used regulatory sequences for mammalian host cell expression include viral elements that direct high levels of protein expression in mammalian cells, such as promoters and enhancers derived from retroviral LTRs, cytomegalovirus (CMV) (such as the CMV promoter/enhancer), Simian Virus 40 (SV40) (such as the SV40 promoter/enhancer), adenovirus, (*e.g*., the adenovirus major late promoter (AdmlP)), polyoma and strong mammalian promoters such as native immunoglobulin and actin promoters. For further description of viral regulatory elements, and sequences thereof, *see, e.g.,* Stinski, U.S. Pat. No. 5,168,062; Bell, U.S. Pat. No. 4,510,245; and Schaffner, U.S. Pat. No. 4,968,615.

The recombinant expression vectors may carry sequences that regulate replication of the vector in host cells (*e.g*., origins of replication) and selectable marker genes. The selectable marker gene facilitates selection of host cells into which the vector has been introduced (*see, e.g.,* Axel, U.S. Pat. Nos. 4,399,216; 4,634,665 and 5,179,017). For example, typically the selectable marker gene confers resistance to a drug, such as G418, hygromycin or methotrexate, on a host cell into which the vector has been introduced. Frequently used selectable marker genes include the dihydrofolate reductase (DHFR) gene (for use in dhfr- host cells with methotrexate selection/amplification) and the neo gene (for G418 selection).

Vectors encoding Sp35 antagonists can be used for transformation of a suitable host cell. Transformation can be by any suitable method. Methods for introduction of exogenous DNA into mammalian cells are well known in the art and include dextran-mediated transfection, calcium phosphate precipitation, polybrene-mediated transfection, protoplast fusion, electroporation, transfection via encapsulation of the polynucleotide(s) in liposomes, and direct microinjection of the DNA into nuclei. In addition, nucleic acid molecules may be introduced into mammalian cells by viral vectors. Mammalian cells may also be transduced by recombinant viruses containing the exogenous DNA which is to be introduced into the mammalian cells.

Transformation of host cells can be accomplished by conventional methods suited to the vector and host cell employed. For transformation of prokaryotic host cells, electroporation and salt treatment methods can be employed (Cohen et al., Proc. Natl. Acad. Sci. USA 69:2110-14 (1972)). For transformation of vertebrate cells, electroporation, cationic lipid or salt treatment methods can be employed. *See, e.g.,* Graham et al., Virology 52:456-467 (1973); Wigler et al., Proc. Natl. Acad. Sci. USA 76:1373-76 (1979).

The host cell line used for protein expression is most preferably of mammalian origin; those skilled in the art are credited with ability to preferentially determine particular host cell lines which are best suited for the desired gene product to be expressed therein. Exemplary host cell lines include, but are not limited to, NSO, SP2 cells, baby hamster kidney (BHK) cells, monkey kidney cells (COS), human hepatocellular carcinoma cells (e.g., Hep G2), A549 cells DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3x63-Ag3.653 (mouse myeloma), BFA-1c1BPT (bovine endothelial cells), RAJI (human lymphocyte) and 293 (human kidney). Host cell lines are typically available from commercial services, the American Tissue Culture Collection or from published literature.

Expression of polypeptides from production cell lines can be enhanced using known techniques. For example, the glutamine synthetase (GS) system is commonly used for enhancing expression under certain conditions. *See, e.g.,* European Patent Nos. 0 216 846, 0 256 055, and 0 323 997 and European Patent Application No. 89303964.4.

### Host Cells

Host cells for expression of an Sp35 antagonist for use in a method of the invention may be prokaryotic or eukaryotic. Exemplary eukaryotic host cells include, but are not limited to, yeast and mammalian cells, *e.g*., Chinese hamster ovary (CHO) cells (ATCC Accession No. CCL61), NIH Swiss mouse embryo cells NIH-3T3 (ATCC Accession No. CRL1658), and baby hamster kidney cells (BHK). Other useful eukaryotic host cells include insect cells and plant cells. Exemplary prokaryotic host cells are *E. coli* and *Streptomyces.*

### Gene Therapy

An Sp35 antagonist can be produced *in vivo* in a mammal, *e.g*., a human patient, using a gene-therapy approach to treatment of a disease, disorder or injury associated with DA neuronal degeneration, death or lack or regeneration: This involves administration of a suitable Sp35 antagonist-encoding nucleic acid operably linked to suitable expression control sequences. Generally, these sequences are incorporated into a viral vector. Suitable viral vectors for such gene therapy include an adenoviral vector, an alphavirus vector, an enterovirus vector, a pestivirus vector, a lentiviral vector, a baculoviral vector, a herpesvirus vector, an Epstein Barr viral vector, a papovaviral vector, a poxvirus vector, a vaccinia viral vector, an adeno-associated viral vector and a herpes simplex viral vector. The viral vector can be a replication-defective viral vector. Adenoviral vectors that have a deletion in their E1 gene or E3 genes are typically used. When an adenoviral vector is used, the vector usually does not have a selectable marker gene.

### Pharmaceutical Compositions

The Sp35 antagonists used in the methods of the invention may be formulated into pharmaceutical compositions for administration to mammals, including humans. The pharmaceutical compositions used in the methods of this invention comprise pharmaceutically acceptable carriers, including, *e.g*., ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions used in the methods of the present invention may be administered by any suitable method, *e.g*., parenterally, intraventricularly, orally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. As described previously, Sp35 antagonists used in the methods of the invention act in the nervous system to promote survival, regeneration and differentiation of oligodendrocytes and myelination of neurons. Accordingly, in the methods of the invention, the Sp35 antagonists are administered in such a way that they cross the blood-brain barrier. This crossing can result from the physico-chemical properties inherent in the Sp35 antagonist molecule itself, from other components in a pharmaceutical formulation, or from the use of a mechanical device such as a needle, cannula or surgical instruments to breach the blood-brain barrier. Where the Sp35 antagonist is a molecule that does not inherently cross the blood-brain barrier, *e.g*., a fusion to a moiety that facilitates the crossing, suitable routes of administration are, *e.g*., intrathecal or intracranial, *e.g*., directly into a chronic lesion of MS. Where the Sp35 antagonist is a molecule that inherently crosses the blood-brain barrier, the route of administration may be by one or more of the various routes described below.

Sterile injectable forms of the compositions used in the methods of this invention may be aqueous or oleaginous suspension. These suspensions may be formulated according to techniques known in the art using suitable dispersing or wetting agents and suspending agents. The sterile, injectable preparation may also be a sterile, injectable solution or suspension in a non-toxic parenterally acceptable diluent or solvent, for example as a suspension in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Parenteral formulations may be a single bolus dose, an infusion or a loading bolus dose followed with a maintenance dose. These compositions may be administered at specific fixed or variable intervals, *e.g*., once a day, or on an "as needed" basis.

Certain pharmaceutical compositions used in the methods of this invention may be orally administered in an acceptable dosage form including, *e.g*., capsules, tablets, aqueous suspensions or solutions. Certain pharmaceutical compositions also may be administered by nasal aerosol or inhalation. Such compositions may be prepared as solutions in saline, employing benzyl alcohol or other suitable preservatives, absorption promoters to enhance bioavailability, and/or other conventional solubilizing or dispersing agents.

The amount of an Sp35 antagonist that may be combined with the carrier materials to produce a single dosage form will vary depending upon the host treated, the type of antagonist used and the particular mode of administration. The composition may be administered as a single dose, multiple doses or over an established period of time in an infusion. Dosage regimens also may be adjusted to provide the optimum desired response (*e.g*., a therapeutic or prophylactic response).

The methods of the invention use a "therapeutically effective amount" or a "prophylactically effective amount" of an Sp35 antagonist. Such a therapeutically or prophylactically effective amount may vary according to factors such as the disease state, age, sex, and weight of the individual. A therapeutically or prophylactically effective amount is also one in which any toxic or detrimental effects are outweighed by the therapeutically beneficial effects.

A specific dosage and treatment regimen for any particular patient will depend upon a variety of factors, including the particular Sp35 antagonist used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

In the methods of the invention the Sp35 antagonists are generally administered directly to the nervous system, intracerebroventricularly, or intrathecally, *e.g*. into a chronic lesion of MS. Compositions for administration according to the methods of the invention can be formulated so that a dosage of 0.001- 10 mg/kg body weight per day of the Sp35 antagonist polypeptide is administered. In some embodiments of the invention, the dosage is 0.01- 1.0 mg/kg body weight per day. In some embodiments, the dosage is 0.001- 0.5 mg/kg body weight per day.

For treatment with an Sp35 antagonist antibody, the dosage can range, *e.g*., from about 0.0001 to 100 mg/kg, and more usually 0.01 to 5 mg/kg (*e.g*., 0.02 mg/kg, 0.25 mg/kg, 0.5 mg/kg, 0.75 mg/kg, 1mg/kg, 2 mg/kg, etc.), of the host body weight. For example, dosages can be 1 mg/kg body weight or 10 mg/kg body weight or within the range of 1-10 mg/kg, preferably at least 1 mg/kg. Doses intermediate in the above ranges are also intended to be within the scope of the invention. Subjects can be administered such doses daily, on alternative days, weekly or according to any other schedule determined by empirical analysis. An exemplary treatment entails administration in multiple dosages over a prolonged period, for example, of at least six months. Additional exemplary treatment regimes entail administration once every two weeks or once a month or once every 3 to 6 months. Exemplary dosage schedules include 1-10 mg/kg or 15 mg/kg on consecutive days, 30 mg/kg on alternate days or 60 mg/kg weekly. In some methods, two or more monoclonal antibodies with different binding specificities are administered simultaneously, in which case the dosage of each antibody administered falls within the ranges indicated.

In certain embodiments, a subject can be treated with a nucleic acid molecule encoding a Sp35 antagonist polynucleotide. Doses for nucleic acids range from about 10 ng to 1 g, 100 ng to 100 mg, 1 µg to 10 mg, or 30-300 µg DNA per patient. Doses for infectious viral vectors vary from 10-100, or more, virions per dose.

Supplementary active compounds also can be incorporated into the compositions used in the methods of the invention. For example, a soluble Sp35 polypeptide or a fusion protein may be coformulated with and/or coadministered with one or more additional therapeutic agents.

The invention encompasses any suitable delivery method for an Sp35 antagonist to a selected target tissue, including bolus injection of an aqueous solution or implantation of a controlled-release system. Use of a controlled-release implant reduces the need for repeat injections.

The Sp35 antagonists used in the methods of the invention may be directly infused into the brain. Various implants for direct brain infusion of compounds are known and are effective in the delivery of therapeutic compounds to human patients suffering from neurological disorders. These include chronic infusion into the brain using a pump, stereotactically implanted, temporary interstitial catheters, permanent intracranial catheter implants, and surgically implanted biodegradable implants. *See, e.g.,* Gill *et al., supra;* Scharfen et al., "High Activity Iodine-125 Interstitial Implant For Gliomas," Int. J. Radiation Oncology Biol. Phys. 24(4):583-591 (1992); Gaspar et al., "Permanent 125I Implants for Recurrent Malignant Gliomas," Int. J. Radiation Oncology Biol. Phys. 43(5):977-982 (1999); chapter 66, pages 577-580, Bellezza et al., "Stereotactic Interstitial Brachytherapy," in Gildenberg et al., Textbook of Stereotactic and Functional Neurosurgery, McGraw-Hill (1998); and Brem et al., "The Safety of Interstitial Chemotherapy with BCNU-Loaded Polymer Followed by Radiation Therapy in the Treatment of Newly Diagnosed Malignant Gliomas: Phase I Trial," J. Neuro-Oncology 26:111-23 (1995).

The compositions may also comprise an Sp35 antagonist dispersed in a biocompatible carrier material that functions as a suitable delivery or support system for the compounds. Suitable examples of sustained release carriers include semipermeable polymer matrices in the form of shaped articles such as suppositories or capsules. Implantable or microcapsular sustained release matrices include polylactides (U.S. Patent No. 3,773,319; EP 58,481), copolymers of L-glutamic acid and gamma-ethyl-L-glutamate (Sidman et al., Biopolymers 22:547-56 (1985)); poly(2-hydroxyethylmethacrylate), ethylene vinyl acetate (Langer et al., J. Biomed. Mater. Res. 15:167-277 (1981); Langer, Chem. Tech. 12:98-105 (1982)) or poly-D-(-)-3hydroxybutyric acid (EP 133,988).

In some embodiments of the invention, an Sp35 antagonist is administered to a patient by direct infusion into an appropriate region of the brain. *See, e.g.,* Gill et al., "Direct brain infusion of glial cell line-derived neurotrophic factor in Parkinson disease," Nature Med. 9: 589-95 (2003). Alternative techniques are available and may be applied to administer an Sp35 antagonist according to the invention. For example, stereotactic placement of a catheter or implant can be accomplished using the Riechert-Mundinger unit and the ZD (Zamorano-Dujovny) multipurpose localizing unit. A contrast-enhanced computerized tomography (CT) scan, injecting 120 ml of omnipaque, 350 mg iodine/ml, with 2 mm slice thickness can allow three-dimensional multiplanar treatment planning (STP, Fischer, Freiburg, Germany). This equipment permits planning on the basis of magnetic resonance imaging studies, merging the CT and MRI target information for clear target confirmation.

The Leksell stereotactic system (Downs Surgical, Inc., Decatur, GA) modified for use with a GE CT scanner (General Electric Company, Milwaukee, WI) as well as the Brown-Roberts-Wells (BRW) stereotactic system (Radionics, Burlington, MA) can be used for this purpose. Thus, on the morning of the implant, the annular base ring of the BRW stereotactic frame can be attached to the patient's skull. Serial CT sections can be obtained at 3 mm intervals though the (target tissue) region with a graphite rod localizer frame clamped to the base plate. A computerized treatment planning program can be run on a VAX 11/780 computer (Digital Equipment Corporation, Maynard, Mass.) using CT coordinates of the graphite rod images to map between CT space and BRW space.

The methods of treatment of disorders as described herein are typically tested *in vitro,* and then *in vivo* in an acceptable animal model, for the desired therapeutic or prophylactic activity, prior to use in humans. Suitable animal models, including transgenic animals, are will known to those of ordinary skill in the art. For example, *in vitro* assays to demonstrate the differentiation and survival effect of the Sp35 antagonists are described herein. The effect of the Sp35 antagonists on myelination of axons can be tested *in vitro* as described in the Examples. Finally, *in vivo* tests can be performed by creating transgenic mice which express the Sp35 antagonist or by administering the Sp35 antagonist to mice or rats in models as described herein.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning: A Laboratory Manual (3-Volume Set), J. Sambrook, D. W. Russell, Cold Spring Harbor Laboratory Press (2001); Genes VIII, B. Lewin, Prentice Hall (2003); PCR Primer, C.W. Dieffenbach and G.S. Dveksler, CSHL Press (2003); DNA Cloning, D. N. Glover ed., Volumes I and II (1985); Oligonucleotide Synthesis: Methods and Applications (Methods in Molecular Biology), P. Herdewijn (Ed.), Humana Press (2004); Culture of Animal Cells: A Manual of Basic Technique, 4th edition, R. I. Freshney, Wiley-Liss (2000); Oligonucleotide Synthesis, M. J. Gait (Ed.), (1984); Mullis et al. U.S. Pat. No: 4,683,195; Nucleic Acid Hybridization, B. D. Hames & S. J. Higgins eds. (1984); Nucleic Acid Hybridization, M. L. M. Anderson, Springer (1999); Animal Cell Culture and Technology, 2nd edition, M. Butler, BIOS Scientific Publishers (2004); Immobilized Cells and Enzymes: A Practical Approach (Practical Approach Series), J. Woodward, Irl Pr (1992); Transcription And Translation, B. D. Hames & S. J. Higgins (Eds.) (1984); Culture Of Animal Cells, R. I. Freshney, Alan R. Liss, Inc., (1987); Immobilized Cells And Enzymes, IRL Press, (1986); A Practical Guide To Molecular Cloning, 3rd edition, B. Perbal, John Wiley & Sons Inc. (1988); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155, Wu et al. (Eds.); Immunochemical Methods In Cell And Molecular Biology, Mayer and Walker, (Eds.), Academic Press, London (1987); Handbook Of Experimental Immunology, Volumes I-IV, D. M. Weir and C.C. Blackwell (Eds.), (1986); Immunology Methods Manual: The Comprehensive Sourcebook of Techniques (4 Volume Set), 1st edition, I. Lefkovits, Academic Press (1997); Manipulating the Mouse Embryo: A Laboratory Manual, 3rd edition, Cold Spring Harbor Laboratory Press (2002); and in Ausubel et al., Current Protocols in Molecular Biology, John Wiley and Sons, Baltimore, Maryland (1989).

General principles of antibody engineering are set forth in Antibody Engineering: Methods and Protocols (Methods in Molecular Biology), B.L. Lo (Ed.), Humana Press (2003); Antibody engineering, R. Kontermann and S. Dubel (Eds.), Springer Verlag (2001); Antibody Engineering, 2nd edition, C.A.K. Borrebaeck (Ed.), Oxford Univ. Press (1995). General principles of protein engineering are set forth in Protein Engineering, A Practical Approach, Rickwood, D., et al. (Eds.), IRL Press at Oxford Univ. Press, Oxford, Eng. (1995). General principles of antibodies and antibody-hapten binding are set forth in: Antibodies: A Laboratory Manual, E. Harlow and D. Lane, Cold Spring Harbor Laboratory Press (1988); Nisonoff, A., Molecular Immunology, 2nd edition, Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (Eds.), Immunochemical Protocols (Methods in Molecular Biology), 2nd edition, J. D. Pound (Ed.), Humana Press (1998), Weir's Handbook of Experimental Immunology, 5th edition, D. M. Weir (Ed.), Blackwell Publishers (1996), Methods in Cellular Immunology, 2nd edition, R. Fernandez-Boltran, CRC Press (2001); Basic and Clinical Immunology, 8th edition, Appleton & Lange, Norwalk, CT (1994) and Mishell and Shiigi (Eds.), Selected Methods in Cellular Immunology, W.H. Freeman and Co., New York (1980).

Standard reference works setting forth general principles of immunology include Current Protocols in Immunology, John Wiley & Sons, New York; Klein, J.; Kuby Immunology, 4th edition, R. A. Goldsby, et al., H. Freeman & Co. (2000); Basic and Clinical Immunology, M. Peakman, et al., Churchill Livingstone (1997); Immunology, 6th edition, I. Roitt, et al., Mosby, London (2001); Cellular and Molecular Immunology, 5th edition; A.K. Abbas, A.H. Lichtman, Elsevier - Health Sciences Division (2005); Immunology Methods Manual: The Comprehensive Sourcebook of Techniques (4 Volume Set), 1st edition, I. Lefkovits, Academic Press (1997) Immunology, 5th edition, R.A. Goldsby, et al., W. H. Freeman (2002); Monoclonal Antibodies : Principles and Practice, 3rd Edition , J.W. Goding, Academic Press (1996); Immunology: The Science of Self-Nonself Discrimination, John Wiley & Sons, New York (1982); Kennett, R., et al. (Eds.), Monoclonal Antibodies, Hybridoma: A New Dimension in Biological Analyses, Plenum Press, New York (1980); Campbell, A., "Monoclonal Antibody Technology" in Burden, R., et al. (Eds.), Laboratory Techniques in Biochemistry and Molecular Biology, Vol. 13, Elsevere, Amsterdam (1984).

All of the references cited above, as well as all references cited herein, are incorporated herein by reference in their entireties.

### Examples

### Example 1

### Sp35 (LINGO-1) is expressed in Rat Midbrain Dopaminergic (DA) Neurons

The expression of Sp35 was evaluated in postnatal day 7 (P7 stage) rat brain, adult rat brain and rat primary embryonic cultures (E15) by immunohistochemistry and/or *in situ* hybridization. Frozen rat brain sections were prepared from rats at the P7 and adult stages of development mentioned above. Brain sections were prepared for *in situ* hybridization using the following protocol and as described in Mi et al. Neurosci. 7:221-228 (2004). Animals were euthanized with CO₂. The brains were quickly removed and fixed with 10% neutral buffered formalin for 48 hours. Brains were equilibrated in 30% sucrose in PBS for cryoprotection and sectioned serially. *In situ* hybridization was performed on randomly selected series of sections that contain every 6^{th} of the total ventral midbrain.

The brain sections were probed with digoxigenin-labeled Sp35 antisense and sense RNA. The sections were stained using the TSA plus fluorescence and anti-digoxigenin conjugated antibodies kit (Perkin Elmer) following the manufacturer's instructions. Sections were then stained with DAPI (Sigma) and anti-tyrosine hydroxylase (TH) antibodies (Chemicon). At the P7 stage, Sp35 mRNA was moderately expressed in midbrain tyrosine hydroxylase (TH)-positive DA neurons. There was also strong Sp35 mRNA expression in the cerebellum and weak expression in the striatum as reported previously. *See* Mi et al. Nat. Neurosci. 7: 221-228 (2004). In the adult midbrain, however, there was less Sp35 mRNA expression in TH positive DA neurons.

Primary embryonic ventral mesencephalon (VM) cultures were isolated from E15 Sprague Dawley rats (Charles River, MA) as described in Lin, L. et al. Mol. Cell Neurosci. 28:547-555 (2005). Briefly, brain tissue was mechanically dissociated with polished Pasteur pipettes in a cold Dulbecco's modified Eagle's medium (DMEM; Gibco, NY) containing heat-inactivated horse serum (10%), glucose (6.0 mg/ml), penicillin (10,000 U/ml), streptomycin (10 mg/ml; Sigma), and glutamine (2mM; Gibco). 2x10⁵ cells were resuspended in the medium, and seeded on a coverslip precoated with 15 mg/ml poly-L-ornithine (Sigma) and 1 mg/ml fibronectin (Sigma) of each well of a 24-well tray (Falcon).

Fluorescent immunohistochemistry was performed on the brain sections and VM primary cultures as described in Lin, L., et al., Mol. Cell. Neurosci. 28:547-555 (2005). Briefly, coverslips containing approximately 2x10⁵ cells were treated with 10% normal goat serum (Jackson Laboratories, Maine) and 0.1% Triton X-100 in 0.1 M phosphate-buffered saline (PBS) for 30 minutes at room temperature. Subsequently, the coverslips were incubated with primary antibodies at 4°C overnight and then with appropriate secondary antibodies conjugated with distinct fluorescence at room temperature for 1 hour. Antibodies against tyrosine hydroxylase (TH), a marker for DA neurons, (Chemicon) at a 1:300 dilution were used. Secondary antibodies conjugated with Alexa 488 (Molecular Probes) at a concentration of 1:500 were used. Omission of primary antibodies or antibodies pre-incubated with excess antigens were used as controls. Fluorescent signal was examined by a confocal imaging system (LSM510 META, Carl Zeiss, NY).

In primary VM cultures, Sp35 was observed in DA neurons where Sp35 and TH-staining colocalized. No staining was detected when an Sp35 specific antibody was pre-incubated with excess Sp35 protein. Sp35 is also expressed in the non-TH neurons in both human substantia nigra and rodent midbrain.

These experiments indicate that Sp35 is expressed in midbrain embryonic (E15) and P7 DA neurons, and to a lesser extent in the adult VM. Immunohistochemical studies also show that Sp35 protein is expressed not only in the neurites, but in the plasma membrane of DA neurons in the midbrain primary culture.

### Example 2

### Sp35 (LINGO-1) Antagonists Promote DA Neurite Outgrowth and Survival in vitro.

The cytoplasmic domain of Sp35 contains a canonical EGFR-like tyrosine phosphorylation site and thus has the potential for direct or indirect involvement in signaling. To evaluate the importance of the cytoplasmic domain, a truncated form of Sp35 (DN-Sp35) which lacked the cytoplasmic domain was created (amino acids 34-581 or 34-548 of SEQ ID NO:2). It has been shown that a truncated form of Sp35, with a deletion of the cytoplasmic domain, functions as a dominant negative (DN) molecule by forming a complex with Nogo receptor 1 (NgR1) and p75NTR and/or another receptor such as TAJ/TROY, thereby preventing signaling. See Shao et al. Neuron 45: 353-359 (2005) and Park et al. Neuron 45:345-351 (2005).

Lentiviruses were created which express full-length (FL)-Sp35 (amino acids 34-614 of SEQ ID NO:2) or dominant negative (DN)-Sp35 using the following methods and as described in Mi et al. Neurosci. 7: 221-228 (2004). cDNA sequences of full-length and truncated (DN-Sp35) human Sp35 were subcloned into pSECTAG-A (Invitrogen) to express HA-tagged fusion proteins and then were ligated to the HRST-IRESeGFP vector. Lentiviral constructs were cotransfected with vesicular stomatitis virus glycoprotein (VSV-G) and HIV-1 packaging vector delta 8.9 into 293T cells to generate recombinant lentiviruses as described Wang et al. Nature 417:941-944 (2002).

Cultures of rat primary VM neurons were transduced with lentiviruses producing FL-Sp35, dominant DN-Sp35 or with a vector control. Each group of viruses were added to the VM neuron cultures at a multiplicity of infection (MOI) of 1 or 5. Cells were cultured for 24 hours and then fixed with 4% paraformaldehyde in PB (pH 7.4) for 30 minutes at room temperature. Neurite outgrowth was examined in these infected neurons. For examination of neurite extension or cell numbers, several fields from each well were captured using an integrated Axioskop 2 micropscope (Carl Zeiss, NY) and Steroinvestigator image capture equipment and software (MicroBrightField, VT).

Neurite outgrowth was promoted in DN-Sp35 infected TH-positive neurons. In contrast, the TH-positive neurons showed no difference in neurite length when transduced with FL-Sp35. These observations indicate that DN-Sp35 disrupts the function of endogenous Sp35 thereby promoting DA neurite outgrowth. *See* Figure 1.

Sp35-Fc protein (amino acids 1-532 of SEQ ID NO:2 fused to an Fc domain) was used to determine whether it would function as an antagonist of FL-Sp35 function, in DA neurons, by promoting DA neurite outgrowth. Control-Fc and Sp35-Fc were prepared as described in Mi et al. Nat. Neurosci. 7:221-228 (2004). Briefly, amino acids 1-532 of human Sp35 were fused to the hinge and Fc region of human IgG1. The Sp35-Fc polypeptide was expressed in CHO cells and purified on Protein A Sepharose (Pharmacia). The purified protein (>95% pure) had a molecular weight of 90kDa as measured by gel electrophoresis on an SDS-PAGE gel under reducing conditions and compared to a known protein standard. The protein has a molecular weight of 180 kDa when run on an SDS-PAGE gel under non-reducing conditions and compared to a known standard.

The purified Sp35-Fc polypeptide was provided exogenously to cultures of VM neurons. Neurite outgrowth was promoted by the addition of excess Sp35-Fc. *See* Figure 1. Control IgG polypeptide supplied exogenously did not promote DA neurite outgrowth. Additionally, TH neurite length of cultures treated with lentiviruses expressing DN-Sp35 were examined. Cultures treated with DN-Sp35 and Sp35-Fc were significantly longer compared to treatment with control lentivirus (p<0.05, One-way ANOVA) and control Fc (p<0.003).

The effect of DN-Sp35 was also tested on primary VM cultures which were treated with 1-methyl-phenylpyridium ion (MPP+). MPP+ induces cell death of primary DA neuronal cells normally, and is a well-established model system for the study of PD. *See* Gille et al., Ann NY Acad Sci 1018:533-540 (2004). VM neurons were infected with lentiviruses as described above. Cultured cells were exposed to 10 µM MPP+ at day 4 for 48 hours followed by fixation (day 6). DN-Sp35 protected TH-positive neurons exposed to 10 µM MPP+ in rat midbrain primary cultures. *See* Figure 2. The number of TH neurons when exposed to MPP+ was significantly higher in the DN-Sp35. transduced cells compared to the FL-Sp35 and control transduced neurons (p<0.05, One-way ANOVA). Additionally, primary VM cultures exposed to MPP+ were protected from cell death by exposure to Sp35-Fc and a Sp35 antagonist, antibody 1A7, described in U.S. Provisional Patent Application No. 60/697,336, which is incorporated herein by reference in its entirety. Sp35-Fc and 1A7 antibody treated cells exhibited a significant protective effect against MPP+ toxicity on TH-positive neurons compared to control Fc or control IgG treated cultures (p <0.01, for 1A7 and p<0.05 for Sp35-Fc, One-way ANOVA). *See* Figure 3. These results indicate that inhibition of endogenous Sp35 protects DA neurons against MPP+ neurotoxin exposure.

### Example 3

### Blocking Sp35 Activity Induces Akt Phosphorylation

Akt is a downstream effector of the PI3 kinase survival pathway. Williams and Doherty Mol. Cell. Neurosci. 13:272-280 (1999). Levels of normal Akt phosphorylation in rat primary VM cultures were assessed by Western blot analysis. Rat primary VM neurons were transduced with lentiviruses expressing FL-Sp35 or DN-Sp35 (HA-tagged) as described in Example 2. Transduced rat primary VM neurons were harvested after 48 hours and lysed in 500µl lysis buffer (50 mM HEPES (pH 7.5); 150 mM NaCl; 1.5 mM MgCl₂; 1mM EDTA; 1% Triton X-100 and 10% glycerol) for 30 minutes at 4°C. The supernatants were electrophoresed on a 4-20% SDS-PAGE gel (Bio-Rad, CA), transferred to immunoblot membrane and probed with either anti-HA affinity matrix (Roche, Switzerland) or anti-phospho Akt antibody (Cell Signaling, MA), or anti-total Akt antibody (Cell Signaling, MA).

Akt phosphorylation was significantly increased after transduction of rat primary VM neurons with DN-Sp35 expressing lentivirus compared to transduction with a lentivirus which expresses FL-Sp35 or a control vector. *See* Figure 4. These results suggest that DN-Sp35 influences survival of TH-positive neurons, in part, by the involvement of PI3/Akt signaling pathway.

### Example 4

### Generation of Sp35 Knock-Out Mice

Sp35 knock-out mice were generated with a GFP/Neo (green fluorescent protein/neomycin) replacement vector that targeted the entire, single exon coding sequence of Sp35 as described by Schiemann et al. (Science 293: 2111-2114 (2001). Mouse genomic 129/SvJ DNA was isolated from a lambda genomic library (Stratagene #946313). A 14.6-kb EcoRV fragment was subcloned into pBSK+ and then was targeted by homologous recombination in bacteria to insert the eGFP Q40 reporter gene at the initiating ATG. The final construct deleted the entire 1-1,841 nucleotides of the single-exon coding sequence of Sp35. This construct was used to target the Sp35 locus in D3 (129/Sv) embryonic stem cells. Correctly targeted cells were identified by Southern blotting of EcoRI-digested embryonic stem cell DNA and were injected into C57B1/6 blastocysts to generate chimeric mice. Chimeras were crossed to C57B1/6 mice to generate heterozygous founder mice. Genotypes were determined by three-primer PCR of tail DNA. The forward primer, 5'-CTATCCAAGCACTGCCTGCTC-3' (SEQ ID NO:6), and the two reverse primers, 5'-GAGTTCTAGCTCCTCCAGGTGTG-3' (SEQ ID NO:7) and 5'-GATGCCCTTCAGCTCGATGCG-3' (SEQ ID NO:8), yielded 275 bp wild-type and 356 bp mutant allele products, respectively, in a 35-cycle reaction (94° C for 20s, 65° C for 30s, 72° C for 30s). See Mi, S. et al., Nat. Neurosci. 7: 221-228 (2004). Validation of Sp35 gene deletion was accomplished by Southern blot, RT-PCR and northern blot analyses. Prominent bands were detected in northern blot and RT-PCR in wild-type mice, but a complete absence of bands was found in the knockout mice. Southern blots of the heterozygotes showed both the wild-type and modified Sp35 allele. Sp35 knockout mice appeared normal, with no obvious physical abnormalities or alterations in behavior, locomotion or fecundity. The heterozygous F1 offspring litter mates varied in size. Generation of an Sp35 knock-out mouse is described in Mi et al. Nat. Neuroscience 7:221-228 (2004), which is incorporated herein by reference.

### Example 5

### In vivo 6-OHDA Assay for DA Neuron Survival and Regeneration in Sp35 Knock-out Mice

Sp35 knock-out mice were examined to determine if mice without Sp35 showed increased neuronal survival and improved recovery of function in dopaminergic pathways in the brain after injury. Thirteen Sp35 knockout mice and thirteen wild-type littermate control mice were anesthetized using ketamine and xylazine (100 and 10 mg/kg ip, respectively) and placed in a stereotaxic frame to receive unilateral intrastriatal injection of 6-hydroxydopamine HCl (6-OHDA). The surgical site was wiped with betadine and alcohol and a 0.5 cm midline saggittal incision was made to expose bregma. A small burr hole was made in the skull above the injection site and 10µg 6-OHDA dissolved in 0.02% ascorbate/saline (Sigma) was injected into the left striatum at coordinates AP+04, Lateral 1.5 mm, lateral to the midline, DV-2.5 mm ventral to the surface of the skull. The 6-OHDA is infused over 2 min at a rate of 0.5 µl/min using a 26 gauge 10 µl Hamilton syringe.

After infusion of the 6-OHDA, the cannula was left in place for an additional 2 min then withdrawn slowly. The incision was closed using one auto clip and the mice were placed on a warming pad until recovery from anesthesia. 6-OHDA injection into the striatum of mice produce a. progressive loss of DA axons and neurons, and is a well-established model system for the study of PD. *See* Brundin et al. Brain Res. 366:346-349 (1986).

Rotational testing was conducted 1, 2, 3, and 4 weeks post-6-OHDA infusion. For the rotational testing, mice were injected with apomorphine in 0.02% ascorbate subcutaneously at a dose of 0.4 mg/kg. Rotations contralateral to the lesion-side were counted over a 30 min period.

"Rotational behavior" is the behavior exhibited when an animal with unilateral damage to the nigrostriatal dopamine pathway is administered a dopamine agonist such as apormorphine or a dopamine releasing agent such as amphetamine. The animal repeatedly turns in circles away from the side of the brain experiencing greater striatal dopamine receptor stimulation. The magnitude of the rotational response, (*i.e*., the number of rotations performed) is directly proportional to the degree of damage to the nigrostriatal dopamine pathway. *See, e. g.,* Fuxe et al. Pharmacol. Ther. 2:41-47 (1976).

At least 24 h after measurement of rotational behavior, mice were euthanized by CO₂ asphyxiation. The brains were quickly removed and fixed with 10% neutral buffered formalin for 48 hours. Brains were equilibrated in 30% sucrose in PBS for cryoprotection and sectioned serially. Routine ABC immunohistochemistry was performed on randomly selected series of sections that contain every sixth of the total ventral midbrain. Sections were incubated with anti-tyrosine hydroxylase (TH) (1:300, Pel Freez, AK) overnight at 4°C and followed by incubation with biotinylated goat anti-sheep secondary antibody (1:300, Vector Laboratories, CA) and with streptavidin-biotin complex for one hour at room temperature, respectively. Staining was visualized by incubation with 3,3'-diaminobenzidine solution with nickel enhancement (Vector Laboratories, CA). Omission of the primary antibody served as a control. Stereology was performed on the stained sections using an integrated Axioskop 2 microscope (Carl Zeiss, NY) and Stereo Investigator image capture equipment and software (MicroBrightField, VT). TH positive cells were counted using an optical fraction probe. The estimated total number of cells was obtained using the Microbrightfield software. Stereological analysis was performed by investigators blind to the group treatments.

Motor asymmetry was significantly lower in the knock-out mice compared to wild-type littermate controls at all the timepoints examined (p<0.001 two-way ANOVA). *See* Figure 5. Postmortem analysis at 32 days after 6-OHDA injections revealed a marked reduction in the number of TH neurons in the lesioned midbrain. Stereological analysis revealed that the number of TH neurons in the midbrain did not differ in wild-type and knock-out mice (p > 0.05, unpaired t-test). *See* Figure 6A. To correct differences generated by genotype, the number of TH neurons in the lesioned midbrain was represented as a percentage of the number in the unlesioned side of each animal. Statistical analysis revealed a higher percentage of TH neurons in the knock-out than wild-type mice (p = 0.002, unpaired t-test) demonstrating that knock-out mice were protected in the 6-OHDA model as compared to the wild-type littermate controls. *See* Figure 6B.

The number of TH neurons was stereologically counted in the ventral tegmental area (VTA, A10 area) and substantial niagra compacta (SNc, A9 area) regions using an unbiased optical fractionator method as decribed in Blum, M., Nat. Neurosci. 1:374-377 (1998). There was no difference in the number of TH neurons between KO and WT mice (F_{1,48} = 1.321, p>0.05). However, the TH cell number was significantly different between lesion and non-lesion side (F_{1,48} = 27.53, p<0.0001), and the difference of TH cell numbers between lesion and non-lesion side was signficantly affected by the genotype (interaction between genotype and brain side) (F_{1,48} = 4.34, p>0.05). *See* Fig. 8. The number of neurons on the lesion side was normalised to the non-lesion side in each animal, to prevent any influence of genotype. Statistical analysis showed a higher number of TH neurons in the KO (mean ± s.e.m.: 79% ± 4.5%) than WT mice (56% ± 5.5%) (unpaired Student's t-test, t(24) = 3.34; p = 0.003), demonstrating a neuroprotective effect of the knock out in these mice.

In wild-type mice subjected to the 6-OHDA induced experimental parkinsonism model, Sp35 protein was increased in the striatum 3 days after injury. *See* Fig. 9. Sp35 is upregulated in the lesioned side (6-OHDA) compared to the contralateral side (control) 3 days after striatal 6-OHDA administration in wild type mice. Three mice were examined at each each time point (days 0, 3, 7 and 15) (unpaired Student's t-test, p<0.05).

### Example 6

### In vivo MPTP Assay for DA Neuron Survival and Regeneration in Sp35 Knock-out Mice

To further confirm that mice without Sp35 showed increased neuronal survival and improved recovery of function in dopaminergic pathways in the brain after injury, mice were also evaluated in an MPTP model of PD. Thirteen WT and fourteen Sp35 knock-out mice were injected intraperitoneally with 25 mg/kg of MPTP hydrochloride (Sigma) four times, with two hours between each injection. *See, e.g.,* Battaglia G. et al., Neuropharmacology 45:155-166 (2003). All animals were sacrificed 7 days post-injection. Mice were euthanized by CO₂ asphyxiation. The brains were quickly removed and fixed with 10% neutral buffered formalin for 48 hours. Brains were equilibrated in 30% sucrose in PBS for cryoprotection and sectioned serially. Stereology was performed on the stained sections using an integrated Axioskop 2 microscope (Carl Zeiss, NY) and Stereo Investigator image capture equipment and software (MicroBrightField, VT). TH positive cells were counted using an optical fraction probe. The estimated total number of cells was obtained using the Microbrightfield software. Stereological analysis was performed by investigators blind to the group treatments. Postmortem stereological analysis at 7 days after MPTP treatment revealed a higher number of TH neurons in the midbrain in the KO mice compared to WT. *See* Figure 6C.

In another experiment, 10 KO mice and 10 WT littermates were evaluated in an MPTP model of PD. Saline injected WT (n=7) and KO mice (n=8) served as controls. Postmortem stereological analysis 7 days after MPTP treatment revealed that the TH cell numbers in the substantia nigra compacta (SNc, A9 area) did not differ between the control WT and KO mice. However, the number of TH neurons was reduced in the SNc of the WT mice treated with MPTP compared with vehicle treated WT and KO mice (Kruskal-Wallis test, p<0.001, respectively). *See* Fig. 10. The number of TH neurons was not statistically different between control and MPTP treated KO mice.

The striatal dopamine levels of control and MPTP treated KO and WT mice were also examined. Dissected striata were sonicated and centrifuged in chilled 0.1M perchoric acid (PCA, about 100 µl/mg tissue). The supernatants were taken for measurements of dopamine as described in Yang, L. et al., Exp. Neurol. 191:86-93 (2005). Briefly, 15 µl of supernatant was isocratically eluated theough an 80 x 4.6 mm C18 column (ESA, Inc., Chelmsford, MA) with a mobile phase containing 0.1M LiH₂PO₄, 0.85mM 1-octanesulfonic acid and 10%(v/v) methanol and detected by a 2-channel Coulochem II electrochemical detector (ESA, Inc., Chelmsford, MA). Concentrations of dopamine were expressed as nanogram per milligram protein. Protein concentration of tissue homogenates was determined using the Bradford method (Bio-Rad Laboratories, Hercules, CA) and Perkin Elmer Bio Assay Reader (Norwalk, CT). The striatal dopamine levels were lower in the MPTP treated WT compared to the control WT mice (Kruskal-Wallis test, p<0.01) and control KO mice (p<0.05). *See* Fig. 11. The dopamine levels were not statistically different between the control and MPTP treated KO mice which is consistant with the neuroprotection also observed in the 6-OHDA paradigm.

To determine whether Sp35 genetic ablation alters MPTP toxin uptake and metabolism, striatal MPP+ levels in Sp35 KO and WT (n=6 for each group) were measured 90 minutes after MPTP treatment. For measuring MPP+, striatal tissues were sonciated and centrifuged in 0.1M PCA and an aliquot of supernatant was injected onto a META 250 x 4.6 C18 column (ESA, Inc., Chelmsford, MA). Samples were eluted isocratically with 20 mM boric acid-sodium borate buffer (pH 7.75) containing 3mM tetrabutylammonium hydrogensulfate, 0.25mM 1-heptanesulfonic acid and 10% isopropanol. MPP+ was detected with a fluorescence detector set by excitation at 295nm and emission at 375nm. MPP+ levels in the KO and WT, after MPTP treatment, were not significantly different (unpaired Student's test; t(10)=1.69; p>0.05, mean ± s.e.m., 39.68 ± 3.92 for WT and 62.06 ± 12.67 for KO). Western blot analysis also showed that dopamine transport (DAT) levels were not altered in the Sp35 KO mice. *See* Fig. 14.

Additionally, Western blots were performed on the lysates of VM tissue from KO and WT mice exposed to MPTP as well as controls. Mice tissues were lysed in 500 µl lysis buffer [50 mM HEPES, pH 7.5, 150 mM NaCl, 1.5 mM MgCl2, 1 mM EDTA, 1% Triton x-100, 10% glycerol containing Complete Protease Inhibitors (Roche, Basel, Switzerland) and Phosphatase Inhibitors (Sigma)]. The supernatants were electrophoresed on either 4-20% or 10% SDS-PAGE gel (Bio-Rad, Hercules, CA) and immunoblotted with anti-phospho-Akt, anti-Akt, and anti-EGFR antibodies (Cell Signaling, Beverly, MA). An increase of phosphorylated Akt in the ventral midbrain of KO mice exposed to MPTP was also found compared to those of WT littermate controls exposed to MPTP. *See* Figures 6D-6F.

Additionally, Sp35-Fc protein (6.5µg/µl, total 2 µl), which is the truncated form of Sp35 that functions as a dominant-negative molecule, was unilaterally injected in the striatum of C57BL/6 mice (n=9). These mice received MPTP 6 days after surgery and were then allowed to survive another 7 days. Postmortem analysis of TH neuron numbers in these animals showed a greater number in the ipsilateral SNc than the contralateral SNc (unpaired Student's t-test; t(16)=2.114; p<0.05. *See* Fig. 15A. The striatal dopamine levels were greater in the Sp35-Fc injected brain. (Mann-Whitney test, p<0.05), thus indicating a neuroprotective effect. *See* Fig. 15B. Moreover, the striatal MPP+ levels examined 90 minutes after MPTP injection were not different between the Sp35-Fc injected side and the contralateral side of the brain. *See* Fig. 15C.

### Example 7

### Generation of RNAi knockdown Lentiviruses

Sp35-specific RNAi is used to ablate Sp35 expression in DA neurons to examine how Sp35 contributes to DA neurite survival, regeneration and differentiation. DA neuronal cultures are infected with lentivirus carrying Sp35-specific RNAi sequence or control RNAi prepared as follows.

Murine and rat Sp35 DNA sequences were compared to find homologous regions to use for candidate small-hairpin RNAs (shRNA). CH324, for lentivirus expression of Sp35 RNAi, was constructed by annealing oligonucleotides LV1-035 and LV1-036 and ligating to *Hpa*I and *Xho*I digested pLL3.7. The pLL3.7 vector, additional methodology and virus production were as described in Rubinson et al., Nat. Genet. 33, 401-06 (2003). The Sp35 RNAi oligonucleotides were purchased from MWG and have the following sequences: 
LV1-035 (sense oligo)-
5' – TGATCGTCATCCTGCTAGACTTCAAGAGAGTCTAGCAGGATGACGATCTTTTTTC – 3'
(SEQ ID NO:9)
 and

LV1-036 (antisense oligo) 5'-
TCGAGAAAAAGATCGTCATCCTGCTAGACTCTCTTGAAGTCTAGCAGGATGACGATCA
- 3' (SEQ ID NO:10).

Control RNAi was designed with the same oligonucleotide sequences except for the nucleotide changes indicated in lower-case letters:
5'-TGATCcTCATcCttCTAtACTTCAAGAGAGTgTAGCAGGATGAcGATCTTTTTTCTCGA-3' (SEQ ID NO:11) and

5′-TCGAGAAAAAGATCGTCATCCTGCTAGACTCTCTTGAAGTaTAGaAGGATGACGA
TCA -3'. (SEQ ID NO:12).

Prior to producing the lentivirus, DNA from pLL3.7 or candidate shRNA in pLL3.7 were cotransfected with murine Sp35-HA tagged plasmid at a ratio of 5 to 1 into CHO cells in 6-well format. Knockdown was analyzed by western blot detection of Sp35-HA tag from transfected CHO cell lysates as well as by northern blot of total RNA prepared from duplicate wells. The blot was probed with a fragment of Sp35 cDNA. Assays were performed 48 hours post-transfection. As expected, there was a 10-fold reduction of Sp35 mRNA in CH324 RNAi-treated CHO cells relative to control-treated cells.

### Example 8

### Sp35-specific RNAi knockdown of Sp35 expression promotes DA neuronal survival and differentiation

To examine the effects of the lack of Sp35 expression in DA neurons, lentiviruses expressing RNAi molecules which ablate Sp35 expression, as described in Example 7, are used to infect rat primary VM neurons. Lentiviruses carrying green fluorescent protein (GFP) are generated as described in Rubinson *et al.* and Example 7. Rat primary VM neuronal cultures are infected at a multiplicity of infection (MOI) of 1-5 with either control or Sp35 RNAi. GFP positive cells indicate lentivirus infected DA neurons. The effect of the Sp35 knock-down is determined by DA neuronal extension and survival.

### Example 9

### Sp35-Fc and Sp35 1A7 antibody increases EGFR expression and phosphorlyation of Akt in MPP+-treated VM cultures.

To examine the phosphorylation of Akt and the interaction between Sp35 and EGFR in VM cultures treated with MPP+, primary cultures of DA neurons were obtained from the ventral midbrain of E15 or E16 Sprague Dawley rats (Charles River, MA). *See* Shah et al., J Cell Physiol in press. Briefly, tissues were mechanically dissociated with polished Pasteur pipets in a cold Dulbecco's modified Eagle's medium (DMEM; Gibco, NY) containing heat-inactivated horse serum (10%), glucose (6.0 mg/mL), penicillin (10,000 U/mL), streptomycin (10 mg/mL; Sigma), and glutamine (2 mM; Gibco). 2 x 10⁵ cells were resuspended in the medium and seeded on a coverslip pre-coated with 15 mg/ml poly-L-ornithine (Sigma) and 1 mg/ml Fibronectin (Sigma) in each well of a 24-well tray (Falcon). Unattached cells were aspirated on day 4, and 1 ml of fresh medium containing LINGO-1-Fc, 1A7, control IgG or control-Fc was added at a concentration of 10 µg/ml. 8 hours later, cultured cells were exposed to 10 µM MPP+ for 48 hours. The cultured VM cells were lysed 500 µl lysis buffer [50 mM HEPES, pH 7.5, 150 mM NaCl, 1.5 mM MgCl₂, 1 mM EDTA, 1% Triton x-100, 10% glycerol containing Complete Protease Inhibitors (Roche, Basel, Switzerland) and Phosphatase Inhibitors (Sigma)]. The supernatants were electrophoresed on either 4-20% or 10% SDS-PAGE gel (Bio-Rad, Hercules, CA) and immunoblotted with anti-phospho-Akt, anti-EGFR antibodies (Cell Signaling, Beverly, MA) or anti-Actin antibodies. EGFR and phosphorylation of Akt was increased in MPP+-treated VM neuron cultures incubated with the 1A7 antibody and Sp35-Fc protein compared to control IgG and control-Fc protein, respectively (F_{3,12}= 23.645; p<0.001 for 1A7; F _{3,12} = 18.89, p<0.001 for Sp-Fc). *See* Figures 7A - 7C and 7G.

Additionally, full length Sp35 decreases EGFR expression. COS7 cells were transfected with FL-Sp35 lentivirus, described in Example 2, at 0, 1 and 5 MOI for 2 days. The expression of EGFR protein in the transfected cells was,measured at each MOI. As can been seen in Fig. 12, the expression level of EGFR decreased in a dose dependent manner.

Direct interaction between Sp35 and EGFR in cultured cells and VM brain tissues from WT and KO mice was demonstrated by immunoprecipitation using anti-Sp35 (1A7 or 2F3) or anti-EGFR antibodies (see Figures 7D-7F). COS-7 or HEK 293 cells (100mm dishes) were transfected with Sp35, EGFR and Sp35/EGFR. The cells were harvested after 48 hours and lysed in 1 ml lysis buffer (50 mM HEPES, pH 7.5, 150 mM NaCl, 1.5 mM MgCl₂, 1 mM EDTA, 1% Triton x-100, 10% glycerol) or RIPA buffer (50mM TRIS, pH 7.2, 1% Triton X-100, 0.5% sodium deoxycholate, 0:1% SDS, 150mM NaCl, 10 mM MgCl₂, 5% glycerol) for 30 minutes at 4°C. After centrifugation at 14,000xg for 15 minutes, the supernatants were incubated with Protein A/G-Sepharose beads (Santa Cruz Biotechnology) at 4°C for 1 hour, and then 200 µl of the precleared lystates were incubated with either anti-EGFR (Santa Cruz Biotechnology) at 4°C for 1 hour, anti-Sp35 antibody (Biogen Idec, described in U.S. Provisional Patent Application No. 60/697,336, which is incorporated by reference herein in its entirety) followed by treatment with Protein A/G-Sepharose beads. The beads were washed 3 times with lysis buffer, boiled in Laemmeli sample buffer, subjected to 4-20% SDS-PAGE, and analyzed by Western blotting with anti-EGFR antibody or anti-Sp35 antibody. The EGFR and Sp35 antibodies were visualized using anti-rabbit IgG-HRP.

Sp35 KO or WT VMs were lysed in RIPA buffer and pre-cleared by Protein A/G plus-Sepharose beads as described above. 1 mg of the ventral midbrain extracts were then subjected to immunoprecipitations with an anti-Sp35 at 4°C overnight, followed by 1 hour incubation with Protein A/G plus-Sepharose beads. Direct interaction between Sp35 and EGFR was also observed in ventral midbrain cultures. *See* Fig. 7E.

Additionally, in IP experiments with Sp35 and EGFR co-transfected cell lines, the anti-Sp35 antibody 1A7 blocked binding of Sp35 to EGFR while the anti-Sp35 antibody 2F3 does not block binding. *See* Fig. 7F. In this experiment OMgp was used as a control for antibody binding.

### Example 10

Sp35 expression in surviving dopaminergic neurons from the substantia nigra (SN) of postmortem Parkinson Disease (PD) patients was examined. Postmortem tissue was obtained with consent from the Harvard Brain Tissue Resource Center. Table 2 below contains information regarding the PD patients and their age matched controls used in the experiments described in this example.

**TABLE 2**

| Information about PD patients and age-matched controls | | | | | |
|---|---|---|---|---|---|
| **Brain#** | **Diagnosis** | **Age (yrs)** | **Sex** | **PMI (hrs)** | **SN pathology** |
| PD1 | PD | 77 | M | 30.5 | 4+pallor |
| PD2 | PD | 70 | M | 13.58 | 4+pallor |
| PD3 | PD | 75 | M | 14.9 | 4+pallor Diffuse lewy bodies |
| PD4 | PD | 75 | M | 20 | pale, levy bodies |
| PDS | PD | 78 | F | 4.25 | pale, lewy bodies |
| PD6 | PD | 79 | F | 18.5 | 3-4+ pallor |
| C1 | Control | 70 | M | 15.1 | normal |
| C2 | Control | 73 | M | 30.25 | Moderately pigmented |
| C3 | Control | 74 | M | 14.33 | normal |
| C4 | Control | 75 | F | 16.67 | normal |
| C5 | Control | 75 | M | 20.5 | normal |
| C6 | Control | 78 | F | 22.67 | normal |

Sp35 expression was examined in surviving dopaminergic neurons, from the patients described above, by *in situ* hybridization. SN tissue was embedded in Optimum Cutting Temperature (OCT) compound. The frozen sections were prepared, processed and probed with digoxigenin-labeled Sp35 antisense and sense RNA as described in Mi et al. Nat. Neurosci. 7:221-228 (2004). Sections were stained using the TSA plus fluoresence and anti-digoxigenin conjugated antibodies kit (Perkin Elmer, Wellesley, MA) following the manufacturer's instructions. Sections were then stained with anti-TH (Chemicon, Temecula, CA) and DAPI (Sigma). Sp35 in the surviving dopaminergic neurons in the SN of PD postmortem tissue (n=6) was upregulated compared to aged matched controls (n=6).

Semi-quantitative PCR was also used to examine Sp35 expression in the patients described in Table 2. mRNA was extracted from human SN tissue using the Absolutely RNA miniprep kit (Strategene) following the manufacturer's instructions. Sp35 mRNA was amplified using the Sp35 forward primer - 5'-AGAGACATGCGATTGGTGA-3' (SEQ ID NO:14) and the reverse primer 5'-AGAGATGTAGACGAGGTCATT-3' (SEQ ID NO:15). As seen in Fig. 13A, Sp35 was upregulated in the surviving dopaminergic neurons in the SN of PD postmortem tissue compared to aged matched controls. Sp35 mRNA levels were statistically higher in the PD SN than controls (unpaired Student's T-test; *t*(10) = 2.280; p<0.05). *See* Fig. 13B.

The present invention is not to be limited in scope by the specific embodiments described which are intended as single illustrations of individual aspects of the invention, and any compositions or methods which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

All publications and patent applications mentioned in this specification are herein incorporated by reference to the same extent as if each individual publication or patent application was specifically and individually indicated to be incorporated by reference.
Preferred embodiments of the present invention are described below and are referred to as embodiments E1 to E56.
E1. A method for promoting regeneration, outgrowth or survival of dopaminergic neurons, comprising contacting said dopaminergic neurons with an effective amount of a composition comprising an Sp35 antagonist selected from the group consisting of:
   (i) a soluble Sp35 polypeptide;
   (ii) an Sp35 antibody or immunospecific fragment thereof;
   (iii) an Sp35 antagonist polynucleotide,
   (iv) an Sp35 aptamer; and
   (v) a combination of two or more of said Sp35 antagonists.
E2. The method for promoting regeneration, outgrowth or survival of dopaminergic neurons in a mammal, comprising administering to a mammal in need thereof an effective amount of a composition comprising an Sp35 antagonist selected from the group consisting of:
   (i) a soluble Sp35 polypeptide;
   (ii) an Sp35 antibody or fragment thereof;
   (iii) an Sp35 antagonist polynucleotide, and
   (iv) an Sp35 aptamer; and
   (v) a combination of two or more of said Sp35 antagonists.
E3. The method of 2, wherein said mammal is suffering from a disease, disorder, or injury associated with dopaminergic neuronal degeneration.
E4. The method 3, wherein said disease, disorder, or injury is selected from the group consisting of Parkinson's disease (PD), multiple system atrophy, striatonigral degeneration, olivopontocerebellar atrophy, Shy-Drager syndrome, motor neuron disease with parkinsonian features, Lewy body dementia, progressive supranuclear palsy, cortical-basal ganglionic degeneration, frontotemporal dementia, Alzheimer's disease with parkinsonism, Wilson disease, Hallervordern-Spatz disease, Chediak-Hagashi disease, SCA-3 spinocerebellar ataxia, X-linked dystonia-parkinsonism (DYT3), Huntington's disease (Westphal variant), prion disease, Jacob-Creutzfeldt disease (CJD), vascular parkinsonism, cerebral palsy, repeated head trauma, postencephalitic parkinsonism, neurosyphilis and schizophrenia.
E5. The method of 4, wherein said disease, disorder, or injury is Parkinson's disease (PD).
E6. The method of any one of 1 to 5, wherein said Sp35 antagonist comprises a soluble Sp35 polypeptide.
E7. The method of 6, wherein said soluble Sp35 polypeptide comprises an Sp35 region selected from the group consisting of:
   (i) an Sp35 Ig domain or a fragment, variant, or derivative thereof,
   (ii) an Sp35 LRR domain or a fragment, variant, or derivative thereof,
   (iii) an Sp35 cytoplasmic domain or a fragment, variant, or derivative thereof,
   (iv) an Sp35 transmembrane domain or a fragment, variant, or derivative thereof,
   (v) an Sp35 basic region C-terminal to the LRR domain or a fragment, variant, or derivative thereof, and
   (vi) a combination of at least two of said Sp35 domains or a fragments, variants, or derivatives thereof of (i) to (v).
E8. The method of 6, wherein said soluble Sp35 polypeptide lacks an Sp35 region selected from the group consisting of:
   (i) an Sp35 Ig domain,
   (ii) an Sp35 LRR domain,
   (iii) an Sp35 cytoplasmic domain,
   (iv) an Sp35 transmembrane domain,
   (v) an Sp35 basic region C-terminal to the LRR domain and
   (vi) a combination of at least two of said Sp35 domains of (i) to (v).
E9. The method of 8, wherein said soluble Sp35 polypeptide lacks an Sp35 transmembrane domain and an Sp35 cytoplasmic domain.
E10. The method of any one of 6 to 9, wherein said soluble Sp35 polypeptide comprises
   (i) an Sp35 LRR domain or a fragment, variant, or derivative thereof,
   (ii) an Sp35 basic region C-terminal to the LRR domain or a fragment, variant, or derivative thereof, and
   (iii) an Sp35 immunoglobulin (Ig) domain or a fragment, variant, or derivative thereof.
E11. The method of 8 or 9, wherein said soluble Sp35 polypeptide comprises at least a portion of the Sp35 LRR domain, but lacks an Sp35 Ig domain, Sp35 basic region, an Sp35 transmembrane domain, and an Sp35 cytoplasmic domain.
E12. The method of any one of 1 to 11, wherein said soluble Sp35 polypeptide comprises a polypeptide fragment selected from the group consisting of:
   (i) amino acids 1 to 33 of SEQ ID NO:2;
   (ii) amino acids 1 to 35 of SEQ ID NO:2;
   (iii) amino acids 34 to 64 of SEQ ID NO:2;
   (iv) amino acids 36 to 64 of SEQ ID NO:2;
   (v) amino acids 66 to 89 of SEQ ID NO:2;
   (vi) amino acids 90 to 113 of SEQ ID NO:2;
   (vii) amino acids 114 to 137 of SEQ ID NO:2;
   (viii) amino acids 138 to 161 of SEQ ID NO:2;
   (ix) amino acids 162 to 185 of SEQ ID NO:2;
   (x) amino acids 186 to 209 of SEQ ID NO:2;
   (xi) amino acids 210 to 233 of SEQ ID NO:2;
   (xii) amino acids 234 to 257 of SEQ ID NO:2;
   (xiii) amino acids 258 to 281 of SEQ ID NO:2;
   (xiv) amino acids 282 to 305 of SEQ ID NO:2;
   (xv) amino acids 306 to 329 of SEQ ID NO:2;
   (xvi) amino acids 330 to 353 of SEQ ID NO:2;
   (xvii) amino acids 363 to 416 of SEQ ID NO:2;
   (xviii) amino acids 417 to 424 of SEQ ID NO:2;
   (xix) amino acids 419 to 493 of SEQ ID NO:2;
   (xx) amino acids 494 to 551 of SEQ ID NO:2,
   (xxi) amino acids 1 to 64 of SEQ ID NO:2;
   (xxii) amino acids 1 to 89 of SEQ ID NO:2;
   (xxiii) amino acids 1 to 113 of SEQ ID NO:2;
   (xxiv) amino acids 1 to 137 of SEQ ID NO:2;
   (xxv) amino acids 1 to 161 of SEQ ID NO:2;
   (xxvi) amino acids 1 to 185 of SEQ ID NO:2;
   (xxvii) amino acids 1 to 209 of SEQ ID NO:2;
   (xxviii) amino acids 1 to 233 of SEQ ID NO:2;
   (xxix) amino acids 1 to 257 of SEQ ID NO:2;
   (xxxx) amino acids 1 to 281 of SEQ ID NO:2;
   (xxxxi) amino acids 1 to 305 of SEQ ID NO:2;
   (xxxxii) amino acids 1 to 329 of SEQ ID NO:2;
   (xxxxiii) amino acids 1 to 353 of SEQ ID NO:2;
   (xxxxiv) amino acids 1 to 416 of SEQ ID NO:2;
   (xxxxv) amino acids 1 to 424 of SEQ ID NO:2;
   (xxxxvi) amino acids 1 to 493 of SEQ ID NO:2;
   (xxxxvii) amino acids 1 to 551 of SEQ ID NO:2;
   (xxxxviii) amino acids 1 to 531 of SEQ ID NO:2
   (iL) amino acids 1 to 532 of SEQ ID NO:2;
   (Li) amino acids 34 to 89 of SEQ ID NO:2;
   (Lii) amino acids 34 to 113 of SEQ ID NO:2;
   (Liii) amino acids 34 to 137 of SEQ ID NO:2;
   (Liv) amino acids 34 to 161 of SEQ ID NO:2;
   (Lv) amino acids 34 to 185 of SEQ ID NO:2;
   (Lvi) amino acids 34 to 209 of SEQ ID NO:2;
   (Lvii) amino acids 34 to 233 of SEQ ID NO:2; (Lviii) amino acids 34 to 257 of SEQ ID NO:2;
   (Lix) amino acids 34 to 281 of SEQ ID NO:2;
   (Lx) amino acids 34 to 305 of SEQ ID NO:2;
   (Lxi) amino acids 34 to 329 of SEQ ID NO:2;
   (Lxii) amino acids 34 to 353 of SEQ ID NO:2;
   (Lxiii) amino acids 34 to 416 of SEQ ID NO:2;
   (Lxiv) amino acids 34 to 424 of SEQ ID NO:2;
   (Lxv) amino acids 34 to 493 of SEQ ID NO:2;
   (Lxvi) amino acids 34 to 551 of SEQ ID NO:2
   (Lxxi) amino acids 34 to 530 of SEQ ID NO:2;
   (Lxxii) amino acids 34 to 531 of SEQ ID NO:2;
   (Lxxiii) amino acids 34 to 532 of SEQ ID NO:2;
   (Lxxiv) amino acids 34 to 533 of SEQ ID NO:2;
   (Lxxv) amino acids 34 to 534 of SEQ ID NO:2;
   (Lxxvi) amino acids 34 to 535 of SEQ ID NO:2;
   (Lxxvii) amino acids 34 to 536 of SEQ ID NO:2;
   (Lxxviii) amino acids 34 to 537 of SEQ ID NO:2;
   (Lxxix) amino acids 34 to 538 of SEQ ID NO:2;
   (Lxxx) amino acids 34 to 539 of SEQ ID NO:2;
   (Lxxxi) amino acids 30 to 532 of SEQ ID NO:2;
   (Lxxxii) amino acids 31 to 532 of SEQ ID NO:2;
   (Lxxxiii) amino acids 32 to 532 of SEQ ID NO:2;
   (Lxxxiv) amino acids 33 to 532 of SEQ ID NO:2;
   (Lxxxv) amino acids 34 to 532 of SEQ ID NO:2;
   (Lxxxvi) amino acids 35 to 532 of SEQ ID NO:2;
   (Lxxxvii) amino acids 36 to 532 of SEQ ID NO:2;
   (Lxxxviii) amino acids 30 to 531 of SEQ ID NO:2;
   (Lxxxix) amino acids 31 to 531 of SEQ ID NO:2;
   (Lxxxx) amino acids 32 to 531 of SEQ ID NO:2;
   (Lxxxxi) amino acids 33 to 531 of SEQ ID NO:2;
   (Lxxxxii) amino acids 34 to 531 of SEQ ID NO:2;
   (Lxxxxiii) amino acids 35 to 531 of SEQ ID NO:2;
   (Lxxxxiv) amino acids 36 to 531 of SEQ ID NO:2
   (Lxxxxv) amino acids 36 to 89 of SEQ ID NO:2;
   (Lxxxxvi) amino acids 36 to 113 of SEQ ID NO:2;
   (Lxxxxvii) amino acids 36 to 137 of SEQ ID NO:2;
   (Lxxxxviii) amino acids 36 to 161 of SEQ ID NO:2;
   (Lxxxxvc) amino acids 36 to 185 of SEQ ID NO:2;
   (c) amino acids 36 to 209 of SEQ ID NO:2;
   (ci) amino acids 36 to 233 of SEQ ID NO:2;
   (cii) amino acids 36 to 257 of SEQ ID NO:2;
   (ciii) amino acids 36 to 281 of SEQ ID NO:2;
   (civ) amino acids 36 to 305 of SEQ ID NO:2;
   (cv) amino acids 36 to 329 of SEQ ID NO:2;
   (cvi) amino acids 36 to 353 of SEQ ID NO:2;
   (cvii) amino acids 36 to 416 of SEQ ID NO:2;
   (cviii) amino acids 36 to 424 of SEQ ID NO:2;
   (cix) amino acids 36 to 493 of SEQ ID NO:2;
   (cx) amino acids 36 to 551 of SEQ ID NO:2
   (cxi) amino acids 36 to 530 of SEQ ID NO:2;
   (cxii) amino acids 36 to 531 of SEQ ID NO:2;
   (cxiii) amino acids 36 to 532 of SEQ ID NO:2;
   (cxiv) amino acids 36 to 533 of SEQ ID NO:2;
   (cxv) amino acids 36 to 534 of SEQ ID NO:2;
   (cxvi) amino acids 36 to 535 of SEQ ID NO:2;
   (cxvii) amino acids 36 to 536 of SEQ ID NO:2;
   (cxviii) amino acids 36 to 537 of SEQ ID NO:2;
   (cxix) amino acids 36 to 538 of SEQ ID NO:2;
   (cxx) amino acids 36 to 539 of SEQ ID NO:2;
   (cxxi) variants or derivatives of any of said polypeptide fragments, and
   (cxxii) a combination of at least two of any of said polypeptide fragments or variants or derivatives thereof.
E13. The method of 12, wherein said soluble Sp35 polypeptide comprises amino acid residues 34-532 of SEQ ID NO: 2 or amino acids 3 6-532 of SEQ ID NO:2.
E14. The method of any one of 6-13 wherein said soluble Sp35 polypeptide comprises an Sp35 Ig domain or fragment, variant, or derivative thereof.
E15. The method of 6, wherein said soluble Sp35 polypeptide comprises amino acids 417-493 of SEQ ID NO:2.
E16. The method of any one of 6 to 15, wherein said soluble Sp35 polypeptide further comprises a non-Sp35 moiety.
E17. The method of 16, wherein said non-Sp35 moiety is a polypeptide fused to said soluble Sp35 polypeptide.
E18. The method of 17, wherein said non-Sp35 moiety is selected from the group consisting of an antibody Ig moiety, a serum albumin moiety, a targeting moiety, a reporter moiety, and a purification-facilitating moiety.
E19. The method of 18, wherein said non-Sp35 moiety is an antibody Ig moiety.
E20. The method of 19, wherein said antibody Ig moiety is a hinge and Fc moiety.
E21. The method of 17, wherein said soluble Sp35 polypeptide is conjugated to a polymer.
E22. The method of 21, wherein the polymer is selected from the group consisting of a polyalkylene glycol, a sugar polymer, and a polypeptide.
E23. The method of 22, wherein the polymer is a polyalkylene glycol.
E24. The method of 23, wherein the polyalkylene glycol is polyethylene glycol (PEG).
E25. The method of 23, wherein said soluble Sp35 polypeptide is conjugated to 1, 2, 3 or 4 polymers.
E26. The method of 25, wherein the total molecular weight of the polymers is from 5,000 Da to 100,000 Da.
E27. The method of any one of 1 to 5, wherein said Sp35 antagonist comprises an Sp35 antibody, or fragment thereof.
E28. The method of 27, wherein said Sp35 antibody or fragment thereof specifically binds to an epitope consisting essentially of a polypeptide fragment selected from the group consisting of:
   (i) amino acids 66 to 89 of SEQ ID NO:2;
   (ii) amino acids 66 to 113 of SEQ ID NO:2;
   (iii) amino acids 66 to 137 of SEQ ID NO:2;
   (iv) amino acids 90 to 113 of SEQ ID NO:2;
   (v) amino acids 114 to 137 of SEQ ID NO:2;
   (vi) amino acids 138 to 161 of SEQ ID NO:2;
   (vii) amino acids 162 to 185 of SEQ ID NO:2;
   (viii) amino acids 186 to 209 of SEQ ID NO:2;
   (ix) amino acids 210 to 233 of SEQ ID NO:2;
   (x) amino acids 234 to 257 of SEQ ID NO:2;
   (xi) amino acids 258 to 281 of SEQ ID NO:2;
   (xii) amino acids 282 to 305 of SEQ ID NO:2;
   (xiii) amino acids 306 to 329 of SEQ ID NO:2;
   (xiv) amino acids 330 to 353 of SEQ ID NO:2;
   (xv) amino acids 34 to 64 of SEQ ID NO:2;
   (xvi) amino acids 363 to 416 of SEQ ID NO:2;
   (xvii) variants or derivatives of any of said polypeptide fragments; and
   (xviii) a combination of two or more of any of said polypeptide fragments or variants or derivatives thereof.
E29. The method of any one of 1 to 5, wherein said Sp35 antagonist comprises an Sp35 antagonist polynucleotide.
E30. The method of 29, wherein said Sp35 antagonist polynucleotide is selected from the group consisting of:
   (i) an antisense polynucleotide;
   (ii) a ribozyme;
   (iii) a small interfering RNA (siRNA); and
   (iv) a small-hairpin RNA (shRNA).
E31. The method of 30, wherein said Sp35 antagonist polynucleotide is an antisense polynucleotide comprising at least 10 bases complementary to the coding portion of the Sp35 mRNA.
E32. The method of 30, wherein said Sp35 antagonist polynucleotide is a ribozyme.
E33. The method of 30, wherein said Sp35 antagonist polynucleotide is a siRNA.
E34. The method of 30, wherein said Sp35 antagonist polynucleotide is a shRNA.
E35. The method of 34, wherein said shRNA comprises the nucleotide sequence: UGAUCGUCAU CCUGCUAGAC UUCAAGAGAG UCUAGCAGGA UGACGAUCUU UUUUC (SEQ ID NO:13).
E36. The method any one of 1 to 35, wherein said Sp35 antagonist is administered by bolus injection or chronic infusion.
E37. The method of 36, wherein said Sp35 antagonist is administered directly into the central nervous system.
E38. The method of any one of 1 to 37, comprising (a) introducing into said dopaminergic neurons a polynucleotide which encodes said Sp35 antagonist through operable linkage to an expression control sequence, and (b) allowing expression of said Sp35 antagonist.
E39. The method of any one of 38 or 39, wherein said polynucleotide is introduced into said dopaminergic neurons by a method selected from the group consisting of:
   (a) transfection;
   (b) electroporation;
   (c) transduction; and
   (d) direct microinjection.
E40. The method of any one of 2 to 37, comprising (a) administering to said mammal a polynucleotide which encodes said Sp35 antagonist through operable linkage to an expression control sequence, and (b) allowing expression of said Sp35 antagonist.
E41. The method of 39, wherein said polynucleotide is administered in a cultured host cell capable of expressing said polynucleotide.
E42. The method of any one of 38 to 41, wherein said polynucleotide is administered as an expression vector.
E43. The method of 42, wherein said expression vector is a viral vector.
E44. The method of any one of 38 to 41, wherein said polynucleotide is introduced into said mammal at or near the site of the nervous-system disease, disorder or injury.
E45. The method of 41, wherein said cultured host cell is made by a method comprising (a) transforming or transfecting a recipient host cell with the polynucleotide of 40 or the vector of 42 or 43, and (b) culturing said transformed or transfected host cell.
E46. The method of any one of 41 to 45, wherein said host cell is derived from the mammal to be treated.
E47. The method of any one of 1 to 46, wherein said Sp35 antagonist is expressed in an amount sufficient to reduce inhibition of dopaminergic neuronal regeneration, outgrowth or survival at or near the site of the nervous system disease, disorder, or injury.
E48. The method of 43, wherein the viral vector is selected from the group consisting of an adenoviral vector, an alphavirus vector, an enterovirus vector, a pestivirus vector, a lentivirus vector, a baculovirus vector, a herpesvirus vector, a papovavirus vector, a parvovirus and a poxvirus vector.
E49. The method of 48, wherein said herpesvirus vector is selected from the group consisting of a herpes simplex virus vector and an Epstein Barr virus vector.
E50. The method of 48, wherein said poxvirus vector is a vaccinia virus vector.
E51. The method of 48, wherein said lentivirus vector pLL3.7.
E52. The method of 48, wherein said parvovirus as adeno-associated virus (AAV).
E53. The method of any one of 43, or 48 to 52, wherein said vector is administered by a route selected from the group consisting of topical administration, intraocular administration, parenteral administration, intrathecal administration, subdural administration and subcutaneous administration.
E54. A method for inhibiting the EGFR and Sp35 interaction in dopaminergic neurons, comprising contacting said dopaminic neurons with an effective amount of a composition comprising an Sp35 antagonist selected from the group consisting of:
   (i) a soluble Sp35 polypeptide;
   (ii) an Sp35 antibody or fragment thereof;
   (iii) an Sp35 antagonist polynucleotide;
   (iv) an Sp35 antagonist aptamer; and
   (v) a combination of two or more of said Sp35 antagonists.
E55. A method for increasing Akt phosphorylation in dopaminergic neurons, comprising contacting said dopaminic neurons with an effective amount of a composition comprising an Sp35 antagonist selected from the group consisting of:
   (i) a soluble Sp35 polypeptide;
   (ii) an Sp35 antibody or fragment thereof;
   (iii) an Sp35 antagonist polynucleotide,
   (iv) an Sp35 antagonist aptamer; and
   (v) a combination of two or more of said Sp35 antagonists.
E56. A method for increasing EGFR expression in a dopaminergic neuron, comprising contacting said dopaminergic neurons with an effective amount of a composition comprising an Sp35 antagonist selected from the group consisting of:
   (i) a soluble Sp35 polypeptide;
   (ii) an Sp35 antibody or fragment thereof;
   (iii) an Sp35 antagonist polynucleotide,
   (iv) an Sp35 antagonist aptamer; and
   (v) a combination of two or more of said Sp35 antagonists.

## Claims

1. Use of an Sp35 antagonist selected from the group consisting of:
(i) a soluble Sp35 polypeptide;
(ii) an Sp35 antibody or immunospecific fragment thereof;
(iii) an Sp35 antagonist polynucleotide,
(iv) an Sp35 aptamer; and
(v) a combination of two or more of the Sp35 antagonists;
wherein said Sp35 antagonist is capable of inhibiting the binding of Sp35 to EGFR,
for the preparation of a medicament for treating a disease, disorder, or injury associated with dopaminergic neuron degeneration or death in a mammal.

2. The use of claim 1, wherein the disease, disorder, or injury is Parkinson's disease.

3. The use of claim 1 or 2, wherein the Sp35 antagonist comprises a soluble Sp35 polypeptide.

4. The use of claim 3, wherein the soluble polypeptide comprises amino acids 419 to 493 of SEQ ID NO:2 or amino acids 34 to 532 of SEQ ID NO:2

5. The use of claim 3 or 4, wherein the soluble Sp35 polypeptide further comprises a heterologous polypeptide.

6. The use of claim 5, wherein the heterologous polypeptide is selected from the group consisting of an antibody Ig polypeptide, a serum albumin polypeptide, a targeting polypeptide, a reporter polypeptide, and a purification-facilitating polypeptide.

7. The use of claim 1 or 2, wherein the Sp35 antagonist comprises an Sp35 antibody or an immunospecific fragment thereof.

8. The use of claim 7, wherein the Sp35 antibody or immunospecific fragment thereof binds to amino acids 419 to 493 of SEQ ID NO:2.

9. The use of claim 1 or 2 wherein the Sp35 antagonist polynucleotide is selected from the group consisting of:
(i) an antisense polynucleotide;
(ii) a ribozyme;
(iii) a small interfering RNA (siRNA); and
(iv) a small-hairpin RNA (shRNA).

10. The use of claim 9, wherein the shRNA comprises the nucleotide sequence: UGAUCGUCAU CCUGCUAGAC UUCAAGAGAG UCUAGCAGGA UGACGAUCUU UUUUC (SEQ ID N0:13).
